(19) Europäisches Patentamt
European Patent Office
Office européen des brevets

(11) EP 0 587 088 B1

(12) EUROPÄISCHE PATENTSCHRIFT

(45) Veröffentlichungstag und Bekanntmachung des Hinweises auf die Patenterteilung: **08.05.1996 Patentblatt 1996/19**

(21) Anmeldenummer: **93114261.6**

(22) Anmeldetag: **06.09.1993**

(51) Int. Cl.⁶: **C07C 69/757**, A61K 31/215, A61K 31/28, A61K 31/395, C07D 333/16, C07D 213/79, C07D 333/38

# (54) Substituierte Cyclohexan-Derivate, Verfahren zu ihrer Herstellung und die Anwendung der Verbindungen zur Behandlung von Krankheiten

Derivatives of substituted cyclohexane, their process of preparation and their application with the treatment of diseases

Dérivés du cyclohexane substitué, leur procédé de préparation et l'utilisation des composés pour le traitement de maladies

(84) Benannte Vertragsstaaten:
**AT BE CH DE DK ES FR GB GR IE IT LI LU MC NL PT SE**

(30) Priorität: **09.09.1992 DE 4230067**

(43) Veröffentlichungstag der Anmeldung:
**16.03.1994 Patentblatt 1994/11**

(73) Patentinhaber: **HOECHST AKTIENGESELLSCHAFT**
**D-65929 Frankfurt am Main (DE)**

(72) Erfinder:
- **Hemmerle, Horst, Dr.**
  **D-64653 Lorsch (DE)**
- **Schindler, Peter, Dr.**
  **D-65812 Bad Soden (Taunus) (DE)**
- **Herling, Andreas, Dr.**
  **D-65520 Bad Camberg (DE)**

(56) Entgegenhaltungen:
- **JOURNAL OF THE CHEMICAL SOCIETY, SECTION C: ORGANIC CHEMISTRY 1971 , LETCHWORTH GB Seiten 1496 - 1500 E.HASLAM ET AL. 'The Shikimate Pathway.Part II.Conformational Analysis of (-)-Quinic Acid and Its Derivatives by Proton Magnetic Resonance Spectroscopy.'**

EP 0 587 088 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.11.6/3.4

## Beschreibung

Das Krankheitsbild des Diabetes ist durch erhöhte Blutzuckerwerte gekennzeichnet.

Beim insulinpflichtigen oder Typ I Diabetes ist die Ursache das Absterben der insulinproduzierenden β-Zellen des Pankreas; die Behandlung erfolgt daher durch Insulingabe (Substitutionstherapie). Der nicht-insulinabhängige oder Typ II Diabetes ist dagegen durch eine verminderte Insulinwirkung auf Muskel- und Fettgewebe (Insulinresistenz) und eine gesteigerte Glucoseproduktion der Leber gekennzeichnet.

Die Ursachen dieser Stoffwechselstörungen sind noch weitgehend ungeklärt. Die etablierte Therapie mit Sulfonylharnstoffen versucht die Insulinresistenz durch Steigerung der körpereigenen Insulinfreisetzung Zu kompensieren, führt jedoch nicht in allen Fällen zu einer Normalisierung des Blutzuckerspiegels und vermag das Fortschreiten der Krankheit nicht aufzuhalten; viele Typ II Diabetiker werden schließlich durch "Erschöpfung" der β-Zellen insulinpflichtig und leiden an Spätschäden wie Katarakten, Nephropathien und Angiopathien.

Neue Therapieprinzipien zur Behandlung des Typ II Diabetes sind deshalb wünschenswert.

Die Konzentration der Blutglucose im nüchternen Zustand wird durch die Glucoseproduktion der Leber bestimmt. Verschiedene Arbeitsgruppen konnten zeigen, daß die Erhöhung der Blutzuckerwerte bei Typ II Diabetes mit einer proportional erhöhten Glucoseabgabe aus der Leber korrelieren. Die von der Leber in das Blut abgegebene Glucose kann sowohl durch Abbau von Leber-Glycogen (Glycogenolyse) als auch durch Gluconeogenese gebildet werden.

Glucose-6-Phosphat ist das gemeinsame Endprodukt sowohl der Gluconeogenese als auch der Glykogenolyse. Der terminale Schritt der hepatischen Freisetzung von Glucose aus Glucose-6-Phosphat wird von der Glucose-6-Phosphatase (EC 3.1.3.9) katalysiert. Die Glucose-6-Phosphatase stellt einen im endoplasmatischen Reticulum (ER) vorkommenden Multienzym-Komplex dar. Dieser Enzym-Komplex besteht aus einer in der ER-Membran vorliegenden Glucose-6-Phosphat-Translocase, einer auf der luminalen Seite des endoplasmatischen Reticulum lokalisierten Glucose-6-Phosphatase und einer Phosphat-Translocase [für eine Übersicht siehe: Ashmore, J. und Weber G., "The Role of Hepatic Glucose-6-phosphatase in the Regulation of Carbohydrate Metabolism", in Vitamins and Hormones, Vol XVII (Harris R.S., Marrian G.F., Thimann K.V., Edts.), 92-132, (1959); Burchell A., Waddell I.D., "The molecular basis of the hepatic microsomal glucose-6-phoshatase system", Biochim. Biophys. Acta 1092, 129-137, (1990)]. Die vorliegende umfangreiche Literatur zeigt, daß unter allen untersuchten Bedingungen, die im Tierversuch zu erhöhten Blutglucose-Werten führen, z.B. Streptozotocin, Alloxan, Cortison, Thyroid-Hormone und Hungern, die Aktivität dieses Multienzym-Komplexes ebenfalls erhöht ist. Darüber hinaus weisen zahlreiche Untersuchungen darauf hin, daß die bei Typ II Diabetikern beobachtete erhöhte Glucose-Produktion mit einer erhöhten Glucose-6-Phosphatase-Aktivität verbunden ist. Die Bedeutung des Glucose-6-Phosphatase-Systems für eine normale Glucose-Homeostase wird ferner unterstrichen durch die hypoglykämischen Symptome von Patienten mit Glykogen-Speicherkrankheit Typ Ib, denen die Translocase-Komponente des Glucose-6-Phosphat-Systems fehlt.

Eine Verringerung der Glucose-6-Phosphatase-Aktivität durch geeignete Wirkstoffe (Inhibitoren) sollte zu einer entsprechend verringerten hepatischen Glucose-Freisetzung führen. Diese Wirkstoffe sollten in der Lage sein, die Glucose-Produktion der Leber dem effektiven peripheren Verbrauch anzupassen. Die dadurch im nüchternen Zustand von Typ II Diabetikern gesenkten Blutglucose-Werte dürften darüber hinaus auch eine präventive Wirkung im Hinblick auf diabetische Spätschäden haben.

In der Literatur ist eine Reihe von unspezifischen Inhibitoren der Glucose-6-Phosphatase beschrieben worden wie z.B. Phlorrhizin [Soodsma, J. F., Legler, B. und Nordlie, R. G., J. Biol. Chem. 242, 1955-1960, (1967)], 5,5'-Dithio-bis-2-nitrobenzoesäure [Wallin, B. K. und Arion, W. J., Biochem. Biophys. Res. Commun. 48, 694-699, (1972)], 2,2'-Di-isothiocyanato-stilben und 2-Isothiocyanato-2'-acetoxy-stilben [Zoccoli, M. A. und Karnowski, M. L., J. Biol. Chem. 255, 1113-1119, (1980)]. Bisher liegen jedoch noch keine therapeutisch verwertbaren Inhibitoren des Glucose-6-Phosphatase-Systems vor.

Die nachfolgend näher charakterisierten Cyclohexan-Derivate sind neue, in der chemischen und biologischen Literatur bisher nicht beschriebene Verbindungen. Wir haben nun gefunden, daß bestimmte Ester von substituierten Cyclohexancarbonsäuren, wie z. B. Beispiel 14, Hemmer des Glucose-6-Phosphatase-Systems sind.

Die Erfindung betrifft daher Cyclohexan-Derivaten der Formel I

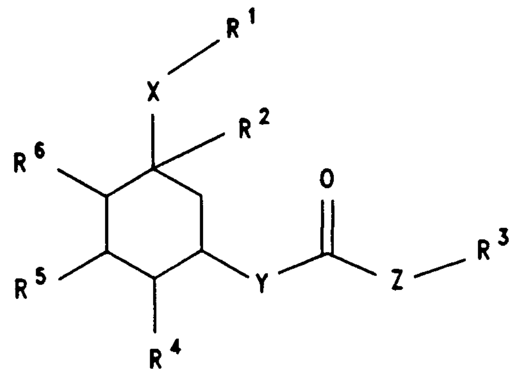

I

worin die Reste die folgenden Bedeutungen haben:

| | |
|---|---|
| $R^1$: | CN, COOH, eine mit einer Schutzgruppe geschützte COOH-Gruppe, $C_1$-$C_4$-Alkanoyl, $SO_3$-$C_1$-$C_4$-Alkyl, $SO_3H$, $SO_2NR^8R^9$, $PO(OH)_2$, $PO(OH)(O$-$C_1$-$C_4$-Alkyl), $PO(O$-$C_1$-$C_4$-Alkyl$)_2$, |
| $R^2$: | $C_1$-$C_{10}$-Alkyl $(R^{11})_n$, O-$C_1$-$C_{10}$-Alkyl $(R^{11})_n$, $C_2$-$C_{10}$-Alkenyl $(R^{11})_n$, O-$C_3$-$C_{10}$-Alkenyl $(R^{11})_n$, $C_2$-$C_{10}$-Alkinyl $(R^{11})_n$, O-$C_3$-$C_{10}$-Alkinyl $(R^{11})_n$, S-$C_1$-$C_{10}$-Alkyl $(R^{11})_n$, S-$C_3$-$C_{10}$-Alkenyl $(R^{11})_n$, S-$C_3$-$C_{10}$-Alkinyl $(R^{11})_n$, NH-$C_1$-$C_{10}$-Alkyl $(R^{11})_n$, NH-$C_3$-$C_{10}$-Alkenyl $(R^{11})_n$ oder NH-$C_3$-$C_{10}$-Alkinyl $(R^{11})_n$, wobei $R^{11}$ gegebenenfalls jeweils mit $R^{12}$ substituiert ist; |
| $R^3$, $R^{11}$ und $R^{13}$: | Alkyl mit 1 bis 10 C-Atomen, Cycloalkyl mit 3-8 Ring-C-Atomen, Phenyl, Naphtyl, Phenanthryl, Pyridyl, Thienyl, Furyl, Pyrimidyl, Indolyl, Imidazolyl, Coumarinyl, Phthaliminyl, Chinolyl, Piperazinyl, Tetrazolyl, Triazolyl, Oxazolyl oder deren thieno-, pyridino-, pyrimidino- oder benzoanellierte Derivate, wobei der Aromat bzw. Heteroaromat ein- oder mehrfach mit F, Cl, Br, J, OH, $CF_3$, - $NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann, und $R^3$, $R^{11}$ und $R^{13}$ gleich oder verschieden sind; |
| $R^4$, $R^5$ und $R^6$: | H, OH, eine durch übliche Alkoholschutzgruppen geschützte OH-Gruppe, F, Cl, Br oder die für $R^2$ angegebenen Bedeutungen haben, wobei $R^4$, $R^5$ und $R^6$ gleich oder verschieden sind; |
| $R^7$: | $C_1$-$C_4$-Alkyl, Phenyl oder Benzyl; |
| $R^8$ und $R^9$: | H, $C_1$-$C_4$-Alkyl, $C_1$-$C_4$-Alkanoyl, Phenyl, das gegebenenfalls substituiert ist mit F, Cl, Br, J, OH, O-$C_1$-$C_4$-Alkyl, $CF_3$, -$NO_2$ oder CN, wobei $R^8$ und $R^9$ gleich oder verschieden sind, oder $R^8$ und $R^9$ bilden zusammen mit dem Stickstoffatom einen 4 bis 10gliedrigen, gesättigten heterocyclischen Ring, bei dem gegebenenfalls eine $CH_2$-Gruppe durch O, S oder $NR^{10}$ ersetzt sein kann, |
| $R^{10}$: | H, $C_1$-$C_4$-Alkyl, Phenyl oder Benzyl |
| $R^{12}$: | Phenyl, Naphtyl, Phenanthryl, Pyridyl, Thienyl, Furyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, J, OH, $CF_3$, - $NO_2$, CN, $C_1$-$C_4$-Alkoxy, $C_1$-$C_4$-Alkyl, $C_2$-$C_4$-Alkenyl, $NR^8R^9$, Phenyl, Benzyl, Thienyl, Furyl, Imidazolyl, Pyridyl, O-Phenyl oder O-Benzyl gleich oder verschieden substituiert sein kann; |

X: $(CH_2)_m$, -CH=CH-, -C≡C-, $-CH_2-O-CH_2-$, $-CH_2-S-CH_2-$ oder

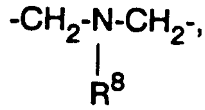

Y: $(CH_2)_m$, O, S, $NR^8$,

Z: $(CH_2)_m$, S, O, S-$C_1$-$C_{10}$-Alkyl, O-$C_1$-$C_{10}$-Alkyl, CH=CH, CH=CF, CH=CCl, CH=CBr, $CH_2$-CO, $CH_2$-CHF, $CH_2$-CHCl, $CH_2$-CHBr, $CH_2$-CHJ, $C_3$-$C_{10}$-Cycloalkylen, $C_3$-$C_{10}$-Cloalkenylen, wobei 1 bis 3 Ring-C-Atome durch Schwefel-, Sauerstoff- oder Stickstoffatome ersetzt sein können, $COOR^7$, C≡C, CH=C($C_1$-$C_4$-Alkyl), CH=C(CN), CH=C($NR^8R^9$), CH=C($C_1$-$C_4$-Alkanoyl), CH=C($R^{13}$), $NR^8$ und wenn Y Sauerstoff ist, kann

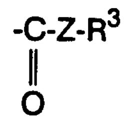

gemeinsam einen Aminosäurerest, ausgewählt aus der Gruppe bestehend aus Ala, Arg, Asn, Asp, Cys, Gln, Glu, Gly, His, Ile,Leu, Lys, Phe, Pro, Ser, Thr, Trp, Tyr und deren durch übliche Schutzgruppen geschützte Derivate, darstellen,

n: null, 1 oder 2

m: null, 1, 2, 3 oder 4.

Die erfindungsgemäßen Verbindungen der Formel I können, soweit sie eine Carboxylgruppe enthalten, Salze mit anorganischen oder organischen Basen bilden. Die Erfindung betrifft daher auch die physiologisch verträglichen Salze von Verbindungen der Formel I, wobei die Verbindung der Formel Ia (vgl. Anspruch 1) ausgenommen ist.
Die erfindungsgemäßen Verbindungen der Formel I enthalten eine Reihe von Stereozentren. Die Erfindung betrifft alle möglichen Enantio- und Diastereomere. Sie werden alle durch die Formel I wiedergegeben.
Sofern nichts anderes angegeben ist, gilt für die vorstehenden und folgenden Ausführungen:
Die unter $R^1$, $R^3$, $R^7$, $R^8$, $R^9$, $R^{11}$, $R^{12}$, $R^{13}$ und Z angegebenen Alkyl-, Alkanoyl und Alkoxyreste sind geradkettig oder verzweigt.
Die unter $R^2$ und $R^{12}$ angegebenen Alkyl-, Alkenyl- und Alkinylgruppen sind geradkettig, verzweigt oder cyclisch, wobei auch nur ein Teil des Restes einen Ring bilden kann.

Eine der $CH_2$-Gruppen kann durch O, S, SO, $SO_2$ oder $NR^8$ ersetzt sein, $R^{11}$ kann mit $R^{12}$ substituiert sein und bei n=2 sind die beiden Reste $R^{11}$ gleich oder verschieden. Ungesättigte Reste sind ein- oder mehrfach ungesättigt.

Unter einem mit einer Schutzgruppe geschützten COOH-Rest wird COO-$C_1$-$C_{10}$-Alkyl (unverzweigt oder verzweigt oder cyclisch), COO-CH($R^7$)-O-$C_1$-$C_4$-Alkanoyl (unverzweigt oder verzweigt), COO-Benzyl, COOPhenyl, $CONH_2$, CONH-$C_1$-$C_{10}$-Alkyl (unverzweigt und verzweigt), $-CONR^8R^9$ verstanden, wobei $R^7$, $R^8$ und $R^9$ die angegebenen Bedeutungen haben.

Alkoholschutzgruppen sind:
Substituierte Ether wie
Methoxymethyl, Methylthiomethyl, t-Butylthiomethyl, Benzyloxymethyl, p-Methoxybenzyloxymethyl, t-Butoxymethyl, Siloxymethyl, 2-Methoxyethoxymethyl, 1-Ethoxyethyl, Allyl, Benzyl, p-Methoxybenzyl, 3,4-Dimethoxybenzyl, o-Nitrobenzyl, p-Nitrobenzyl, p-Halobenzyl, 2,6-Dichlorbenzyl, p-Cyanbenzyl, p-Phenylbenzyl, 2- und 4-Picolyl.

Schutzgruppen für die Aminosäuren sind:

a) Carbamate wie
Methyl und Ethyl, 9-Fluorenylmethyl, 9-(2-Sulfo)fluorenylmethyl, 9-(2,7-Dibrom)fluorenylmethyl, 2,7-Di-t-butyl-[9-(10,10-dioxo-10,10,10,10-tetrahydrothioxanthyl)]methyl, 4-Methoxyphenacyl, 2,2,2-Trichlorethyl, 2-Trimethylsilylethyl, 2-Phenylethyl, 1-(1-Adamantyl)-1-methylethyl, 1,1-Dimethyl-2-haloethyl, 1,1-Dimethyl-2,2-dibromethyl, 1,1-Dimethyl-2,2,2-trichlorethyl, 1-Methyl-1-(4-biphenylyl)ethyl, 1-(3,5-Di-t-butylphenyl)-1-methylethyl, 2-(2'- und 4'-Pyridyl)ethyl, 2-(N,N-Dicyclohexylcarboxamido)ethyl, t-Butyl, l-Adamantyl, Vinyl, Allyl, l-Isopropylallyl, Cinnamyl, 4-Nitrocinnamyl, 8-Chinolyl, N-Hydroxypiperidinyl, Alkylthio, Benzyl, p-Methoxybenzyl, p-Nitrobenzyl, p-Brombenzyl, p-Chlorobenzyl, 2,4-Dichlorbenzyl, 4-Methylsulfinylbenzyl, 9-Anthrylmethyl und Diphenylmethyl, t-Amyl, S-Benzylthiocarbamat, p-Cyanobenzyl, Cyclobutyl, Cyclohexyl, Cyclopentyl, Cyclopropylmethyl, p-Decycloxybenzyl, Diisopropylmethyl, 2,2-Dimethoxycarbonylvinyl, o-(N,N-Dimethylcarboxamido)benzyl, 1,1-Dimethyl-3-(N,N-dimethylcaboxamido)propyl, 1,1-Dimethylpropynyl, Di-(2-pyridyl)methyl, 2-Furanylmethyl, 2-Jodoethyl, Isobornyl, Isobutyl, Isonicotinyl, p-(p'-Methoxyphenylazo)benzyl, 1-Methylcyclobutyl, 1-Methylcyclohexyl, 1-Methyl-1-cyclopropylmethyl, 1-Methyl-1-(3,5-dimethoxyphenyl)ethyl, 1-Methyl-1-(p-phenylazophenyl)ethyl, 1-Methyl-1-phenylethyl, 1-Methyl-1-(4-pyridyl)ethyl, Phenyl, p-(Phenylazo)benzyl, 2,4,6-Tri-t-butylphenyl, 4-(Trimethylammonium)benzyl und 2,4,6-Trimethylbenzyl.

b) Harnstoffderivate wie
Phenothiazinyl-(10)-carbonyl Derivate, N'-p-Toluolsulfonylaminocarbonyl und N'-Phenylaminothiocarbonyl.

c) Amide wie
N-Formyl, N-Acetyl, N-Chloroacetyl, N-Trichloroacetyl, N-Trifluoracetyl, N-Phenylacetyl, N-3-Phenylpropionyl, N-Picolinoyl, N-3-Pyridylcarboxamid, N-Benzoylphenylalanyl Derivate, N-Benzoyl und N-p-Phenylbenzoyl.

Bevorzugt sind Verbindungen der Formel I, in denen

$R^1$ CN, COOH, eine mit einer Schutzgruppe geschützte COOH-Gruppe oder $C_1$-$C_4$-Alkanoyl bedeutet und die übrigen Reste die vorstehenden Bedeutungen haben. Besonders bevorzugt sind Verbindungen der Formel I, worin die Reste folgende Bedeutungen haben:

$R^1$: CN, COOH, eine mit einer Schutzgruppe geschützte COOH-Gruppe oder $C_1$-$C_4$-Alkanoyl

$R^2$: O-$C_1$-$C_{10}$-Alkyl$(R^{11})_n$ (n=0,1,2), wobei der Alkylteil unverzweigt, verzweigt oder cyclisch ist, sowie eine der $CH_2$-Gruppen durch O ersetzt sein kann und $R_{11}$ mit $R_{12}$ substituiert sein kann und bei n=2 die beiden Reste $R^{11}$ gleich oder verschieden sind. O-$C_3$-$C_{10}$-Alkenyl$(R^{11})_n$ (n=0,1,2), wobei der Alkenylteil unverzweigt, verzweigt oder cyclisch ist, eine der $CH_2$-Gruppen durch O, S, SO, $SO_2$, oder $NR^8$ ersetzt sein kann sowie ein- oder mehrfach ungesättigt ist, und $R^{11}$ mit $R^{12}$ substituiert sein kann und bei n=2 die beiden Reste $R^{11}$ gleich oder verschieden sind, O-$C_3$-$C_{10}$-Alkinyl$(R^{11})_n$ (n=0,1,2), wobei der Alkinylteil unverzweigt, verzweigt oder cyclisch ist, ein- oder mehrfach ungesättigt ist sowie eine der $CH_2$-Gruppen durch O, S, SO, $SO_2$, oder $NR^8$ ersetzt sein kann und $R^{11}$ mit $R^{12}$ substituiert sein kann und bei n=2 die beiden Reste $R^{11}$ gleich oder verschieden sind,

$R^3$ bis $R^{13}$ die vorstehend angegebenen Bedeutungen haben,

X: $(CH_2)_m$, (m=0,1,2,3,4) CH=CH, C≡C, $CH_2OCH_2$, $CH_2SCH_2$

Y: $(CH_2)_m$, (m=0,1,2,3,4) O, S, $NR^8$,

Z: $(CH_2)_m$, (m=0,1,2,3,4) S, O, S-$C_1$-$C_{10}$-Alkyl, (unverzweigt oder verzweigt), CH=CH, CH=CF, CH=CCl, CH=CBr, $CH_2$-C(O), $CH_2$-CHF, $CH_2$-CHCl, $CH_2$-CHBr, $CH_2$-CHJ, $C_3$-$C_{10}$-Cycloalkylen, $C_3$-$C_{10}$-Cycloalkenylen, $COOR^7$, C≡C, CH=C($C_1$-$C_4$-Alkyl) (unverzweigt oder verzweigt), CH=C(CN), CH=C($R^{13}$), $NR_8$.

Die erfindungsgmäßen Verbindungen der Formel I können, soweit sie eine Carboxylgruppe enthalten, Salze mit anorganischen oder organischen Basen bilden. Bevorzugt sind Salze mit anorganischen Basen, besonders die physiologisch unbedenklichen Alkalimetall-Salze, vor allem Natrium- und Kaliumsalze.

Die Verbindungen der Formel I hemmen das Glucose-6-Phosphatase-System der Leber in Säugetieren. Die Verbindungen eignen sich daher als Arzneimittel. Die Erfindung betrifft daher auch Arzneimittel auf Basis der Verbindungen der Formel, gegebenenfalls in Form der physiologisch verträglichen Salze.

Die Erfindung betrifft weiterhin die Anwendung von Verbindungen der Formel I oder der Salze zur Behandlung von Krankheiten, die mit einer erhöhten Aktivität des Glucose-6-Phosphatase-Systems verbunden sind.

Die Erfindung betrifft auch die Anwendung von Verbindungen der Formel I bzw. der Salze zur Behandlung von Krankheiten, die mit einer erhöhten Glucoseproduktion der Leber verbunden sind.

Die Erfindung betrifft außerdem die Anwendung von Verbindungn der Formel I bzw. der Salze zur Behandlung des Typ II Diabetes (nicht insulinabhängiger oder Altersdiabetes).

Die Erfindung beinhaltet weiterhin die Anwendung von Verbindungen der Formel I bzw. der Salze zur Herstellung von Arzneimitteln zur Behandlung des Diabetes und anderer Erkrankungen, die durch einen erhöhten Glucose-Ausstoß aus der Leber oder eine erhöhte Aktivität des Glucose-6-Phosphatase-Systems gekennzeichnet sind.

Die Wirkung der erfindungsgemäßen Verbindungen auf das Glucose-6-Phosphatase-Sytem wurde in einem Enzymtest in Lebermikrosomen untersucht.

Für die Präparation der die Glucose-6-Phosphatase enthaltenden Mikrosomen-Fraktion wurden frische Leber-Organe von männlichen Wistar-Ratten verwendet und wie in der Literatur beschrieben aufgearbeitet [Canfield, W. K. und Arion, W. J., J. Biol. Chem. 263, 7458-7460, (1988)]. Diese Mikrosomen-Fraktion kann bei -70 °C mindestens 2 Monate lang ohne signifikanten Aktivitätsverlust aufbewahrt werden. Der Nachweis der Glucose-6-Phosphatase-Aktivität wurde wie in der Literatur angegeben (Arion, W. J. in Methods Enzymol. 174, Academic Press 1989, Seite 58-67) durch Bestimmung des aus Glucose-6-Phosphat freigesetzten Phosphats durchgeführt. 0,1 ml Testansatz enthielten Glucose-6-Phosphat (1 mMol/l), die Testsubstanz, 0,1 mg Mikrosomen-Fraktion und 100 mMol/l HEPES-Puffer (4-(2-Hydroxyethyl)piperazin-1-ethansulfonsäure), pH 7,0. Die Reaktion wurde durch Zugabe des Enzyms gestartet. Nach Ablauf von 20 min bei Raumtemperatur wurde die Reaktion durch Zugabe von 0,2 ml Phosphat-Reagens gestoppt. Die Probe wurde 30 min lang bei 37 °C inkubiert, und die Absorption (A) der blauen Farbe anschließend bei 570 nm gemessen. Die inhibitorische Wirksamkeit der Testsubstanz ergab sich durch Vergleich mit einer Kontroll-Reaktion, die keine Testsubstanz enthielt nach der Formel

$$\text{Prozent Hemmung} = \frac{\text{A(Kontrolle)-A(Testsubstanz)}}{\text{A(Kontrolle)}} \times 100$$

Sofern erforderlich wurde die hemmende Wirkung der Testsubstanz als Funktion der eingesetzten Konzentration der Testsubstanz ermittelt, und daraus die Konzentration für die 50 %ige Hemmung der Enzymaktivität ($IC_{50}$) berechnet.

Für die nachfolgend aufgeführten Verbindungen wurde der $IC_{50}$-Wert ermittelt:

Beispiel 1

[1S,3R,4R,5S]-3-[(E)-3-(4-Hydroxyphenyl)propenoyl]oxy-4,5-dihydroxy-1-phenylmethyloxy-cyclohexancarbonsäure:
$IC_{50}$= 190 µM

Beispiel 2

[1S,3R,4R,5S]-3-[(E)-3-(4-Hydroxyphenyl)propenoyl]oxy-4,5-dihydroxy-1-(2-thienylmethyl)oxy-cyclohexancarbonsäure:
$IC_{50}$= 110 µM

Beispiel 3

[1S,3R,4R,5S]-3-[(E)-3-(4-Hydroxyphenyl)propenoyl]oxy-4,5-dihydroxy-1-(2-propinyl)oxy-cyclohexancarbonsäure:
$IC_{50}$= 560 µM

Beispiel 8

[1S,3R,4R,5S]-3-[(E)-3-(4-Hydroxyphenyl)propenoyl]oxy-4,5-dihydroxy-1-propyloxy-cyclohexancarbonsäure:
$IC_{50}$= 230 µM

Beispiel 4

[1S,3R,4R,5S]-1-(4-Chlorphenylpropyl)oxy-4,5-dihydroxy-3-(2-pyridincarbonyl)oxy-cyclohexancarbonsäure:
$IC_{50}$= 26 µM

Beispiel 44

[1S,3R,4R,5S]-1-(4-Chlorphenylpropyl)oxy-3-[(E)-3-(4-hydroxyphenyl)propoyl]oxy-4,5-dihydroxy-cyclohexancarbonsäure:
$IC_{50}$= 9.3 µM

Tabelle

| Verbindung aus Beispiel | $IC_{50}$ [µm] |
|---|---|
| 69 | 170 |
| 113 | 3,7 |
| 114 | 5,0 |
| 115 | 8,9 |
| 116 | 4,5 |
| 117 | 41,0 |
| 118 | 1,3 |
| 119 | 12,0 |
| 120 | 0,69 |

Die Herstellung der erfindungsgemäßen Verbindungen der Formel I, in denen die Reste $R^2$ = O-Alkyl$(R^{11})_n$, O-Alkenyl$(R^{11})_n$ oder O-Alkinyl$(R^{11})_n$, $R^4$=$R^5$= OH und Y= O ist, kann auf den im folgenden Schema aufgezeigten Weg A erfolgen.

Verfahren A

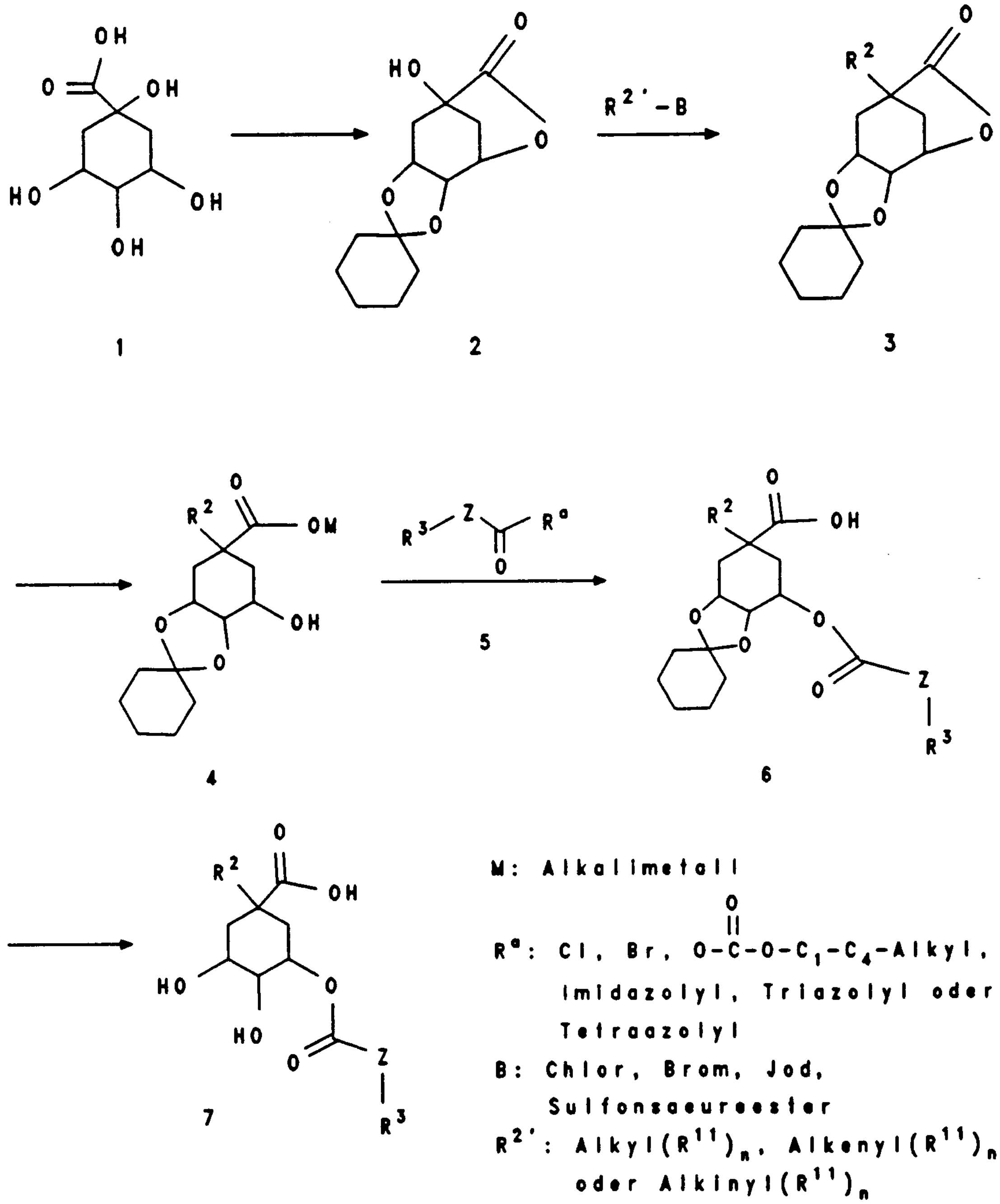


(7 = Formel I, $R^2$ = O-$C_1$-$C_{10}$-Alkyl$(R^{11})_n$, O-$C_3$-$C_{10}$-Alkenyl$(R^{11})_n$ oder O-$C_3$-$C_{10}$-Alkinyl$(R^{11})_n$, $R^4$ = $R^5$ = OH, $R^6$ = H, Y = O, X = $(CH_2)_m$ mit m = null, $R^1$ = COOH Z, $R^3$, $R^{11}$ und n wie zu Formel I angegeben).

Das Verfahren A ist dadurch charakterisiert, daß man die aus Verbindung 1 zugängliche literaturbekannte Verbindung 2 mit einer starken Base wie Kalium-tert.-butylat, Natriumhydrid oder Kaliumhydrid deprotoniert und zur Einführung von $R^2$ mit entsprechenden Halogeniden, Trifluorsulfonsäureestern, Methylsulfonsäureestern oder p-Toluolsulfonsäureestern vorteilhafterweise in polaren aprotischen Lösungsmittel wie Dimethylformamid, Dimethylsulfoxid oder Tetrahydrofuran umgesetzt, wobei Verbindung 3 entsteht. Bevorzugt wird die Verwendung von Natriumhydrid als Base und Dimethylformamid als Lösungsmittel.

Die Reaktion von 2 zu 3 wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Die bevorzugte Ausführungsform der Reaktion 2 zu 3 erfolgt in Dimethylformamid in Gegenwart von Natriumhydrid oder Kaliumhydrid bei Temperaturen von 0 bis 60°C. Die Reaktion wird dabei vorteilhafterweise unter Feuchtigkeitsausschluß unter einem Schutzgas (Stickstoff oder Argon) durchgeführt.

Die für die Umsetzung von 2 nach 3 notwendigen Ausgangsmaterialien, die dem Rest $R^2$ entsprechen, lassen sich nach dem für den Fachmann bekannten Standardverfahren darstellen. Hierbei handelt es sich um Strukturen des Typs $R^2$-B mit der für Verfahren A genannten Einschränkung (allerdings ohne das verknüpfende Sauerstoffatom). B hat z. B. die Bedeutung einer Abgangsgruppe wie Cl, Br, J, oder $OSO_2R$ (R= $CH_3$, Ph, Tolyl, $CF_3$).

Statt der Cyclohexyliden-Schutzgruppe in 2 bzw. 3 können auch andere unter milden saueren Bedingungen abspaltbaren Schutzgruppen wie Isopropyliden-, oder Benzylidenacetale sowie tert.-Butyl, Methoxymethyl, 1-Ethoxyethyl oder Tetrahydropyranylether, Silylether wie Trimethylsilyl- oder tert.-Butyldimethylsilyl oder Carbonate wie Benzyloxycarbonyl- und tert.-Butoxycarbonylderivate, die aus der Peptid- und Steroidchemie wohlbekannt sind, verwendet werden. Die Herstellung solcher geschützter Verbindungen geht ebenfalls von Verbindung 1 aus.

Weitere Schritt bei Verfahren A ist die Hydrolyse des Lactons 3 zum Alkalisalz 4 mit Alkalihydroxiden wie Lithiumhydroxid, Natriumhydroxid oder Kaliumhydroxid. Die Reaktion erfolgt vorteilhafterweise in protischen oder aprotischen Lösungsmitteln wie niederen Alkoholen, Tetrahydrofuran oder Dioxan, bevorzugt ist die Verwendung von Dioxan.

Die Reaktion von 3 zu 4 wird bei Temperaturen von -20°C bis zum Siedepunkt des verwendeten Lösungsmittels durchgeführt. Bevorzugt ist ein Temperaturbereich von -10 bis 60°C, besonders von 0 bis 30°C.

Weiterer Schritt ist die Reaktion von 4 zu 6, bei der der Rest $R^3$-Z-C(O)- an 4 angebunden wird. Dazu wird 4 in einem aprotischen organischen Lösungsmittel wie z.B. Tetrahydrofuran, Dimethylformamid, Dichlormethan, Pyridin oder Dimethylsulfoxid mit einer Verbindung $R^3$-Z-C(O)-$R^a$ (5) umgesetzt, wobei $R^a$ z.B. Cl, Br, OC(O)-$C_1$-$C_4$-Alkyl, Imidazolyl, Triazolyl oder Tetraazolyl sein kann, wobei Imidazolyl und Triazolyl besonders bevorzugt sind. Besonders bevorzugt ist die Ausführung der Reaktion in Dimethylformamid in Gegenwart einer Base wie z.B. Natriumhydrid, Kaliumhydrid, 4-Dialkylaminopyridin oder tert. Aminen, besonders aber von Natriumhydrid.

Die Reaktion 4 zu 6 wird bei Temperaturen von -20°C bis zum Siedepunkt des verwendeten Lösungsmittels durchgeführt. Bevorzugt ist ein Temperaturbereich von - 10 bis 60°C, besonders von 0 bis 30°C.

Die Verbindungen $R^3$-Z-C(O)-$R^a$ (5) lassen sich nach dem Fachmann bekannten Standardverfahren darstellen.

Eine bevorzugte Ausführungsform der Reaktion 4 zu 6 besteht in der Umsetzung von 4 mit Natriumhydrid in Dimethylformamid und anschließender Zugabe einer Lösung von $R^3$-Z-C(O)-Imidazol (5) in Dimethylformamid bei 0 bis 20°C, vorteilhafterweise unter Ausschluß von Feuchtigkeit unter Schutzgas (Argon oder Stickstoff).

Die Abspaltung der Schutzgruppe bei der Reaktion 6 zu 7 erfolgt in allgemein bekannter Weise, z. B. durch Behandeln mit verdünnten anorganischen Säuren wie z.B. Salzsäure oder starken organischen Säuren wie z.B. Trifluoressigsäure in inerten organischen Lösungsmittel, wie cyclischen Ethern, optional in Gegenwart von Wasser. bei Temperaturen von -20°C bis zum Siedepunkt des Lösungsmittels, bevorzugt von 0 bis 30°C.

Die erhaltenen erfindungsgemäßen Verbindungen der Formel I können, soweit sie eine Carboxylgruppe enthalten, Salze mit anorganischen oder organischen Basen bilden. Bevorzugt sind daher auch solche Salze mit anorganischen Basen, besonders die physiologisch unbedenklichen Alkalimetall-Salze, vor allem Natrium- und Kaliumsalze.

Aus den Alkalimetallsalzen der Verbindungen der Formel I mit einer Carboxylgruppe können die für $R^1$ angegebenen Ester dargestellt werden. Hierzu wird Verbindung 7 in einem inerten organischen Lösungsmittel wie Tetrahydrofuran, Dimethylsulfoxid, bevorzugt Dimethylformamid bei -10 bis 60°C z. B. mit einem $C_1$-$C_4$-Alkylhalogenid, bevorzugt $C_1$-$C_4$-Alkyliodid, Benzylbromid, oder $C_1$-$C_4$-Alkanoyl-O-CH($R^7$)-Br oder $C_1$-$C_4$-Alkanoyl-O-CH($R^7$)-J zu den erfindungsgemäßen Verbindungen der Formel I mit einer Estergruppe als $R^1$ und X=$(CH_2)_m$ m=0 mit den zum Verfahren A genannten Details umgesetzt.

Verfahren B (Definition für M, $R^a$ und B: s. Verfahren A)

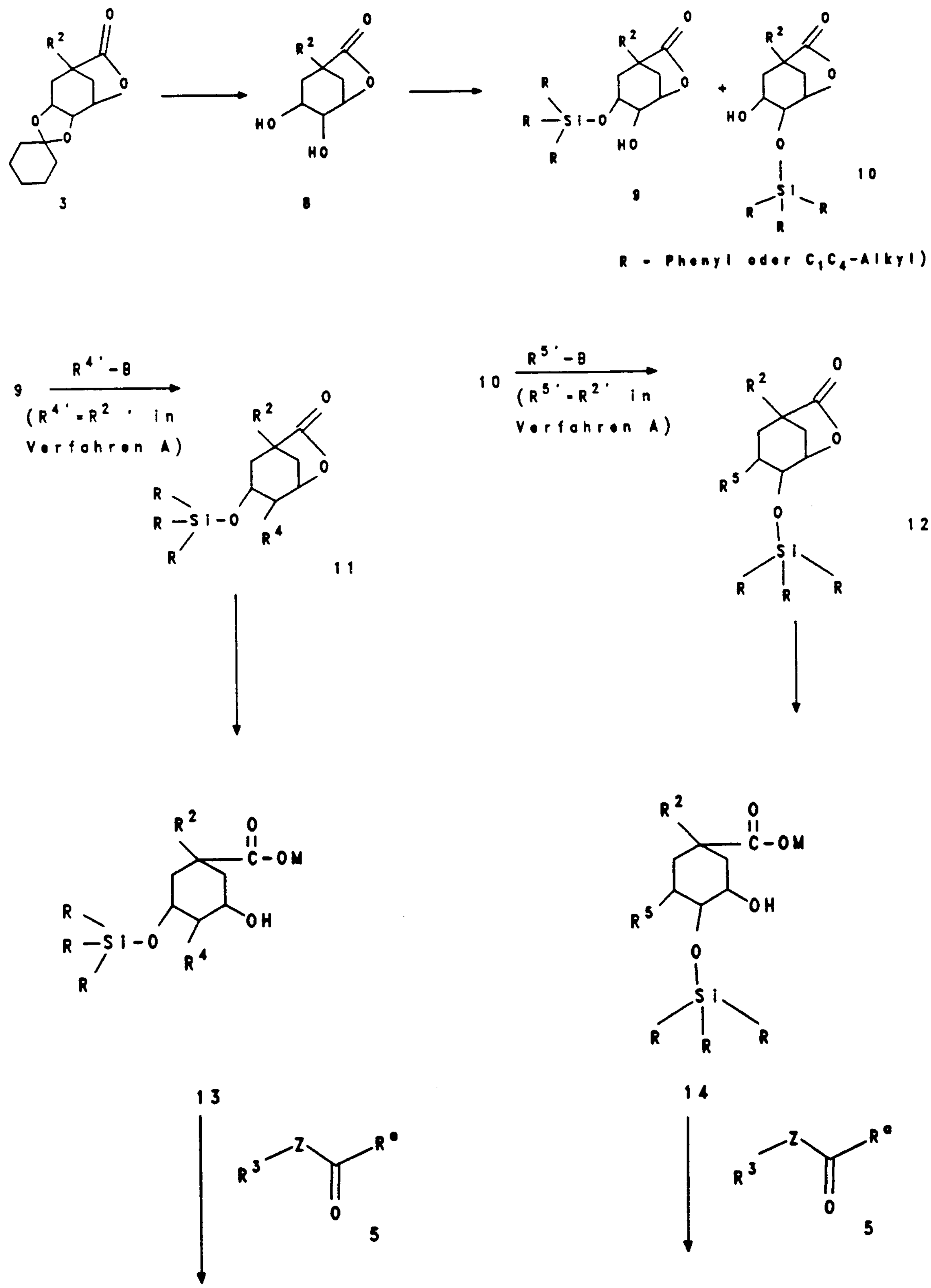


3, 8, 9, 10, 11, 12, 13, 14, 5, 5

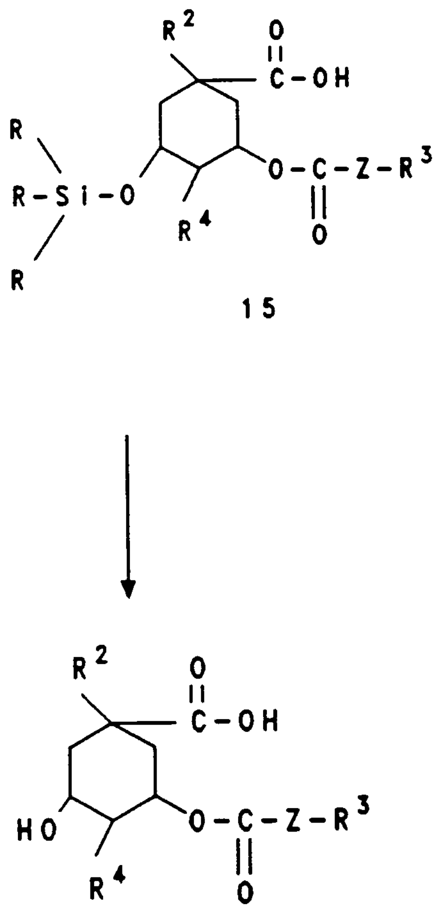

15

17

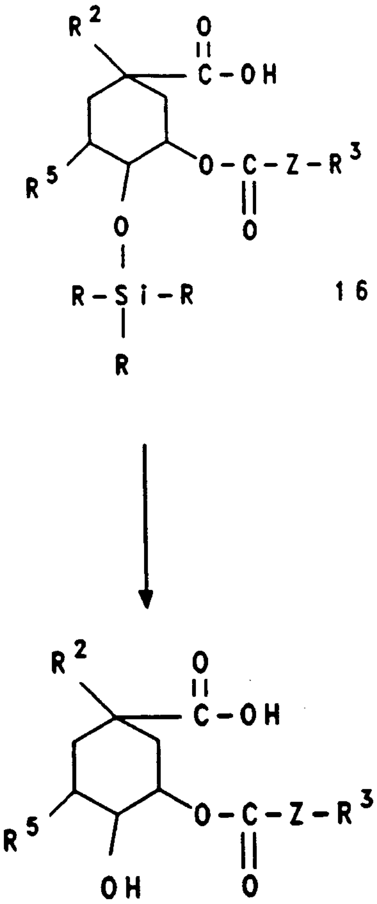

16

18

(17 und 18 = Formel I mit $R^2$ = O-$C_1$-$C_{10}$-Alkyl$(R^{11})_n$, O-$C_3$-$C_{10}$-Alkenyl$(R^{11})_n$ oder O-$C_3$-$C_{10}$-Alkinyl$(R^{11})_n$, $R^4$ in der für $R^2$ angegebenen Bedeutung und $R^5$ = OH bzw. $R^4$ = OH und $R^5$ in der für $R^2$ angegebenen Bedeutung, $R^6$ = H, Y = O, X = $(CH_2)_m$ mit m = null, $R^1$ = COOH, Z, $R^3$ und $R^{11}$ sowie n wie zu Formel I angegeben)

Um die Reste $R^4$ und $R^5$ zu variieren wird das Verfahren B angewendet. Dabei ist es notwendig die Cyclohexyliden-Schutzgruppe in 3 zur Verbindung 8 abzuspalten. Dies kann nach einem dem Fachmann bekannten Standardverfahren erfolgen. Bevorzugt ist dabei die Hydrolyse von 3 in inerten organischen Lösungsmitteln wie niederen Alkoholen in Gegenwart von starken organischen Säuren wie Sulfonsäuren, z.B. p-Toluolsulfonsäure oder Trifluoressigsäure.

Die Reaktion 3 zu 8 wird z. B. bei Temperaturen von -20°C bis zum Siedepunkt des verwendeten Lösungsmittels durchgeführt. Bevorzugt ist ein Temperaturbereich von -10 bis + 60°C, besonders von 20 bis 50°C.

Besonders bevorzugt ist die Umsetzung von 3 zu 8 in Isopropanol in Gegenwart von p-Toluolsulfonsäure bei 40°C.

Ein für das Verfahren B charakteristischer Schritt ist die Differenzierung der beiden freien Hydroxylgruppen in 8. Hierzu wird Verbindung 8 mit sterisch anspruchsvollen Trialkylsilylhalogeniden, wie z.B. tert.-Butyldimethylsilylchlorid, tert.-Butyldiphenylsilylchlorid oder Triisopropylsilylchlorid in einem inerten organischen Lösungsmittel, besonders Dimethylformamid, bei Temperaturen zwischen -10 und 40°C in gegenwart einer Base, besonders Imidazol, zu den Verbindungen 9 und 10 umgesetzt, die sich chromatographisch trennen lassen.

Die Verbindungen 9 und 10 können analog den Umsetzungen von 2 nach 7 aus Verfahren A umgesetzt werden, so daß die für Formel I angegebenen Variationen für die Reste $R^4$ und $R^5$ nach diesem Verfahren möglich sind.

Verfahren C (Definition für $R^a$, s. Verfahren A)

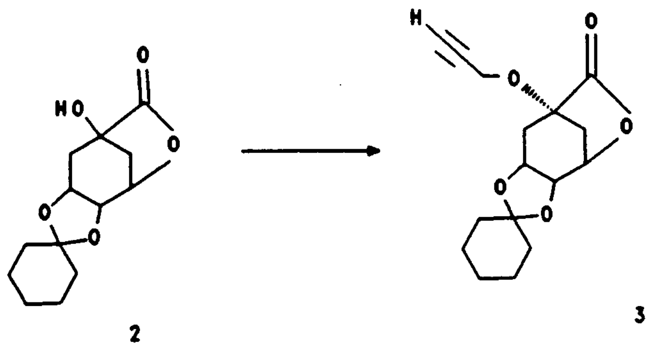

2

3'

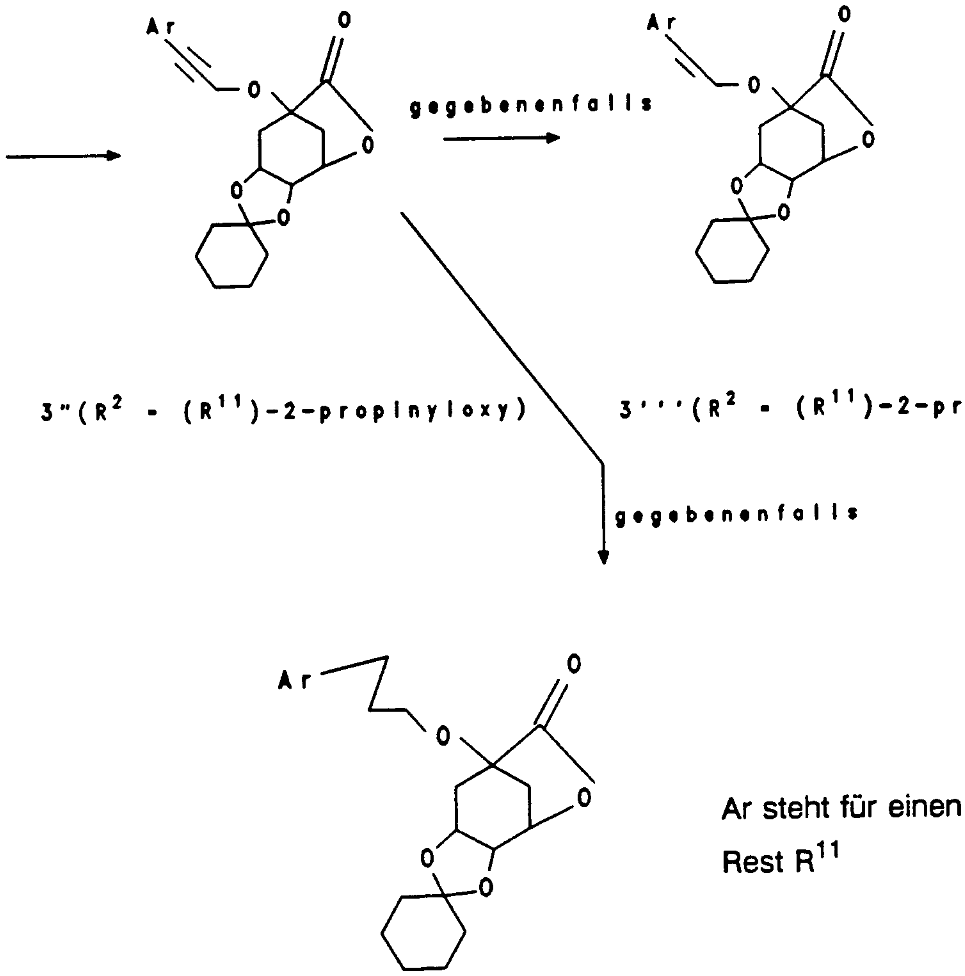


3''($R^2$ = ($R^{11}$)-2-propinyloxy)

3'''($R^2$ = ($R^{11}$)-2-propenyloxy)

$3^{IV}$($R^2$= ($R^{11}$)propyloxy)

Ar steht für einen aromatischen Rest $R^{11}$

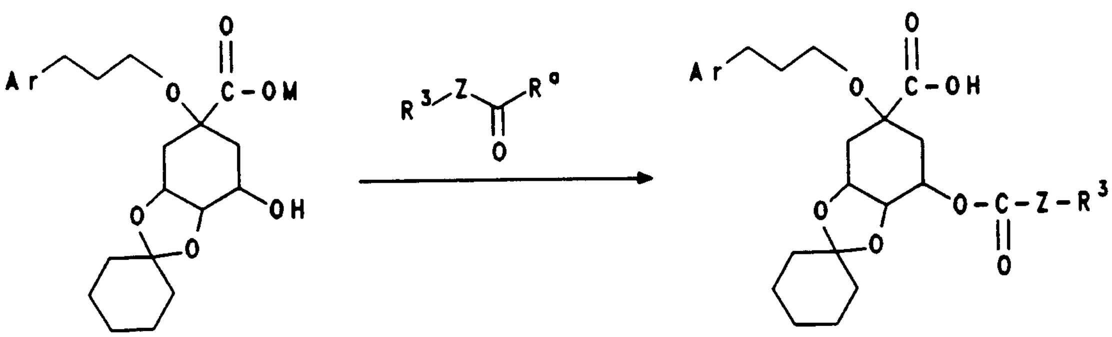

4'

6'

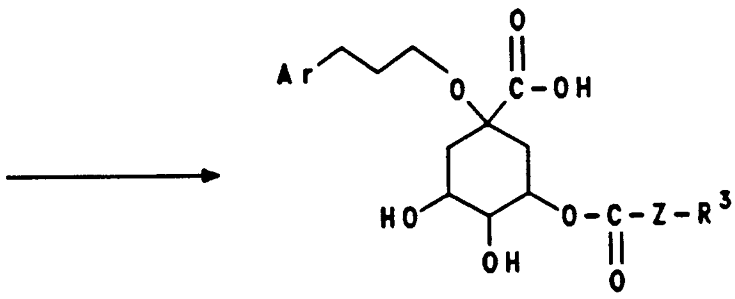

7'

(7' = Formel I mit $R^2$ = O-$C_3$-Alkyl$(R^{11})_n$ mit n = 1 und $R^{11}$ aromatischer Rest wie zu Formel I angegeben, $R^4$, $R^5$ = OH, $R^6$ = H, Y = O, X = $(CH_2)_m$ mit m = Null und $R^1$ = COOH, Z und $R^3$ wie zu Formel I angegeben, wobei Verbindungen mit $R^2$ = O-$C_3$-Alkenyl$(R^{11})_n$ bzw. O-$C_3$-Alkinyl$(R^{11})_n$ erhalten werden, wenn die gegebenenfalls erfolgende Hydrierung zu 3''' bzw. $3^{IV}$ unterbleibt).

Ein alternatives Verfahren zu A für eine Reihe erfindungsgemäßer Verbindungen der Formel I stellt das Verfahren C dar. Für $R^2$ = O-$C_3$-Alkinyl$(R^{11})$ kann die Zwischenstufen 3', bei der $R^2$= 2-Propinyloxy entspricht, dienen. Hierbei wird 3' ($R^2$= 2-Propinyloxy) in einem inerten organischen Lösungsmittel wie z.B. Toluol, Benzol oder n-Heptan unter Katalyse eines Palladium-Komplexes und Kupfer(I)halogenid, besonders Kupfer(I)iodid, mit einem Arylhalogenid, besonders Arylbromid oder Aryliodid, zu 3'' ($R^2$= $R^{11}$-2-popinyloxy) umgesetzt. Hierzu muß eine Base wie z. B. primäre, sekundäre oder tertiäre Amine, besonders Triethylamin, zugegeben werden. Optional kann die Base auch gleichzeitig als Lösungsmittel dienen und auf den Zusatz eines weieteren organischen Lösungsmittels verzichtet werden.

Die Reaktion 3' ($R^2$= 2-Propinyloxy) zu 3'' ($R^2$= $R^{11}$-2-popinyloxy) wird bei Temperaturen von -20°C bis zum Siedepunkt des verwendeten Lösungsmittels durchgeführt. Bevorzugt ist ein Temperaturbereich von 20 bis 90°C, besonders von 60 bis 80°C.

Als Palladium-Komplex kann zum Beispiel der in situ aus Palladiumdichlorid und Triphenylphosphin darstellbare Komplex Ditriphenylphosphinpalladiumdichlorid oder der in gleicher Weise aus Palladium(II)acetat zugängliche Komplex Ditriphenylphosphinpalladiumdiacetat dienen, bevorzugt ist Ditriphenylphosphinpalladiumdichlorid.

Aus 3'' ($R^2$= $R^{11}$-2-popinyloxy) lassen sich durch Hydrierkatalysatoren gezielt 3''' ($R^2$= $R^{11}$-2-popenyloxy) oder $3^{IV}$ ($R^2$= $R^{11}$-2-popyloxy) darstellen. Die Reaktionen werden in Ethanol oder Pyridin unter Wasserstoffatmosphäre bei Normaldruck durchgeführt.

Die Reaktion 3'' ($R^2$= $R^{11}$-2-popinyloxy) zu 3''' ($R^2$= $R^{11}$-2-popenyloxy) wird mit einem Katalysator Palladium auf Bariumsulfat bei Temperaturen von 0°C bis zum Siedepunkt des verwendeten Lösungsmittels durchgeführt. Bevorzugt ist Pyridin als Lösungsmittel und ein Temperaturbereich von 20 bis 50°C, besonders von 20 bis 30°C .

Die Reaktion 3''' ($R^2$= $(R^{11})$-2-popinyloxy) zu $3^{IV}$ ($R^2$= $(R^{11})$propyloxy) wird mit Palladium auf Kohle als Katalysator in Ethanol bei Temperaturen von -20°C bis zum Siedepunkt des verwendeten Lösungsmittels durchgeführt. Bevorzugt ist ein Temperaturbereich von 20 bis 50°C, besonders von 20 bis 30°C .

Die weiteren Reaktionen von $3^{II}$-$^{IV}$ sowie von 4' zu 7', d.h. zu den Verbindungen der allgemeinen Formel I sind unter Verfahren A ausführlich beschrieben.

Verfahren D (Definition für $R^a$, s. Verfahren A)

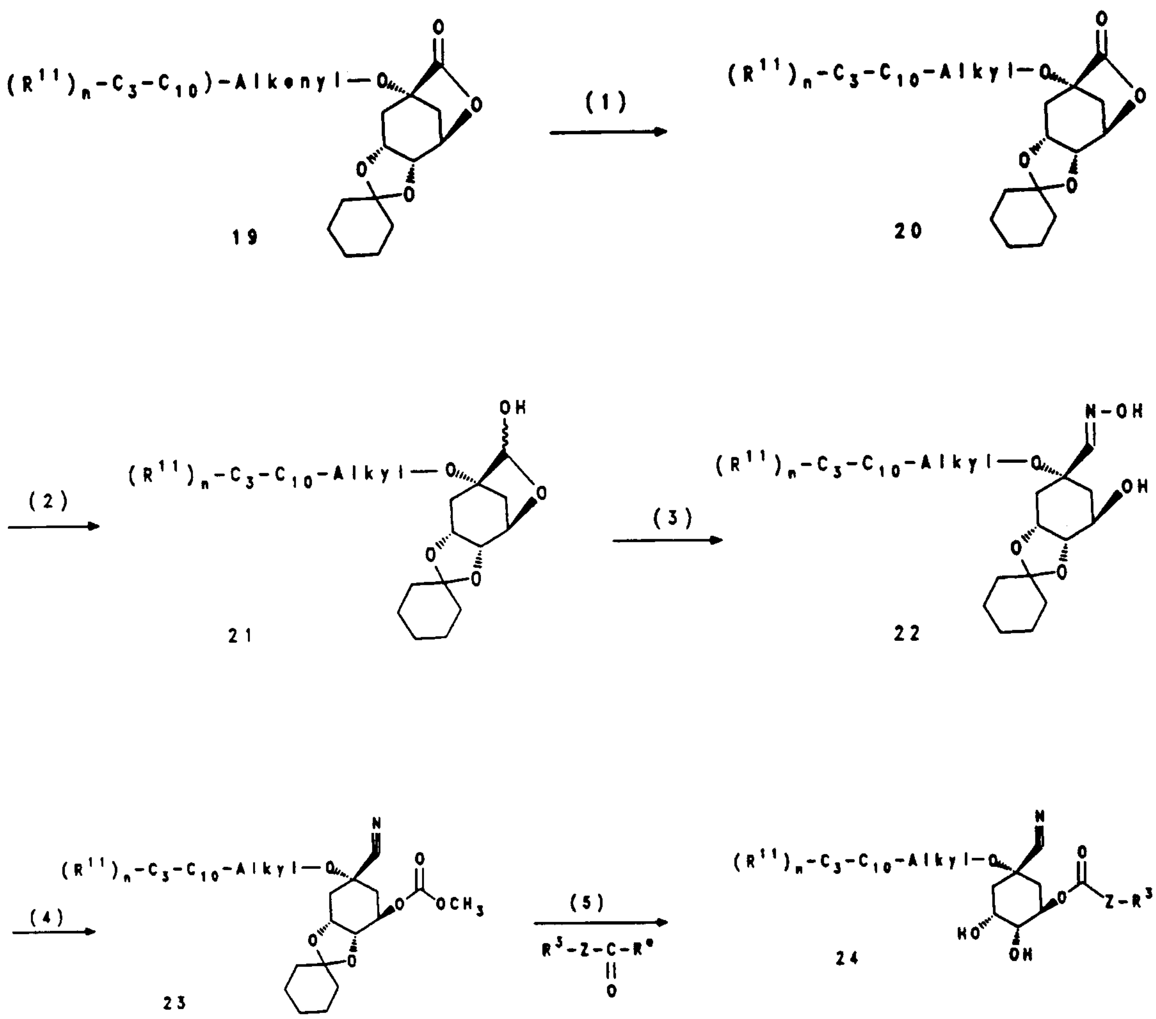


(24 = Formel I mit $R^1$ = CN, X = $(CH_2)_m$ mit m = null, $R^2$ = O-$C_3$-$C_{10}$-Alkyl-$(R^{11})_n$, mit n = null oder 1, $R^4$, $R^5$ = OH, $R^6$ = H, Y = O und $R^3$, $R^{11}$ und Z wie zu Formel I angegeben).

Die Durchführung des Verfahrens D entsprechend den Verfahrensschritten (1) bis (5) ist im Beispiel 68 erläutert.

Verfahren E (Definition für $R^a$, s. Verfahren A)

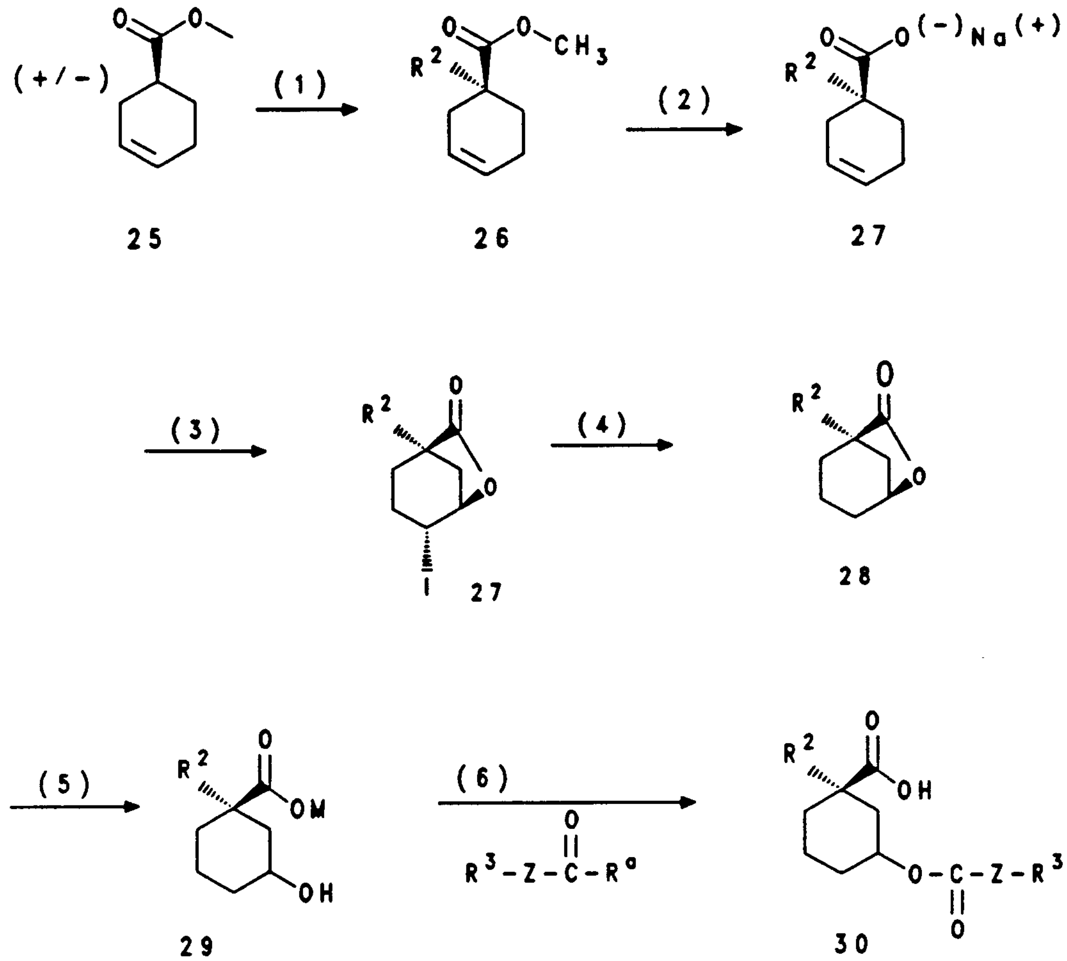


25 26 27

27 28

29 30

(30 = Formel I mit $R^2$ = $C_1$-$C_{10}$-Alkyl$(R^{11})_n$, $R^4$, $R^5$, $R^6$ = H.

Y = O, X = $(CH_2)_m$ mit m = null, $R^1$ = COOH, Z, $R^3$, $R^{11}$ und n wie zu Formel I angegeben).

Verfahren E ist geeignet für die Herstellung der im Beispiel 70 beschriebenen Verbindung. Diesem Beispiel sind geeignete Reaktionsbedingungen zu entnehmen.

Verfahren F (Defintion für $R^a$, s. Verfahren A)

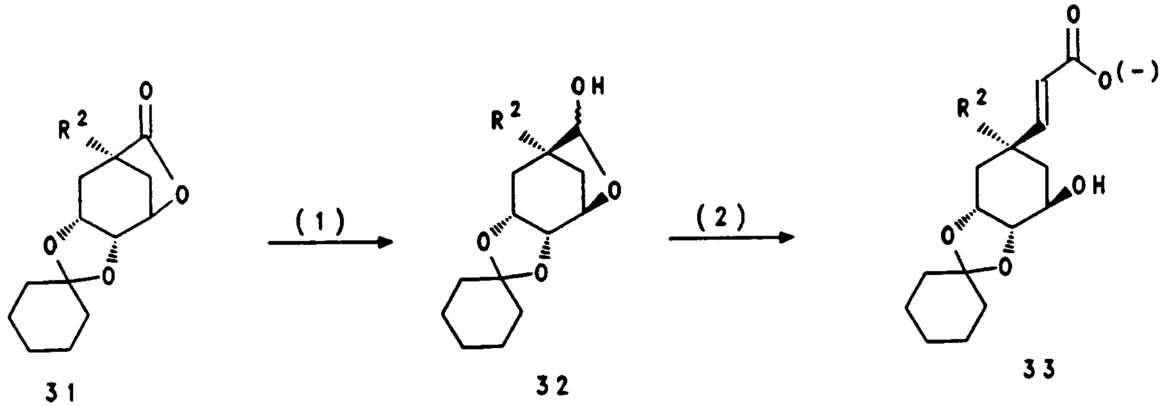


31 32 33

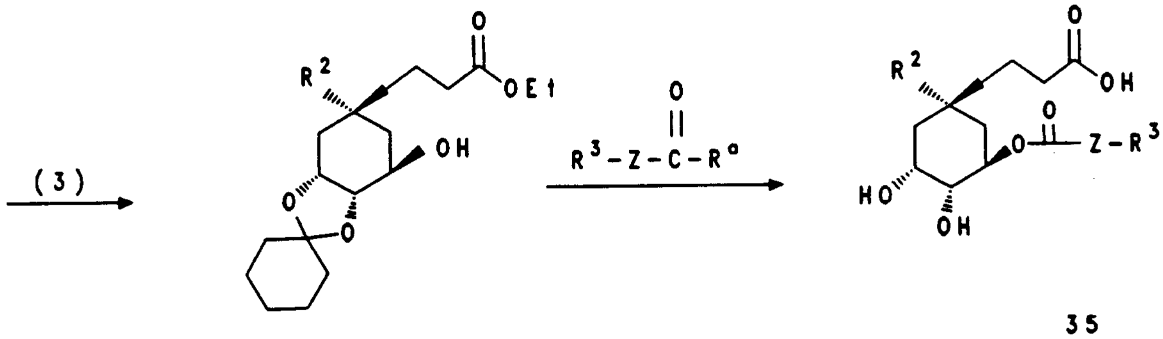


34 35

(35 = Formel I mit $R^1$ = COOH, X = $(CH_2)_m$ mit m = 3, $R^2$ = O-$C_1$-$C_{10}$-Alkyl$(R^{11})_n$, O-$C_3$-$C_{10}$-Alkenyl$(R^{11})_n$ oder O-$C_3$-$C_{10}$-Alkinyl$(R^{11})_n$, $R^4$, $R^5$ = OH, $R^6$ = H und Z, $R^3$, $R^{11}$ und n wie zu Formel I angegeben).

Das Verfahren ist in Beispiel 63 erläutert.

Die Ausgangsverbindungen für die Verfahren A bis F sind bekannt bzw. können analog literaturbekannten Methoden hergestellt werden oder sind nach den in der Anmeldung beschriebenen Verfahren erhältlich.

Die Herstellung der Verbindung 5 (vergl. Verfahren A), die z.B. bei der Synthese der Verbindungen der Beispiele 68, 78, 82, 96, 97 und 98 eingesetzt wird, erfolgt zweckmäßigerweise nach Verfahren I, II, III wie nachfolgend beschrieben.

Verfahren I

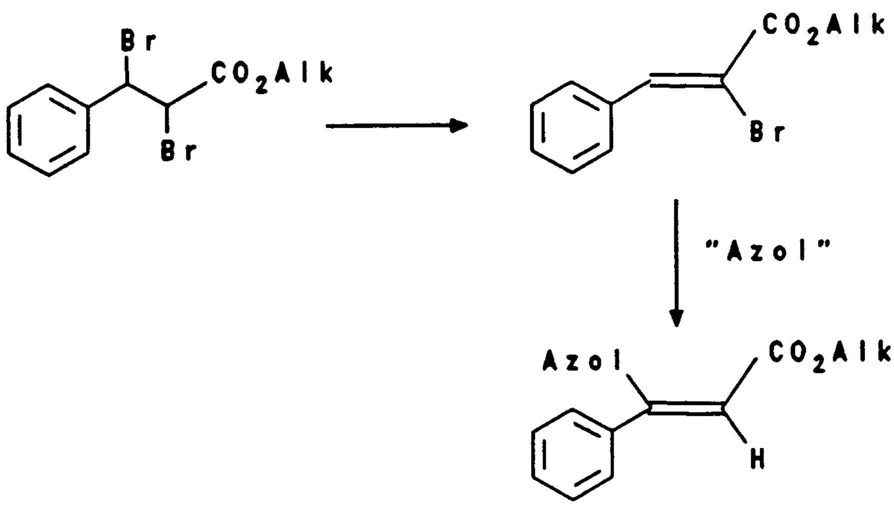


Verfahren II

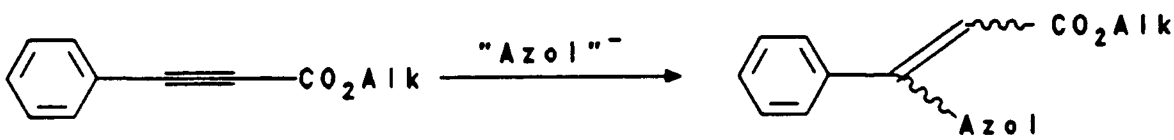


Verfahren III

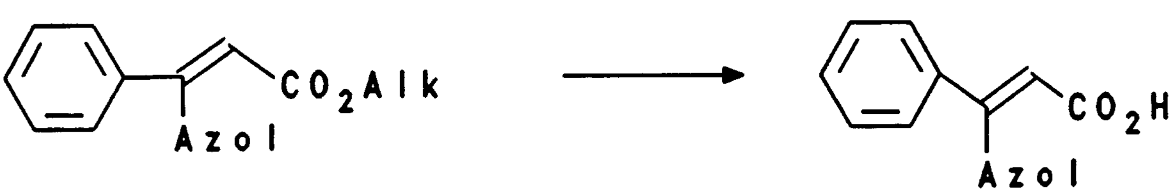

Alk steht für $C_1$-$C_4$-Alkyl

Azol steht für $R^{13}$ in der Bedeutung Imidazolyl, Indolyl, Piperazinyl, Tetrazolyl, Triazolyl oder deren thieno-, pyridino-, pyrimidino- oder benzoannelierte Derivate.

Verfahren I

Herstellung β-Azol-substituierter Zimtsäuremethylester

Ein Gemisch von 50 g 2,3-Dibrom-3-phenylpropansäuremethylester, 100 ml Triethylamin und 500 ml Toluol wird 1 h zum Sieden erhitzt, anschließend auf Raumtemperatur gekühlt und filtriert. Das Filtrat wird im Vakuum eingedampft und die so erhaltene α-Bromzimtsäure ohne Reinigung weiterverwendet. Zu einer Suspension von 4,7 g NaH (80 %ig in Mineralöl) in 100 ml wasserfreiem DMF werden 0,2 mol des Azol-Derivats, gelöst in 150 ml wasserfreiem DMF, unter Rühren zugetropft. Die Temperatur des Gemisches wird dabei durch Kühlung mit Eis unter 35°C gehalten. Nach beendeter Zugabe wird 1 h bei Raumtempertur gerührt. Die zuvor hergestellte α-Bromzimtsäure wird in 200 ml wasserfreiem DMF gelöst und unter Kühlung mit Eis die Lösung des Azol-Natriumsalzes unter Rühren zugetropft. Nach zweistündigem Rühren bei Raumtemperatur werden 10,8 ml Eisessig zugegeben, das Gemisch wird in 1,5 l Eiswasser eingerührt, mehrfach mit Ethylacetat extrahiert und die organischen Phasen werden mit Wasser gewaschen. Die organischen Phasen werden getrocknet, im Vakuum eingedampft und der Rückstand wird durch Säulenchromatographie an Kieselgel (Laufmittel: n-Heptan/Ethylacetat) oder Umkristallisieren gereinigt.

Verfahren II

Herstellung β-Azol-substituierter Zimsäureethylester

Ein Gemisch aus 20 g Phenylpropiolsäureethylester, 0,11 mol Azol-Derivat und 15 ml wasserfreiem DMF wird bei Raumtemperatur unter Argon-Begasung gerührt. Ein Spatel NaH (80 %ig in Mineralöl) wird zugegeben. Wenn die Wasserstoffentwicklung beendet ist, wird auf 100-150°C (Badtemperatur) erwärmt und die Reaktion mit DC (Laufmittel n-Heptan/Ethylacetat) verfolgt. Nach beendeter Reaktion wird auf Raumtemperatur abgekühlt, im Vakuum eingeengt und der Rückstand aus n-Heptan umkristallisiert oder mit wenig n-Heptan/Ethylacetat verdünnt und durch Säulenchromatographie an Kieselgel (Laufmittel: n-Heptan/Ethylacetat) gereinigt.

Verfahren III

Herstellung β-Azol-substituierter Zimtsäuren aus β-Azol-substituierten Zimtsäureestern

In einer Lösung von 0,77 g NaOH in 50 ml Wasser und 10 ml Methanol werden 6,4 mmol β-Azol substituierter Zimtsäuremethyl- oder ethylester suspendiert und das Gemisch bei Raumtemperatur gerührt, bis im DC (Laufmittel n-Heptan/Ethylacetat) vollständiger Umsatz erkennbar ist und eine klare Lösung entstanden ist. Diese wird im Vakuum eingeengt, mit ca. 50 ml Wasser verdünnt und unter Eiskühlung mit 2 N HCl auf pH 2-3 gestellt. Falls ein Feststoff ausfällt, wird er abgesaugt und im Vakuum getrocknet. Andernfalls wird mit $CH_2Cl_2$ mehrfach extrahiert, die organischen Phasen werden getrocknet, im Vakuum eingedampft und der Rückstand wird durch Umkristallisieren oder Chromatographie an Kieselgel (Laufmittel: n-Heptan/Ethylacetat/Eisessig) gereinigt.

Ein weiterer Gegenstand der Erfindung sind Arzneimittel, die ein oder mehrere erfindungsgemäße Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen ( = Wirkstoff) entweder als solche, oder vorzugsweise in Kombination mit geegneten pharmazeutischen Hilfsstoffen in Form von Tabletten, Dragees, Kapseln, Suppositorien, Emulsionen, Suspensionen, Granulaten, Pulvern, Lösungen oder Präparate mit protalierter Wirkstoff-Freigabe, eingesetzt, wobei der Wirkstoffgehalt vorteilhafterweise 0,1 bis 95 % beträgt.

Welche Hilfstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidatien, Dispergiermittel, Emulgatoren, Entschäumer, Geschmackskorrigentien, Konservierungsmittel, Lösungsvermittler oder Farbstoffe verwendet werden.

Die Wirkstoffe können topisch, oral, parenteral oder intravenös appliziert werden, wobei die bevorzugte Applikationsart von der zu behandelnden Krankheit abhängig ist. Die orale Verabreichung ist bevorzugt.

Für eine orale Anwendungsform werden die aktiven Verbindungen mit den dafür geeigneten Zusatzstoffen wie Trägerstoffen, Stabilisatoren oder inerten Verdünndungsmittel vermischt und durch üblichen Methoden in geeigneten Dareichungsformen gebracht, wie Tabletten, Dragees, Steckkapseln, wässrige, alkoholische oder ölige Suspensionen oder wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus verschiedenen Lösungsmitteln.

Als pharmazeutische Präparate für topische und lokale Verwendung eignen sich Augentropfen, welche die aktive Verbindung in wässriger oder öliger Lösung enthalten. Für die Anwendung an der Nase sind Aerosole und Sprays, sowie grobe Puder, die durch schnelles Inhalieren durch die Nasenlöcher verabreicht werden, und vor allem Nasentropfen, welche die aktive Verbindungen in wässriger oder öliger Lösung enthalten, geeignet.

Die Dosierung des zu verabreichenden Wirkstoffs der Formel I und die Häufigkeit der Verabreichung hängen von der Wirkstärke und Wirkdauer der verwendeten erfindungsgemäßen Verbindung ab; außerdem auch von der Art und Stärke der zu behandelnden Krankheit sowie von Geschlecht, Alter, Gewicht und individuellen Ansprechbarkeit des zu behandelnden Säugers. Im Durchschnitt liegt die empfohlene tägliche Dosis einer erfindungsgemäßen Verbindung bei einem etwa 75 Kg schweren Säuger - in erster Linier einem Menschen - im Bereich von etwa 10 bis 500 mg, vorzugweise von etwa 25 bis 250 mg, wobei die Verabreichung nach Bedarf in mehreren Dosen pro Tag erfolgen kann.

Die nachfolgenden Beispiele sollen die vorliegende Erfindung illustrieren, ohne sie jedoch in ihren Umfang einzuschränken.
wfr bedeutet wasserfrei; Raumtemperatur steht für etwa 18 bis 25° C

## Beispiel 1

Darstellung von 1L-(1(OH),3,4-O-Cyclohexyliden-5-tetrahydroxycyclohexancarbonsäure 1,5-Lacton 2 aus D-Chinasäure 1:

163.3 g (0.85 mol) 1 wurden in 186 ml (1.8 mol) Cyclohexanon suspendiert. Es wurden 0.5 ml konz. Schwefelsäure zugesetzt. Anschließend erhitzte man langsam auf 200°C Heizbad-Temperatur und destillierte ein Wasser/Cyclohexanon-Azetrop ab. Nachdem kein Azetrop mehr überdestillierte, wurde die hellbraune Reaktionslösung weitere 2 h bei 200°C Badtemperatur gerührt. Danach ließ man die Reaktionslösung auf 70°C abkühlen unf gab 10 g Natriumhydrogencarbonat zu. Anschließend versetzte man mit 700 ml Essigsäureethylester, wusch die organische Phase mit Wasser und gesättigter Natriumchlorid-Lösung. Danach engte man die organische Phase im Vakuum ein. Den hellgelben Rückstand kristallisierte man aus Isopropanol/Wasser 1:1 und erhielt 142.1 g (75%) Lacton 2 als farblose Kristalle. F.p.: 140-141°C.

Darstellung von [1R,2R,3R,5S-1,2-O-Cyclohexyliden-5-Phenylmethyloxy-3,5-lactonylcyclohexan-1,2-diol (3, $R^2$=O-$CH_2$Ph)

0.81 g (28 mmol) Natriumhydrid (80%ig in Mineralöl) wurden unter Argon in 14 ml wfr. Dimethylformamid suspendiert und bei 0°C wurden 7.1 g (28 mmol) Alkohol 2 gelöst in 16 ml wfr. Dimethylformamid zugetropft. Man rührte anschließend 1 h bei 25°C und gab danach wieder bei 0°C 3.5 ml Benzylbromid zu. Man ließ die Reaktionsmischung 4 h bei Raumtemperatur rühren und versetzte danach bei 0°C mit gesättigter Ammoniumchlorid-Lösung. Es wurde mit Essigester extrahiert, die vereinigten organischen Phasen wurden mit gesättigter Natriumchlorid-Lösung gewaschen und mit Natriumsulfat getrocknet. Nach dem Einengen im Vakuum wurde der Rückstand aus n-Heptan/Methyl-tert.-butylether (5:1) umkristallisiert. Man erhielt 7.18 g (75%) Benzylether 3 ($R^2$=O-$CH_2$Ph) als farblose Kristalle. F.p.: 122-126°C.

Darstellung von [1S,3R,4R,5S]-3-Hydroxy-4,5-O-cyclohexyliden-1-phenylmethyloxy-cyclohexancarbonsäurenatriumsalz 4 ($R^2$=O-$CH_2$Ph) aus 3 ($R^2$=O-$CH_2$Ph):

3.1 g (9 mmol) Lacton 3 ($R^2$=O-$CH_2$Ph) wurden in 20 ml Dioxan gelöst und bei Raumtemperatur wurden 9.5 ml 1 N Natriumhydroxid-Lösung zugegeben. Man ließ die Emulsion 4 Stunden bei Raumtemperatur rühren und engte anschließend im Vakuum ein und trocknete den farblsoen Rückstand 24 h bei 60°C im Hochvakuum über Kaliumhydroxid. Man erhielt 3.32 g (96%) Natriumsalz 4 ($R^2$=O-$CH_2$Ph) als farblosen Feststoff. F.P.: 276-279°C (Zersetzung).

Darstellung von [1S,3R,4R,5S]-3-[(E)-3-(4-(Trimethylsilylethoxymethoxyphenyl)propenoyl]oxy-4,5-O-cyclohexyliden-1-phenylmethyloxy-cyclohexancarbonsäure 6 ($R^2$=O-$CH_2$Ph) aus 4 ($R^2$=O-$CH_2$Ph):

a) Darstellung von
(E)-3-(4-Trimethylsilylethoxymethoxyphenyl)-2-propensäureimidazolid 5
($R^a$= Imidazolyl)

a) 1.62 g (5.5 mmol) (Trimethylsilylethoxymethoxyphenyl)propensäure 5($R^a$=OH, Z = CH=CH, $R^3$(geschützt = 4-(Trimethylsilylethoxymethoxyphenyl)) wurden in 10 ml wfr. Dimethylformamid gelöst. Bei Raumtemperatur wurde eine Lösung von 0.92 g (5.5 mmol) Carbonyldiimidazol gelöst in 10 ml wfr. Dimethylformamid zugetropft. Anschließend erwärmte man diese Lösung 1 h auf 60-70°C, wobei eine $CO_2$-Entwicklung zu beobachten war.

b) 2.1 g (5.5 mmol) 4 ($R^2$=O-$CH_2$Ph) wurden in 20 ml wfr. Dimethylformamid gelöst. Unter Argon wurden bei 25°C 165 mg (5.5 mmol) Natriumhydrid (80%ig in Mineralöl) zugegeben. Man rührte 1 h bei 25°C. Anschließend wurde bei 0°C die unter a) dargestellte Lösung von 5 zugetropft. Nach 3 h bei 0 bis 5°C wurde die Reaktionsmischung auf gesättigte Ammoniumchlorid-Lösung gegeben, mit Essigester extrahiert und die vereinigten organischen Phasen wurden mit gesättigter Natriumchlorid-Lösung gewaschen sowie mit Magnesiumsulfat getrocknet. Nach dem

Einengen im Vakuum wurde der Rückstand an Kieselgel (Laufmittel: Essigester/n-Heptan/Eisessig 20:60:1) chromatographiert. Man erhielt 2.5 g (71%) Ester 6 ($R^2$=O-$CH_2$Ph, Z= CH=CH, $R^3$(geschützt) = 4-(Trimethylsilylethoxymethoxyphenyl)) als farbloses Öl.

Darstellung von [1S,3R,4R,5S]-3-[(E)-3-(4-Hydroxyphenyl)-2-propenoyl]oxy-4,5-dihydroxy-1-phenylmethyloxy-cyclohexancarbonsäure 7 ($R^3$= 4-Hydroxyphenyl, Z= CH=CH,
$R^2$= Phenylmethyloxy) aus 6:

2.7 g (7.0 mmol) 6 wurden in 130 ml Dioxan gelöst und bei Raumtemperatur unter Rühren mit 95 ml (0.19 mol) 2 N Salzsäure versetzt. Man rührte 20 h bei Raumtemperatur. Nach Ende der Reaktion wurde die klare Lösung mit 2 N Natronlauge auf pH 3-4 eingestellt und im Vakuum eingeengt. Der feste Rückstand wurde in Essigsäureethylester unter Erhitzen verrührt und das unlösliche Natriumchlorid abfiltriert. Das Filtrat engte man erneut ein und verrührte den Rückstand mit Methyl-tert.-butylether. Der Rückstand wurde abgesaugt und im Hochvakuum getrocknet. Man erhielt 2.0 g (70%) [1S,3R,4R,5S]-3-[(E)-3-(4-Hydroxyphenyl)-2-propenoyl]oxy-4,5-dihydroxy-1-phenylmethyloxy-cyclohexancarbonsäure 7 als farblosen Feststoff. F.p.: 209-212°C.

In analoger Weise wurden die folgenden Verbindungen dargestellt:

Beispiel 2

[1S,3R,4R,5S]-3-[(E)-3-(4-Hydroxyphenyl)-2-propenoyl]oxy-4,5-dihydroxy-1-(2-thienylmethyl)oxy-cyclohexancarbonsäure:
F.p.: 140°C

Beispiel 3

[1S,3R,4R,5S]-3-[(E)-3-(4-Hydroxyphenyl)-2-propenoyl]oxy-4,5-dihydroxy-1-(2-propinyl)oxy-cyclohexancarbonsäure:
F.p.:197°C

Beispiel 4

[1S,3R,4R,5S]-1-(4-Chlorphenlpropyl)oxy-4,5-dihydroxy-3-(2-pyridincarbonyl)oxy-cyclohexancarbonsäure:
F.p.: 128-130°C

Beispiel 5

[1S,3R,4R,5S]-1-(4-Chlorphenylpropyl)oxy-3-[(E)-3-(4-hydroxyphenyl)-2-propenoyl]oxy-4,5-dihydroxy-cyclohexancarbonsäure:
F.p.: 215-219°C

Beispiel 6

[1S,3R,4R,5S]-1-Methoxy-3-[(E)-3-(4-hydroxyphenyl)-2-propenoyl]oxy-4,5-dihydroxy-cyclohexancarbonsäure:
F.p.:242-243°C

Beispiel 7

[1S,3R,4R,5S]-1-Ethoxy-3-[(E)-3-(4-hydroxyphenyl)-2-propenoyl]oxy-4,5-dihydroxy-cyclohexancarbonsäure:
F.p.:227-228°C

Beispiel 8

[1S,3R,4R,5S]-3-[(E)-3-(4-Hydroxyphenyl)-2-propenoyl]oxy-4,5-dihydroxy-1-propyloxy-cyclohexancarbonsäure:
F.p.:221°C

Beispiel 9

[1S,3R,4R,5S]-1-(3-Phenylpropyl)oxy-3-[(E)-3-(4-hydroxyphenyl)-2-propenoyl]oxy-4,5-dihydroxy-cyclohexancarbonsäure:
F.p.: 203°C

Beispiel 10

[1S,3R,4R,5S]-1-(4-Chlorphenylmethyl)oxy-3-[(E)-3-(4-hydroxyphenyl)-2-propenoyl]oxy-4,5-dihydroxy-cyclohexancarbonsäure:
F.p.: 211°C

Beispiel 11

[1S,3R,4R,5S]-1-(4-Methylphenylmethyl)oxy-3-[(E)-3-(4-hydroxyphenyl)-2-propenoyl]oxy-4,5-dihydroxy-cyclohexancarbonsäure:
F.p.:198°C

Beispiel 12

[1S,3R,4R,5S]-1-(4-Trifluormethylphenylmethyl)oxy-3-[(E)-3-(4-hydroxyphenyl)-2-propenoyl]oxy-4,5-dihydroxy-cyclohexancarbonsäure:
F.p.:195-200°C

Beispiel 13

[1S,3R,4R,5S]-1-(4-Biphenylmethyl)oxy-3-[(E)-3-(4-hydroxyphenyl)-2-propenoyl]oxy-4,5-dihydroxy-cyclohexancarbonsäure:
F.p.: 222°C

Beispiel 14

[1S,3R,4R,5S]-1-(1-Naphtylmethyl)oxy-3-[(E)-3-(4-hydroxyphenyl)-2-propenoyl]oxy-4,5-dihydroxy-cyclohexancarbonsäure:
F.p.:165-170°C

Beispiel 15

[1S,3R,4R,5S]-1-(2-Naphtylmethyl)oxy-3-[(E)-3-(4-hydroxyphenyl)-2-propenoyl]oxy-4,5-dihydroxy-cyclohexancarbonsäure:
F.p.: 198°C

Beispiel 16

[1S,3R,4R,5S]-1-(3-Methoxyphenylmethyl)oxy-3-[(E)-3-(4-hydroxyphenyl)-2-propenoyl]oxy-4,5-dihydroxy-cyclohexancarbonsäure:
F.p.: 189-191°C

Beispiel 17

[1S,3R,4R,5S]-1-(4-Fluorophenylmethyl)oxy-3-[(E)-3-(4-hydroxyphenyl)-2-propenoyl]oxy-4,5-dihydroxy-cyclohexancarbonsäure:
F.p.: 214°C

Beispiel 18

[1S,3R,4R,5S]-1-(4-Cyanophenylmethyl)oxy-3-[(E)-3-(4-hydroxyphenyl)-2-propenoyl]oxy-4,5-dihydroxy-cyclohexancarbonsäure:
F.p.: 238-241°C

Beispiel 19

[1S,3R,4R,5S]-1-(3-(3-Methoxyphenyl)propyl)oxy-3-[(E)-3-(4-hydroxyphenyl)-2-propenoyl]oxy-4,5-dihydroxy-cyclohexancarbonsäure:
F.p.: 208-210° C

Beispiel 20

[1S,3R,4R,5S]-1-((E)-3-(4-Chlorphenyl)-2-propenyl)oxy-3-[(E)-3-(4-hydroxyphenyl)-2-propenoyl]oxy-4,5-dihydroxy-cyclohexancarbonsäure:
F.p.: 170-173°C

Beispiel 21

[1S,3R,4R,5S]-1-((3-Chlorphenyl)propyl)oxy-3-[(E)-3-(4-hydroxyphenyl)-2-propenoyl]oxy-4,5-dihydroxy-cyclohexancarbonsäure:
F.p.: 211°C

Beispiel 22

[1S,3R,4R,5S]-1-(4-Phenylbutyl)oxy-3-[(E)-3-(4-hydroxyphenyl)-2-propenoyl]oxy-4,5-dihydroxy-cyclohexancarbonsäure:
F.p.: 217°C

Beispiel 23

[1S,3R,4R,5S]-1-(3,3-Diphenylpropyl)oxy-3-[(E)-3-(4-hydroxyphenyl)-2-propenoyl]oxy-4,5-dihydroxy-cyclohexancarbonsäure:
F.p.: 155-160°C

Beispiel 24

[1S,3R,4R,5S]-1-(3-(4-tert.-Butylphenyl)methyl)oxy-3-[(E)-3-(4-hydroxyphenyl)-2-propenoyl]oxy-4,5-dihydroxy-cyclohexancarbonsäurenatriumsalz:
F.p.: 80-90°C

Beispiel 25

[1S,3R,4R,5S]-1-(3-(4-Chlor-2-methoxy-phenyl)propyl)oxy-3-[(E)-3-(4-hydroxyphenyl)-2-propenoyl]oxy-4,5-dihydroxy-cyclohexancarbonsäure:
F.p.: 190-194°C

Beispiel 26

[1S,3R,4R,5S]-1-(3-(5-Chlor-2-methoxy-phenyl)propyl)oxy-3-[(E)-3-(4-hydroxyphenyl)-2-propenoyl]oxy-4,5-dihydroxy-cyclohexancarbonsäure:
F.p.: 215-218°C

Beispiel 27

[1S,3R,4R,5S]-1-(3-(4-Chlorphenyl)2-propinyl)oxy-3-[(E)-3-(4-hydroxyphenyl)-2-propenoyl]oxy-4,5-dihydroxy-cyclohexancarbonsäure:
F.p.: 233-234°C

Beispiel 28

[1S,3R,4R,5S]-1-[3,3-Di(4-chlorphenyl)propyl]oxy-3-[(E)-3-(4-hydroxphenyl)-2-propenoyl]oxy-4,5-dihydroxy-cyclohexancarbonsäure:
F.p.: 122-126°C

Beispiel 29

[1S,3R,4R,5S]-1-((E)-3-(2-Chlorphenyl)-2-propenyl)oxy-3-[(E)-3-(4-hydroxyphenyl)-2-propenoyl]oxy-4,5-dihydroxy-cyclohexancarbonsäure:
F.p.: 192-196°C

Beispiel 30

[1S,3R,4R,5S]-1-(4-Phenoxybutyl)oxy-3-[(E)-3-(4-hydroxyphenyl)-2-propenoyl]oxy-4,5-dihydroxy-cyclohexancarbonsäure:
F.p.: 194-195°C

Beispiel 31

[1S,3R,4R,5S]-1-(3-(3,4-Dichlorphenyl)propyl)oxy-3-[(E)-3-(4-hydroxyphenyl)-2-propenoyl]oxy-4,5-dihydroxy-cyclohexancarbonsäure:
F.p.: 213-215°C

Beispiel 32

[1S,3R,4R,5S]-1-(4-(4-Chlorphenyl)butyl)oxy-3-[(E)-3-(4-methoxyphenyl)-2-propenoyl]oxy-4,5-dihydroxy-cyclohexancarbonsäure:
F.p.: 77-82°C

Beispiel 33

[1S,3R,4R,5S]-1-(3-(4-Chlorphenyl)propyl)oxy-3-[(E)-3-(4-methoxyphenyl)-2-propenoyl]oxy-4,5-dihydroxy-cyclohexancarbonsäure:
MS: m/e = 505 ($M+H^+$)

Beispiel 34

[1S,3R,4R,5S]-1-(3-(4-Chlorphenyl)propyl)oxy-3-[(E)-3-(2-methoxyphenyl)-2-propenoyl]oxy-4,5-dihydroxy-cyclohexancarbonsäure:
MS: m/e = 505 ($M+H^+$)

Beispiel 35

[1S,3R,4R,5S]-1-(4-(3-(2-Ethoxycarbonylthienyl)oxy)butyl)oxy-3-[(E)-3-(4-hydroxyphenyl)-2-propenoyl]oxy-4,5-dihydroxy-cyclohexancarbonsäure:
F.p.:144-147°C

Beispiel 36

[1S,3R,4R,5S]-1-(3-(2-Thienyl)propyl)oxy-3-[(E)-3-(4-hydroxyphenyl)-2-propenoyl]oxy-4,5-dihydroxy-cyclohexancarbonsäure:
F.p.: 211-213°C

Beispiel 37

[1S,3R,4R,5S]-1-(2-Thienyl)methyl)oxy-3-[(E)-3-(4-hydroxyphenyl)-2-propenoyl]oxy-4,5-dihydroxy-cyclohexancarbonsäure:
F.p.: 140°C (Z.)

Beispiel 38

[1S,3R,4R,5S]-1-(2-Thienyl)methyl)oxy-3-[(E)-3-(3-methoxythienyl)-2-propenoyl]oxy-4,5-dihydroxy-cyclohexancarbonsäure:
MS: m/e = 455 ($M+H^+$)

Beispiel 39

[1S,3R,4R,5S]-1-(3-(3-Thienyl)methyl)oxy-3-[(E)-3-(4-hydroxyphenyl)-2-propenoyl]oxy-4,5-dihydroxy-cyclohexancarbonsäure:
F.p.:156-160°C

Beispiel 40

[1S,3R,4R,5S]-1-[3-(2-(5-Chlor-thienyl))propyl]oxy-3-[(E)-3-(4-hydroxyphenyl)2-propenoyl]oxy-4,5-dihydroxycyclohexancarbonsäure:
F.p.: 215-218°C

Beispiel 41

[1S,3R,4R,5S]-1-[4-(3,5-Dimethyldithieno(3,2-b:3',2'-e)pyridinyl)butyloxy-3-[(E)-3-(4-hydroxyphenyl)-2-propenoyl]oxy-4,5-dihydroxy-cyclohexancarbonsäure:
F.p.:240-244°C

Beispiel 42

[1S,3R,4R,5S]-1-[(3,5-Dimethyldithieno(3,2-b:3',2'-e)pyridinyl)methyl]oxy-3-[(E)-3-(4-hydroxyphenyl)2-propenoyl]oxy-4,5-dihydroxy-cyclohexancarbonsäure:
F.p.: 240-244°C

Beispiel 43

[1S,3R,4R,5S]-1-(3-(3-Thienyl)propyl)oxy-3-[(E)-3-(4-hydroxyphenyl)2-propenoyl]oxy-4,5-dihydroxy-cyclohexancarbonsäure:
F.p.: 211-213°C

Beispiel 44

[1S,3R,4R,5S]-1-(4-Chlorphenylpropyl)oxy-3-[(E)-3-(4-hydroxyphenyl)propoyl]oxy-4,5-dihydroxy-cyclohexancarbonsäure:
F.p.: 157-159°C

Eine Reihe weiterer Verbindungen wurden nach dem Verfahren C dargestellt.

1. Alkylierung

30.0 g (0.118 mol) Lacton 2 wurden in 200 ml wfr. Dimethylformamid gelöst. Unter Argon-Atmosphäre wurden bei Raumtemperatur 5.3 g (0.176 mol) Natriumhydrid (80%ig in Mineralöl) zugegeben. Nach 1.5 h kühlte auf 0-10°C und tropfte über 30 min 20 ml (0.265 mol) Propargylbromid zu. Die Lösung färbt sich langsam dunkel. Nach 1 h (DC-Kontrolle) wurde der Reaktionsansatz auf halbgesätt. Ammoniumchlorid.Lsg. gegeben. Man extrahiert mit Essigester, wusch die organische Phase mit ges. Natriumchlorid-Lsg. und trocknete mit Magnesiumsulfat. Nach dem Einengen im Vakuum wurde der Rückstand über 1 Kg Kieselgel filtriert (Laufmittel: Essigester/n-Heptan 1:5. Man erhiet 30.0 g (87%) Propargylether 3' ($R^2$= Propinyloxy) als zähes Öl.

2. Stufe: Kopplung

24.0 g (0.082 mmol) Propargylether 3' ($R^2$= Propinyloxy) wurden in 150 ml wasserfreiem Toluol und 50 ml wasserfreiem Triethylamin gelöst. Unter Argon-Atmosphäre wurden nacheinander 0.354 g (0.002 mmol) Palladiumdichlorid, 1.05 g(0.004 mmol) Triphenylphosphin, 19.55 g (0.082 mol) 4-Chlor-iodbenzol und 0.050 g (0,0003 mmol) Kupfer(I)iodid zugegeben. Man erwärmte die Reaktionslösung langsam auf 80°C und beließ den Reaktionsansatz 4 Stunden bei dieser Temperatur. Anschließend kühlte man auf Raumtemperatur ab,filtrierte das entstandene Triethylamoniumhydrobromid ab und wusch den Niederschlag mit Essigester. Das Filtrat wurde im Vakuum eingeengt und der zähe ölige Rückstand durch Chromatographie an 1 kg Kieselgel gereinigt (Laufmittel: EE/n-Heptan 1:5; Rückstand zum Auftragen auf Kieselgel in wenig!! Essigester lösen). Man erhielt 23.0 g (69%) Phenylpropinylether 3" ($R^2$= 3-(4-Chlorphenyl)-2-propinyloxy), der sich aus Methylcyclohexan umkristallisieren ließ. F.p.: 79°C.

Darstellung von Alken 3''' ($R^2$= 3-(4-Chlorphenyl)-2-propenyloxy) aus Alkin 3" ($R^2$= 3-(4-Chlorphenyl)-2-propinyloxy):

12.0 g (29.8 mmol) Alkin 3" ($R^2$= 3-(4-Chlorphenyl)-2-propinyloxy) wurden in 300 ml Pyridin gelöst und 3.0 g Palladium auf Bariumsulfat (10% Palladium) zugegeben. Die Suspension wurde 4 h bei 25°C unter Wasserstoffatmosphäre geschüttelt. Nach der Beendigung der Wasserstoffaufnahme wurde der Katalysator abfiltriert und die Pyridin-Lösung im Vakuum eingeengt. Man erhielt 11.2 g (93%) Alken 3''' ($R^2$= 3-(4-Chlorphenyl)propenyloxy) als farblosen Feststoff. F.p.: 155-157°C.
Die weiteren Reaktionsschritte wurden analog dem Verfahren A durchgeführt (Schritte von 3 nach 7).

Darstellung von Alkan $3^{IV}$ ($R^2$= 3-(4-Chlorphenyl)propyloxy) aus Alkin 3''' ($R^2$= 3-(4-Chlorphenyl)propinyloxy):

6.0 g (14.9 mmol) Alkin 3 ($R^2$= 3-(4-Chlorphenyl)-2-propinyloxy) wurden in 50 ml Ethanol/Essigester (1:4) gelöst und 1.0 g Rhodium auf Aluminiumoxid (5% Rhodium) zugegeben. Die Reaktionsmischung wurde unter Wasserstoffatmosphäre bei Raumtemperatur ca. 15 h geschüttelt. Anschließend filtrierte man den Katalysator ab und engte das Filtrat im Vakuum ein. Man erhielt 6.05 g (100%) Alkan $3^{IV}$ ($R^2$= 3-(4-Chlorphenyl)propyloxy) als farbloses Öl.

Auch die so zugänglichen Alkane 3 wurden nach Verfahren A weiter umgesetzt (Schritte von 3 nach 7).

Beispiel 45

[1S,3R,4R,5S]-1-(3-(4-Fluorphenyl)propyl)oxy-3-[(E)-3-(4-hydroxyphenyl)2-propenoyl]oxy-4,5-dihydroxy-cyclohexancarbonsäure:
F.p.: 140-170°C

Beispiel 46

[1S,3R,4R,5S]-1-((Z)-3-(4-Chlorphenyl)-2-propenyl)oxy-3-[(E)-3-(4-hydroxyphenyl)2-propenoyl]oxy-4,5-dihydroxy-cyclohexancarbonsäure: F.p.:208-209°C

Beispiel 47

[1S,3R,4R,5S]-1-((Z)-3-(5-Pyrimidyl)-2-propenyl)oxy-3-[(E)-3-(4-methoxyphenyl)-2-propenoyl]oxy-4,5-dihydroxy-cyclohexancarbonsäure:
F.p.: 75-78°C

Beispiel 48

[1S,3R,4R,5S]-1-((Z)-3-(5-Pyrimidyl)-2-propenyl)oxy-3-[(E)-3-(4-methoxyphenyl)-2-propenoyl]oxy-4,5-O-Cyclohexyliden-cyclohexancarbonsäure:
F.p.: 165-167°C

Beispiel 49

[1S,3R,4R,5S]-1-((Z)-3-(2-Naphpthyl)-2-propenyl)oxy-3-[(E)-3-(4-methoxyphenyl)-2-propenoyl]oxy-4,5-dihydroxy-cyclohexancarbonsäure:
F.p.: 146-149°C

Beispiel 50

[1S,3R,4R,5S]-1-((Z)-3-(3-Trifluormethylphenyl)-2-propenyl)oxy-3-[(E)-3-(4-hydroxyl)-2-propenoyl]oxy-4,5-dihydroxy-cyclohexancarbonsäure:
F.p.: 187-190°C

Beispiel 51

[1S,3R,4R,5S]-1-(3-(4-Chlorphenyl)propyl)oxy-3-[(E)-3-(4-methoxyphenyl)-2-propenoyl]oxy-4,5-dihydroxy-cyclohexancarbonsäuremethylester:
MS: m/e = 505 ($M+H^+$)

Beispiel 52

[1S,3R,4R,5S]-1-(3-(4-Chlorphenyl)propyl)oxy-3-[(E)-3-phenyl-2-propenoyl]oxy-4,5-dihydroxy-cyclohexancarbonsäure
MS: m/e = 475 ($M+H^+$)

Beispiel 53

[1S,3R,4R,5S]-1-(3-(4-Chlorphenyl)propyl)oxy-3-[(E)-3-(3,4-dichlorphenyl-2-propenoyl]oxy-4,5-dihydroxy-cyclohexancarbonsäure
MS: m/e = 543 ($M+H^+$)

Beispiel 54

[1S,3R,4R,5S]-1-(3-(4-Chlorphenyl)propyl)oxy-3-[(E)-2-phenyl-1-cycloproplycarbonoyl]oxy-4,5-dihydroxy-cyclohexancarbonsäure
MS: m/e= 489 ($M+H^+$)

Beispiel 55

[1S,3R,4R,5S]-1-(3-(4-Chlorphenyl)propyl)oxy-3-[3-(4-hydroxyphenyl)propoyl]oxy-4,5-dihydroxy-cyclohexancarbonsäure
MS: m/e= 493 ($M+H^+$)

Beispiel 56

[1S,3R,4R,5S]-1-(3-(4-Chlorphenyl)propyl)oxy-3-[3-(4-methoxphenyl)propoyl]oxy-4,5-dihydroxy-cyclohexancarbonsäure
MS: m/e= 507 ($M+H^+$)

Beispiel 57

[1S,3R,4R,5S]-1-(2-(4-Chlorphenyl)-1-cyclopropylenmethyl)oxy-3-[(E)-3-(4-hydroxyphenyl)-2-propenoyl]oxy-4,5-dihydroxy-cyclohexancarbonsäure
F.p.: 195-199°C

Beispiele für Variation der Reste $R^4$ und $R^5$ nach Verfahren B

Darstellung von Lactondiol 8 ($R^2$= -O-CH-CH=CH-(p-Cl-phenyl), cis) aus 3 ($R^2$= - O-CH-CH=CH-(p-Cl-phenyl), cis):

11.0 g (27.7 mmol) Lacton 3 ($R^2$= -O-CH-CH=CH-(p-Cl-phenyl), cis) wurden in Isopropanol gelöst. Es wurden 40 ml 2 N Salzsäure zugegeben. Man ließ die Reaktionslösung 48 Stunden bei Raumtemperatur stehen und neutralisierte anschließend mit 1 N Natronlauge, engte im Vakuum ein und chromatographierte den Rückstand an Kieselgel. Man erhielt 7.8 g (90%) Lactondiol 8 ($R^2$= -O-CH-CH=CH-(p-Cl-phenyl), cis) als farblosen Feststoff. F.p.: 117-120°C

Darstellung der 5-tert.-Butyldmethylsilyloxy-Verbindung 9 ($R^2$= -O-CH-CH=CH-(p-Cl-phenyl), cis) aus Lactondiol 8 ($R^2$= -O-CH-CH=CH-(p-Cl-phenyl), cis):

5.0 g (15.4 mmol) Lactondiol 8 ($R^2$= -O-CH-CH=CH-(p-Cl-phenyl), cis) und 4.15 g (61.9 mmol) Imidazol wurden in 50 ml wfr. Dimethylformamid. Bei 0°C wurden 3.9 g (26 mmol) tert.-Butyldimethylsilylchlorid zugegeben. Nach 4 h wurde der Reaktionsansatz mit gesättigter Ammoniumchlorid-Lösung versetzt und mit Methyl-tert.-butylether extrahiert. Die vereinigten organischen Phasen wurden mit Magnesiumsulfat getrocknet und im Vakuum eingeengt. Der Rückstand wurde an Kieselgel (Laufmittel: Essigsäureethylester/n-Heptan 1:3) gereinigt. Man erhielt 5.7 g (84%) Silylether 9 ($R^2$ = -O-CH-CH=CH-(p-Cl-phenyl), cis) als farblosen Feststoff. F.p.: 71°C.

Wenn die Reaktion bei Raumtemperatur durchgeführt wird, kann auf diese Weise auch eine Mischung der beiden Silylether 9 und 10 erzeugt werden, die sich chromatographisch mit dem oben genannten Lösungsmittelgemisch an Kieselgel trennen lassen.

Darstellung von 3,3-Di(4-Chlorphenyl)-2-propenylether 11 ($R^2$= -O-CH-CH=CH-(p-Cl-phenyl), cis) aus 9 ($R^2$= -O-CH-CH=CH-(p-Cl-phenyl), cis).

1.0 g (2.3 mmol) Alkohol 9 ($R^2$= -O-CH-CH=CH-(p-Cl-phenyl), cis) wurden in 20 ml wasserfreiem Dimethylformamid gelöst. Unter Argon wurden bei Raumtemperatur 150 mg (5 mmol) Natriumhydrid (80%ig in Mineralöl) zugetropft und 1 h gerührt. Anschließend kühlte man auf 0°C ab und gab 0.85 g (3.2 mmol) 3,3-Di-(4-Chlorphenyl)-2-propenylbromid gelöst in 5 ml wasserfreiem Dimethylformamid zu und ließ die Reaktionsmischung nauf Raumtemperatur aufwärmen. Nach 14 h wurde der Reaktionsansatz mit gesättigter Ammoniumchlorid-Lösung versetzt und mit Methyl-tert.-butylether extrahiert. Die vereinigten organischen Phasen wurden mit Magnesiumsulfat getrocknet und im Vakuum eingeengt. Der Rückstand wurde an Kieselgel (Laufmittel: Essigsäureethylester/n-Heptan 1:3) gereinigt. Man erhielt 0.5 g (84%) Ether 11 ($R^2$= -O-CH-CH=CH-(p-Cl-phenyl), cis) als farbloses Öl.

11 wurde analog Verfahren A zur Verbindung des Beispiels 58 umgesetzt:

Beispiel 58

[1S,3R,4R,5S]-1-((Z)-3-(4-Chlorphenyl)-2-propenyl)oxy-3-[(E)-3-(4-hydroxyphenyl)propoyl]oxy-4-[3,3-Di(4-Chlorphenyl-2-propenyl]oxy-5-dihydroxy-cyclohexancarbonsäure
F.p.: 157-161°C

Analog dem Beispiel 58 wurde auch Beispiel 59 synthetisiert:

Beispiel 59

[1S,3R,4R,5S]-1-((Z)-3-(4-Chlorphenyl)-2-propenyl)oxy-3-[(E)-3-(4-hydroxyphenyl)propoyl]oxy-4-phenylmethyloxy-5-dihydroxy-cyclohexancarbonsäurenatriumsalz
$^1$H-NMR (270 MHz, $d_6$-DMSO): d=1.85-2.3 ppm (m 3H), 3.3-3.5 (m, 2H), 4..05-4.70 (m, 6 H), 5.2-5.38 (m, 1H), 5.82-5.93 (m, 1H), 6.3 (d, J=10.0 Hz, 1H), 6.42-6.5 (m, 1H), 6.75-6.85 (m, 2H), 7.2-7.55 (m, 12H), 11 ppm (1H).

In analoger Weise wie in Beispiel 1 beschrieben, wurden die Verbindungen der folgenden Beispiele hergestellt:

Beispiel 60

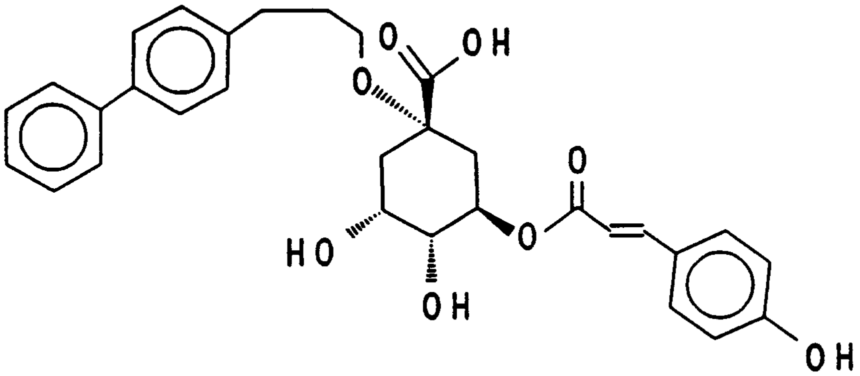

F.p.: 230°C

Beispiel 61

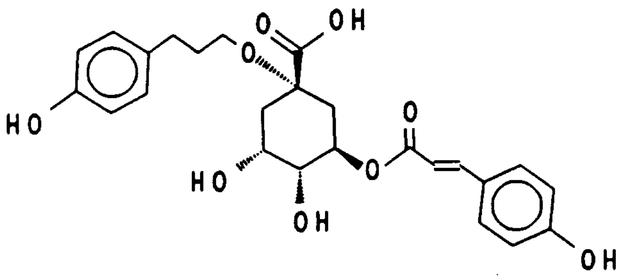

F.p.: 175-179°C

Beispiel 62

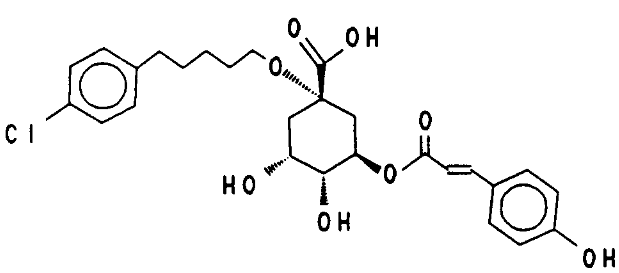

F.p.: 211-212°C

Beispiel 63

1) 30,0 g (73,7 mmol) 63A

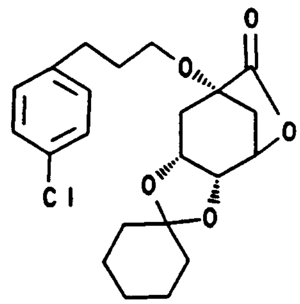

63A

wurden in 250 ml wßr. Toluol unter Argonatmosphäre bei -50 bis -60°C mit 68 ml 1.2 M Diisobutylaluminumhydrid versetzt. Nach 1 h wurden bei -60°C 50 ml einer Methanol/Wasser-Mischung (9:1) zugetropft. Die Reaktionsmischung wurde auf 0°C aufgewärmt. Anschließend gab man die Reaktionsmischung auf 1 N Kaliumhydrogensulfat-Lösung (pH ~ 4), extrahierte mit EE und trocknete die organische Phase mit Natriumsulfat. Nach dem Einengen erhielt man 30,0 g 63B

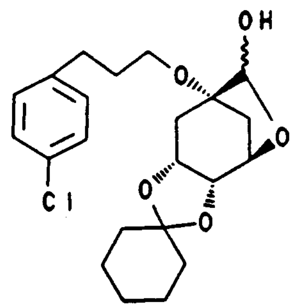

63B

das ohne Reinigung weiter umgesetzt wurde.

2) 19,8 g (88,1 mmol) Triethylphosphonacetat wurden in 200 ml wßr. Tetrahydrofuran gelöst. Unter Argonatmosphäre wurden bei 0 bis 5°C portionsweise 2,65 g 80 %iges Natriumhydrid zugegeben. Nach 20 min erhielt man eine klare bräunliche Lösung, zu der man bei -40 bis -50°C 30,0 g (73,4 mmol) 63B gelöst in 100 ml wßr. Tetrahydrofuran zutropfte. Nach 4 h bei -20 bis -30°C wurde die Reaktionsmischung auf gesättigte Ammoniumchlorid-Lösung gegeben, mit Essigester extrahiert und die vereinigten organischen Phasen wurden mit Natriumsulfat getrocknet und im Vakuum eingeengt. Man erhielt nach chromatographischer Reinigung des Rückstandes an Kieselgel 22,3 g 63C

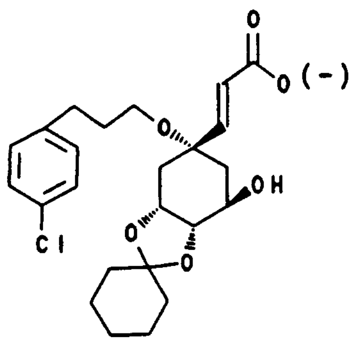

63C

als farbloses Öl.

3) 63C wurde nach dem Fachmann bekannten Verfahren in 63D

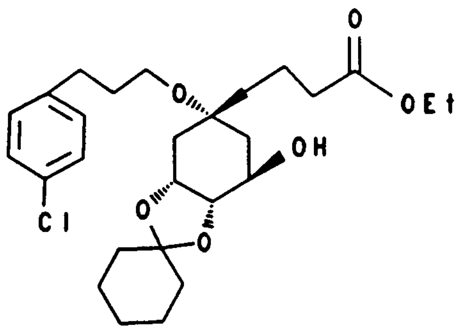

63D

überführt und analog Beispiel 1 (Stufe b und 6 → 7) zu 63 der Formel

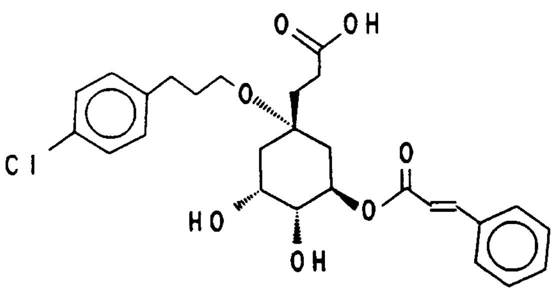

m/e = 503 ($M+H^+$)
weiter umgesetzt.

Analog Beispiel 1 wurden die Verbindungen der folgenden Beispiele hergestellt:

Beispiel 64

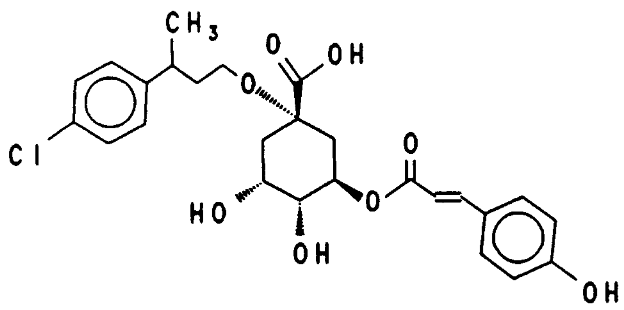

F.p.: 205-208°C

Beispiel 65

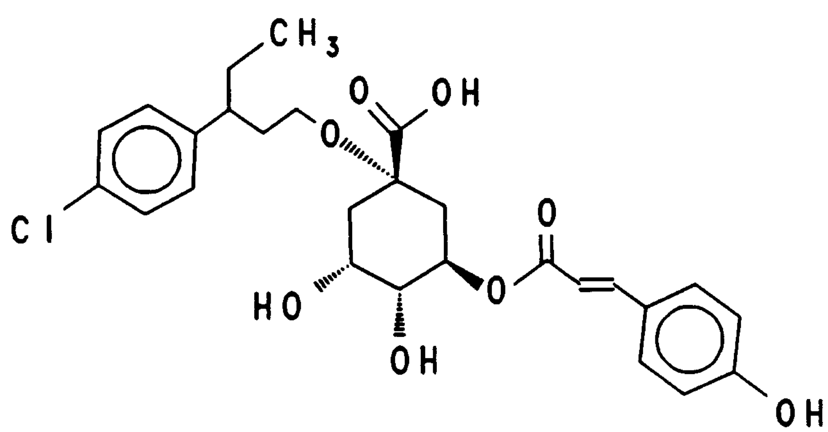

F.p.: 194-195°C

Beispiel 66

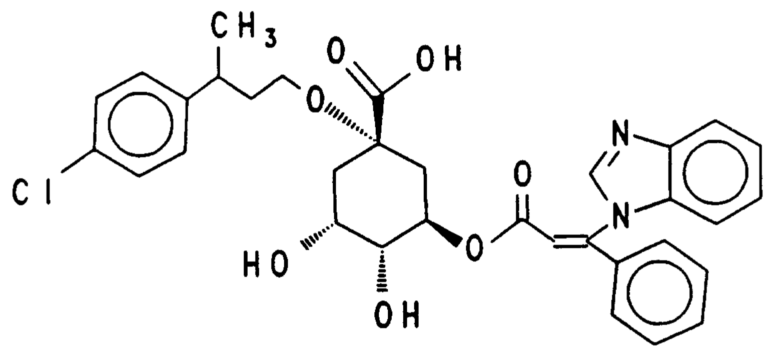

m/e = 605 (M+H⁺)

Beispiel 67

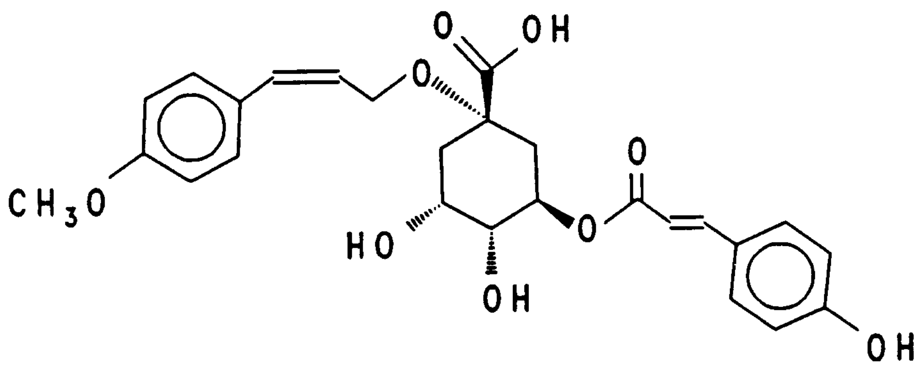

F.p.: 158-161°C

Beispiel 68

1) 15 ml 2 M Diethylzinklösung in Toluol wurden bei 0°C in 150 ml wäßriger Dichlormethan vorgelegt und unter Argonatmosphäre tropfte man 10,4 g (59,2 mmol) Chlorjodmethan zu und rührte bei 0-5°C 30 min. Anschließend tropfte man 6,0 g (14,8 mmol Olefin) 68A

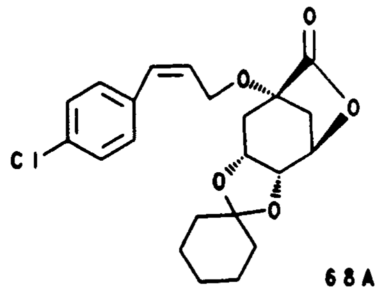

68A

gelöst in 50 ml wasserfreiem Dichlormethan zu. Man ließ die Reaktionslösung innerhalb von 2 h auf 25°C aufwärmen und hydrolisierte anschließend mit gesättigter Ammoniumchlorid-Lösung, extrahierte mit EE und engte im Vakuum ein. Man erhielt 5,5 g (91 %) Cyclopropanderivat 68B der Formeln

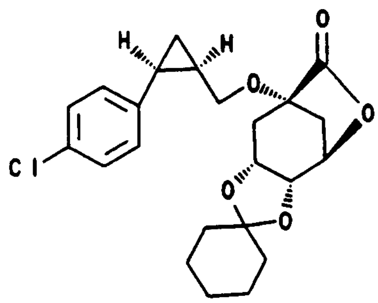

68B

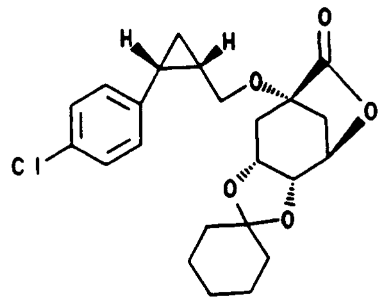

(3:1 Mischung der beiden möglichen Diastereomeren), die sich durch Kristallisation aus i-Propanol trennen ließen.

2) 2,0 g (4,8 mmol) Lacton 68B wurden in 50 ml wäßrigem Toluol unter Argonatmosphäre gelöst und bei -60°C wurden 4,1 ml 1,2 M Diisobutylaluminiumhydrid-Lösung in Toluol zugetropft. Man rührte 2 h bei -60°C und hydrolisierte die Reaktionslösung mit 10 ml $H_2O$. Man versetzte diese Mischung mit gesättigter Ammoniumchlorid-Lösung, extrahierte mit Essigester, trocknete mit Magnesiumsulfat und engte im Vakuum ein. Man erhielt 2,0 g (99 %) Lactol 68C der Formel

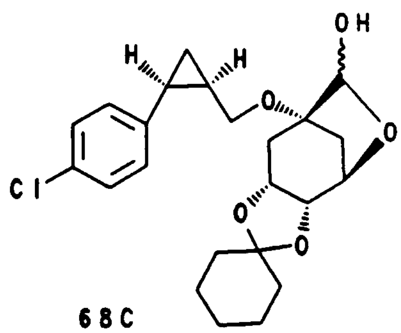

68C

als farbloses Öl.

3) 2,0 g (4,76 mmol) 68C wurden in 50 ml MeOH gelöst. Es wurde eine Lösung von 5,1 g Hydroxylaminhydrochlorid und 5,0 g Kaliumhydroxid in 50 ml MeOH bei 25°C zugetropft. Nach 2 Stunden Rühren bei 25°C wurde die Reaktionslösung auf Wasser gegeben und mit Methyl-tert.-butylether extrahiert. Die vereinigten organischen Phasen wurden mit Magnesiumsulfat getrocknet und im Vakuum eingeengt. Das Rohprodukt 68D der Formel

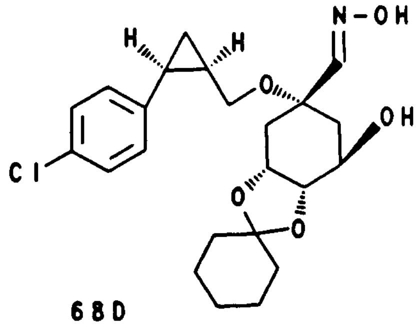

68 D

wurde ohne Reinigung weiter umgesetzt.

4. 2,1 g (4,8 mmol) 68 D wurden in 50 ml wäßrigem Dichlormethan vorgelegt und bei 25°C wurden 4,6 g (12,3 mmol) Carbonyldiimidazol zugegeben. Anschließend erwärmte man 3 h auf 40°C und gab nach beendeter $CO_2$-Entwicklung 100 ml wfr. Methanol zu und erhitzte erneut 4 h auf 40°C. Danach engte man im Vakuum ein, nahm den Rückstand in Methyl-tert.-butylether auf, wusch mit einer 0,1 N Kaliumhydrogensulfat-Lösung, und trocknete die organische Phase mit Magnesiumsulfat. Nach dem Einengen der organischen Phase wurde der Rückstand durch Chromatographie an Kieselgel (Laufmittel:Essigester/n-Heptan 1:4) gereinigt und man erhielt 1,3 g 68E der Formel

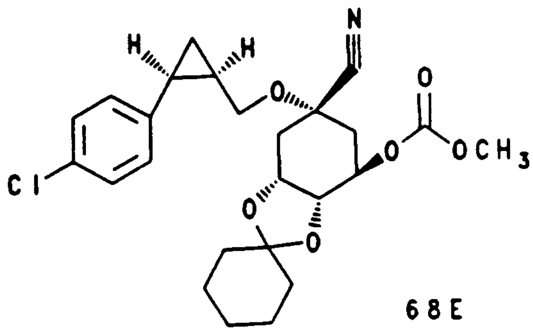

68 E

als farbloses Öl.

5) Analog der Umsetzung von 4 → 6 gemäß Beispiel 1 erhielt man aus 68E die Verbindung 68 der Formel

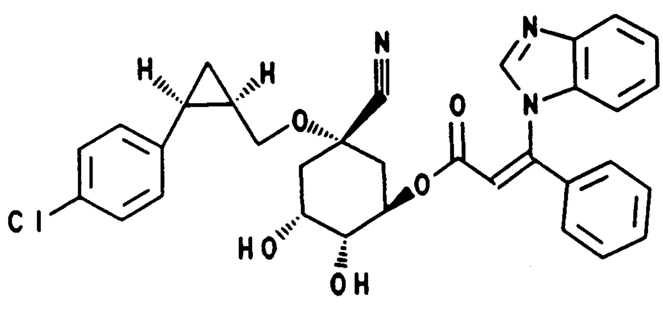

68

m/e = 584 (M+H$^+$) Fp.: 197-202°C

Beispiel 69

Analog Beispiel 1 wurde die Verbindung 69

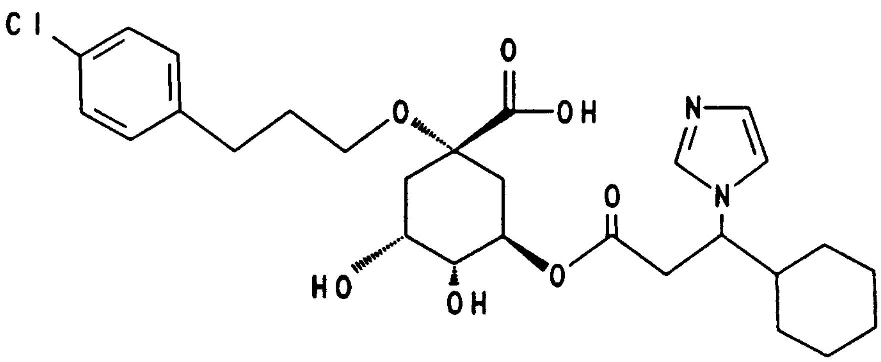

m/e 549 (M+H) hergestellt.

Beispiel 70

Stufe 1 : 1-[4-(4-Chlorphenyl)-butyl]-cyclohex-3-en-carbonsäuremethylester 70B aus 70A

85 mmol (11,9 ml) trockenes Diisopropylamin werden 200 ml trockenem THF gelöst und unter Schutzgas (Stickstoff oder Argon) im Trockeneis/Aceton-Kühlbad gekühlt. Dazu läßt man unter gutem Rühren 80 mmol (50 ml) einer 1,6 molaren Lösung von n-Butyllithium in Hexan zulaufen. Man rührt 10 Min. nach und tropft dann 75 mmol (10,5 g) Cyclohex-3-en-1-carbonsäuremethylester 70A

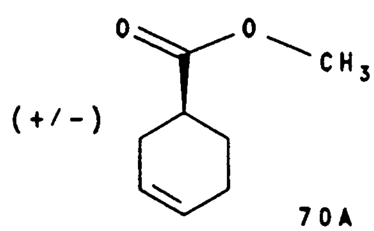

70A

(kommerziell erhältlich) in 10 ml THF gelöst so zu, daß die Innentemperatur -65°C nicht übersteigt. Anschließend läßt man 30 Min. bei -70 bis -80°C rühren und tropft dann 74 mMol (21.8 g) 4-(4-Chlorphenyl)-butyljodid in 25 ml THF gelöst so zu, daß die Innentemperatur -65°C nicht übersteigt. Anschließend läßt man 3 h bei -70 bis -80°C rühren und entfernt dann das Kühlbad. Nachdem die Innentemperatur 10°C erreicht hat, rührt man die Reaktionslösung in 400 ml gesättigte Ammoniumchlorid-Lösung ein, extrahiert 3x mit MTB-Ether, wäscht die vereinigten Extrakte 3x mit Wasser und 2x mit gesättigter Kochsalzlösung, trocknet über Natriumsulfat und entfernt das Lösungsmittel im Vakuum. Das Rohprodukt wird durch Flash-Chromatographie an Kieselgel (Laufmittel: Ethylacetat/n-Heptan 1/9 vol/vol) gereinigt. Das Produkt 70B der Formel

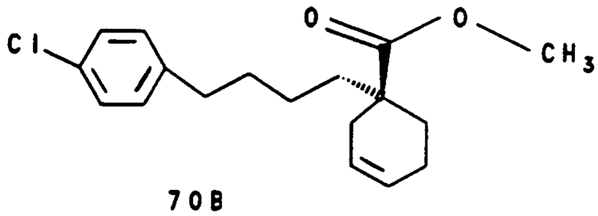

70B

fällt als weißes, niedrigschmelzendes Wachs an.
Massenspektrum: m/e = 307 ($M+H^+$).

Stufe 2: 1-[4-(4-Chlorphenyl)-butyl]-cyclohex-3-en-1-carbonsäure Natriumsalz 70C aus 70B

22,7 g 1-[4-(4-Chlorphenyl)-butyl]-cyclohex-3-en-1-carbonsäuremethylester 70B werden in 100 ml Methanol + 100 ml Dioxan gelöst. Dazu gibt man die Lösung von 8 g Natriumhydroxyd in 50 ml Wasser und kocht unter Schutzgas für 16 h am Rückfluß. Die abgekühlte Reaktionslösung wird mit 200 ml Wasser, 100 ml Toluol und 100 ml n-Heptan versetzt und gut durchgerührt. Das ausgeschiedene Produkt wird abgesaugt, mit wenig kaltem Wasser und n-Heptan/Toluol (1:1 vol/vol) nachgewaschen und im Vakuum getrocknet. Man erhält 70C der Formel

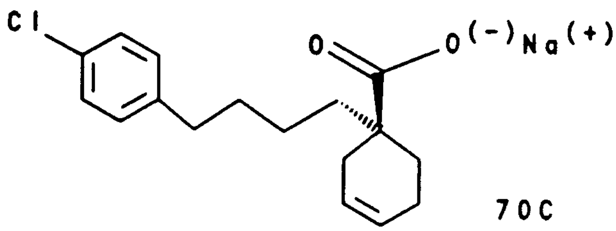

70C

als farblose, glänzende Schuppen, die bis 240°C nicht schmelzen. Die aus dem Natriumsalz 70C durch Ansäuern mit konzentrierter Salzsäure erhaltene freie Säure schmilzt bei 86-7°C.

Stufe 3: 1-[4-(4-Chlorphenyl)-butyl]-4-exo-jodo-6-oxabicyclo[3.2.1]-octan-7-on 70D aus 70C

22,2 g 1-[4-(4-Chlorphenyl)-butyl]-cyclohex-3-en-1-carbonsäure Natriumsalz 70C werden in der Lösung von 22 g Natriumhydrogencarbonat und 68 g Kaliumjodid in 350 ml Wasser suspendiert. Dazu gibt man 175 ml MTB-Ether und 20 g Jod und rührt 16 h bei Raumtemperatur unter Schutzgas. Die Reaktionslösung wird portionsweise mit 10 %iger wäßriger Natriumbisulfitlösung versetzt, bis die Jodfarbe verschwunden ist und dreimal mit Ethylacetat extrahiert. Die Extrakte werden zweimal mit gesättigter Kochsalzlösung gewaschen, über Natriumsulfat getrocknet und im Vakuum eingedampft. Man erhält 70D der Formel

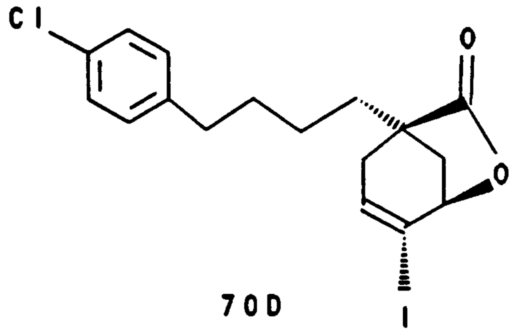

70D

einen leicht gelblichen Feststoff vom Schmelzpunkt 84-6°C.

Stufe 4: 1-[4-(4-Chlorphenyl)-butyl]-6-oxabicyclo[3.2.1]-octan-7-on 70E aus 70D

2 g 1-[4-(4-Chlorphenyl)-butyl]-4-exo-jodo-6-oxabicyclo[3.2.1]-octan-7-on 70D werden in 20 ml trockenen MTB-Ether gelöst. Dazu gibt man unter Schutzgas ca. 0,1 ml einer 1-molaren Lösung von Triethylboran in THF und tropft dann 1,35 ml Tributylzinnhydrid zu. Man läßt 2 h bei Raumtemperatur nachrühren, gibt dann die Lösung von 5 g Kaliumfluorid in 50 ml Wasser zu und läßt 30 Min. kräftig rühren. Der ausgefallene Niederschlag wird abgesaugt, das Filtrat dreimal mit MTB-Ether extrahiert, die Extrakte werden je zweimal mit Wasser und gesättigter Kochsalzlösung gewaschen, über Natriumsulfat getrocknet und das Lösungsmittel wird im Vakuum abdestilliert. Das Rohprodukt wird durch Flash-Chromatographie an Kieselgel (Laufmittel Ethylacetat/n-Heptan 1/3 vol/vol) gereinigt. Man erhält 70E

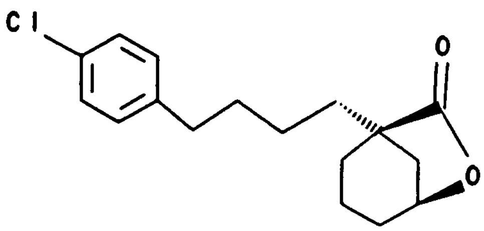

70E

als farbloses Öl, das zu einem niedrigschmelzenden Wachs erstarrt. Massenspektrum: m/e = 293 ($M+H^+$)
Die Stufen 5 und 6 werden analog Beispiel 1 durchgeführt. Man erhält die Verbindung 70 der Formel

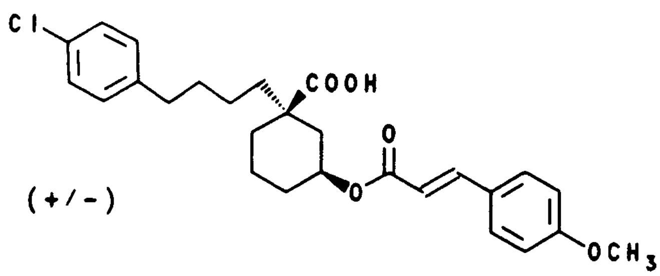

(+/-)

70

m/e = 470

Analog Beispiel 1 wurden die Verbindungen folgender Beispiel hergestellt:

Beispiel 71

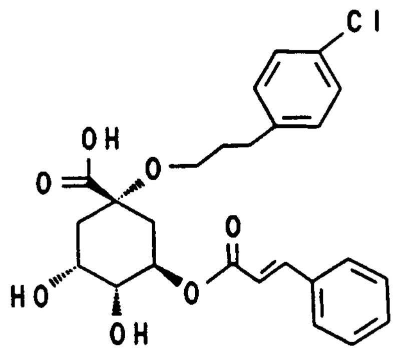

m/e = 475 (M+H⁺)

Beispiel 72

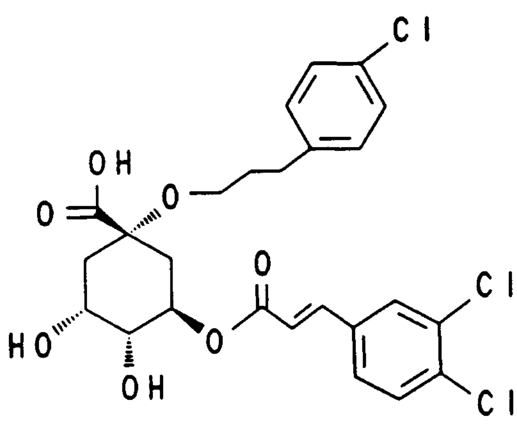

m/e = 543 (M+H⁺)

Beispiel 73

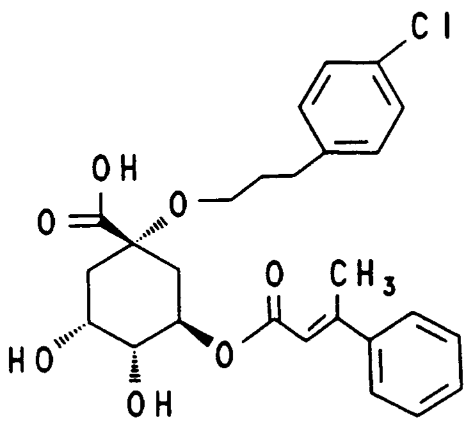

m/e = 488 (M+H⁺)

Beispiel 74

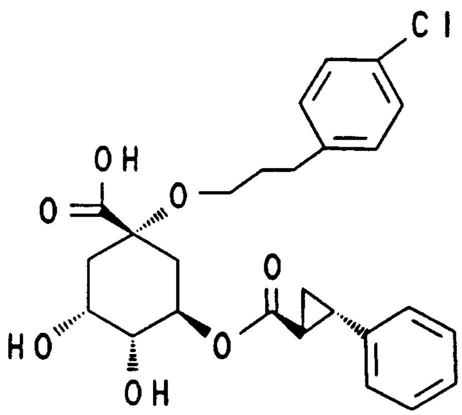

m/e = 489 (M+H⁺)

Beispiel 75

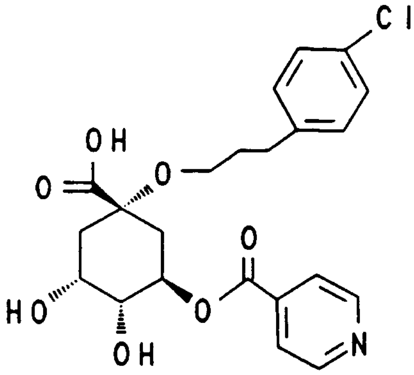

m/e = 450 (M+H+)

Beispiel 76

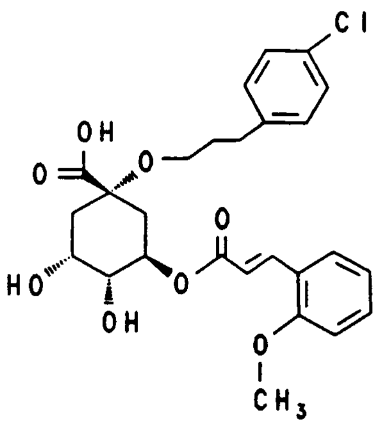

m/e = 505 (M+H+)

Beispiel 77

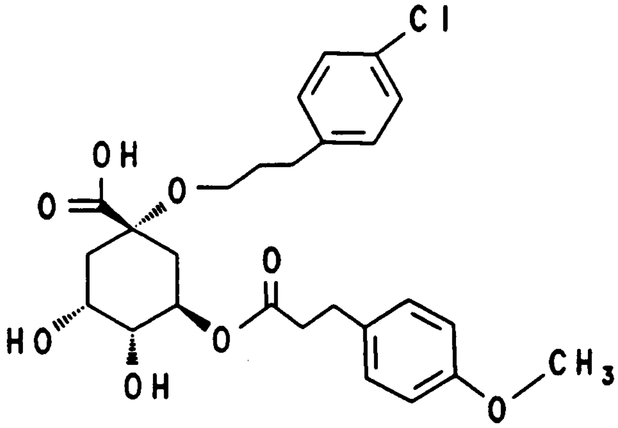

m/e = 507 (M+H+)

Beispiel 78

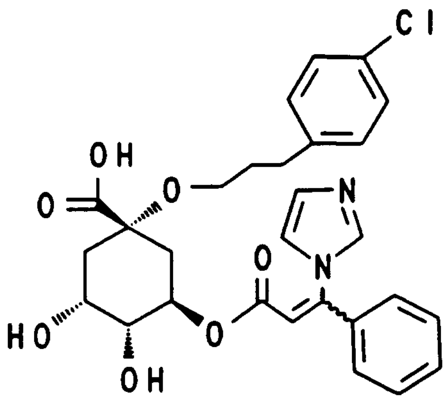

m/e= 541 (M+H+)

Beispiel 79

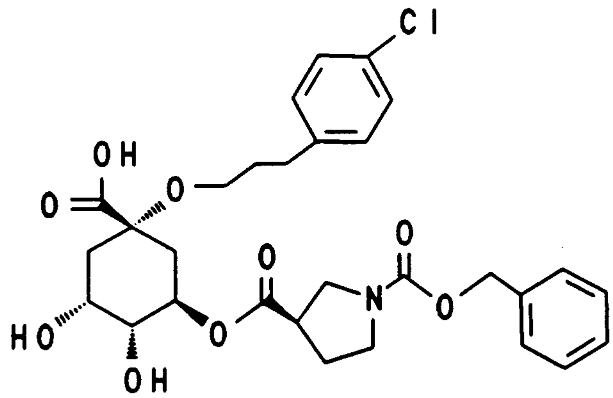

m/e = 576 (M+H⁺)

Beispiel 80

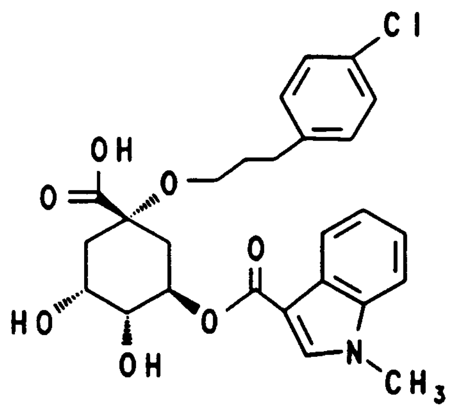

m/e = 502 (M+H⁺)

Beispiel 81

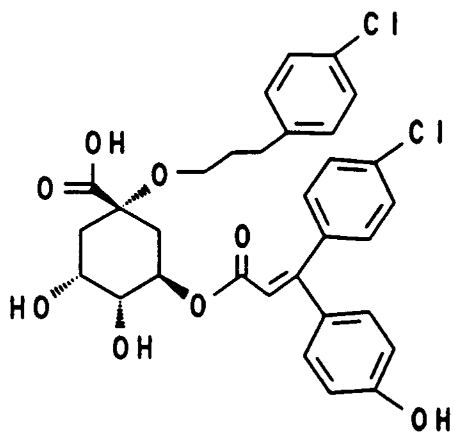

m/e = 601 (M+H$^+$)

Beispiel 82

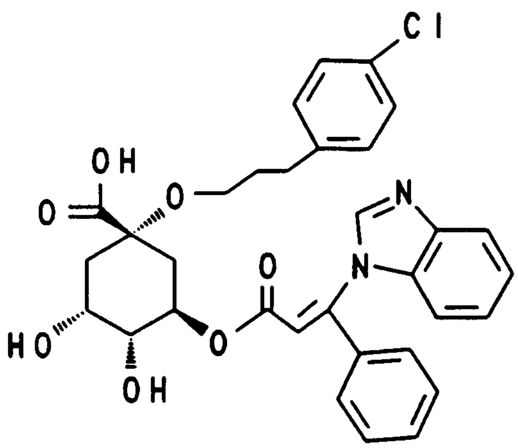

m/e = 591 (M+H$^+$)

Beispiel 83

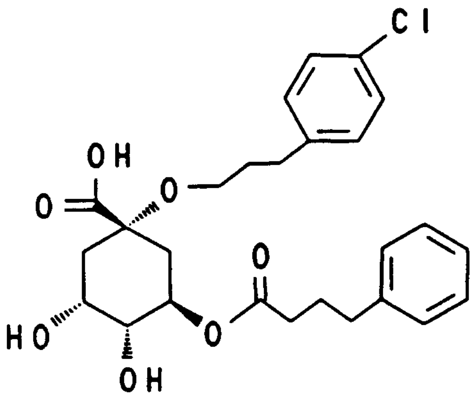

m/e = 491 (M+H⁺)

Beispiel 84

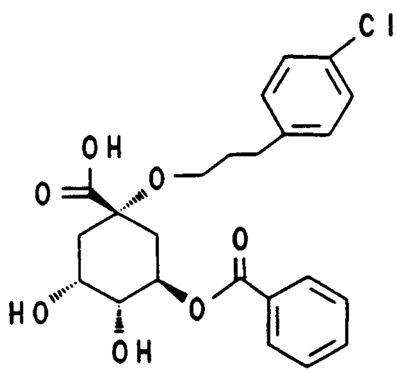

m/e = 449 (M+H⁺)

Beispiel 85

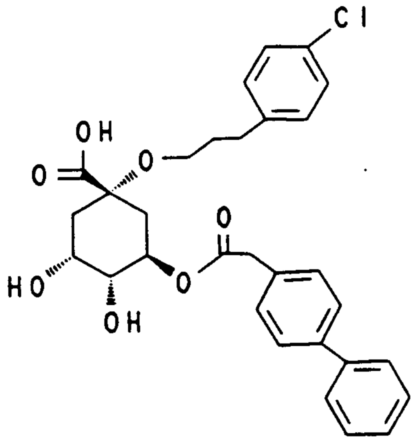

m/e = 539 (M+H⁺)

Beispiel 86

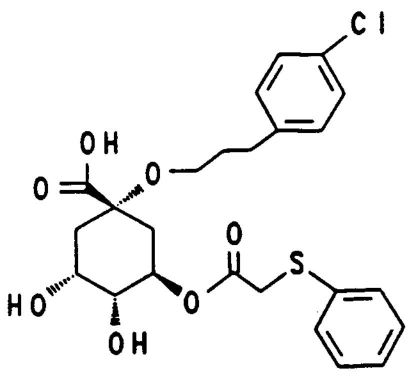

m/e = 495 (M+H⁺)

Beispiel 87

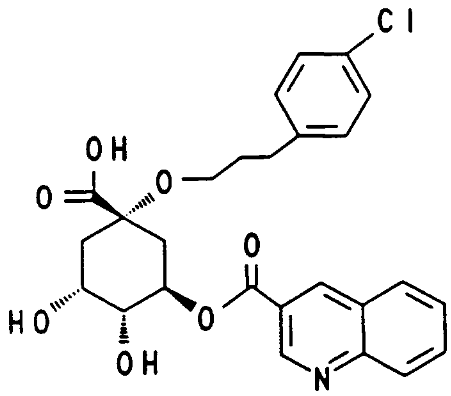

m/e = 500 (M+H⁺)

Beispiel 88

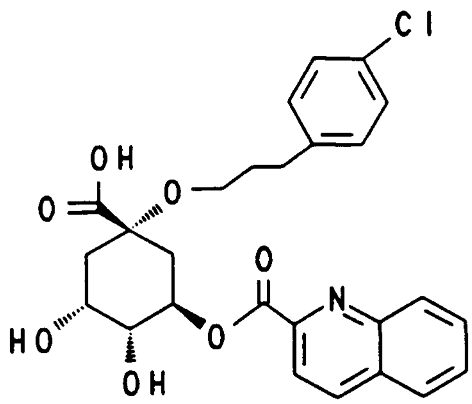

m/e = 500 (M+H⁺)

Beispiel 89

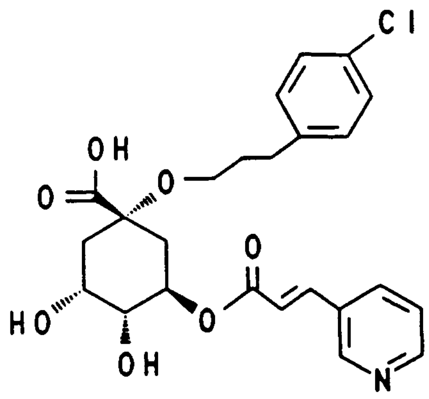

m/e = 476 (M+H$^+$)

Beispiel 90

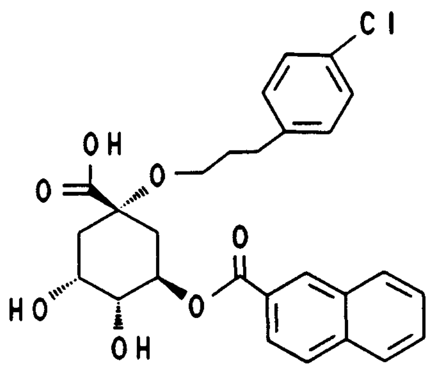

m/e = 499 (M+H$^+$)

Beispiel 91

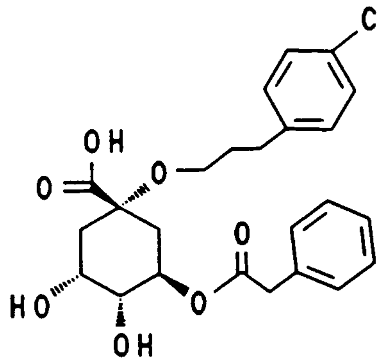

m/e = 463 (M+H⁺)

Beispiel 92

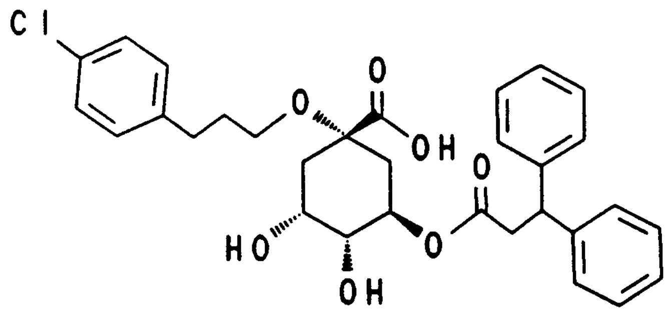

m/e = 553 (M+H⁺)

Beispiel 93

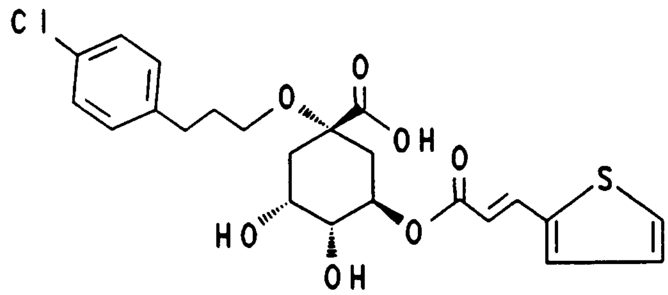

m/e = 481 (M+H⁺)

Beispiel 94

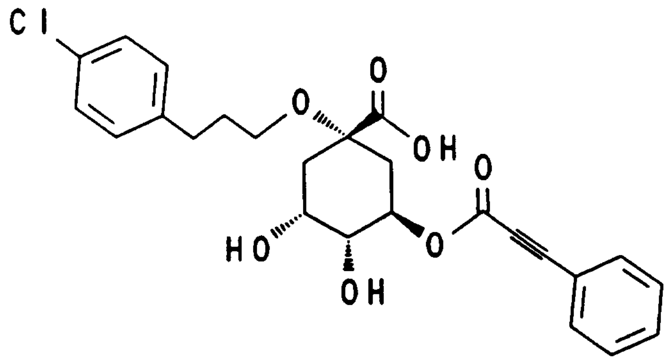

m/e = 473 (M+H⁺)

Beispiel 95

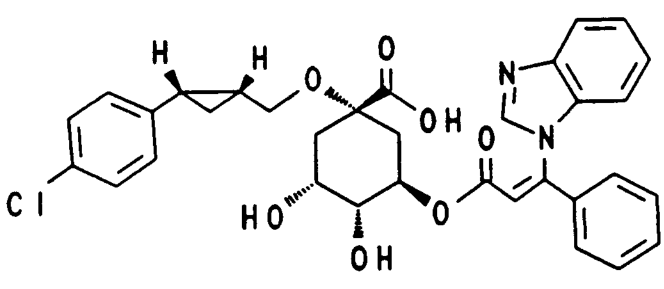

m/e = 603 (M+H⁺)

Beispiel 96

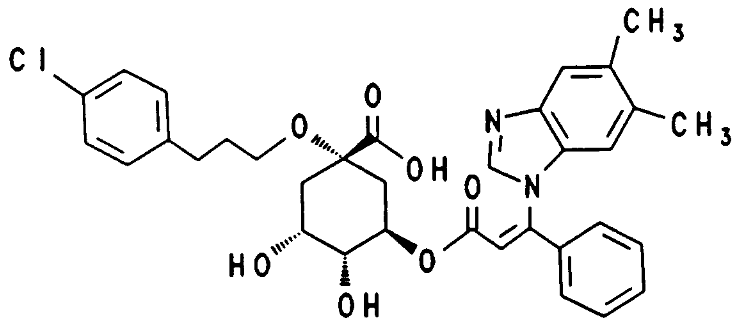

m/e = 619 (M+H+)

Beispiel 97

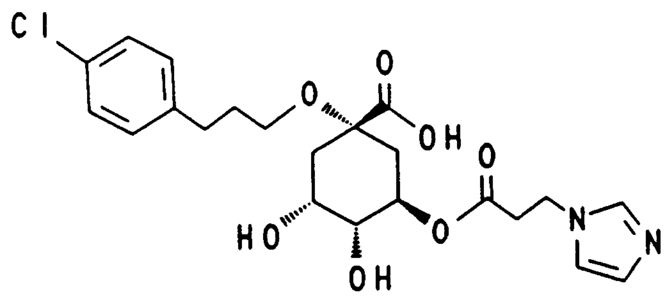

m/e = 467 (M+H+)

Beispiel 98

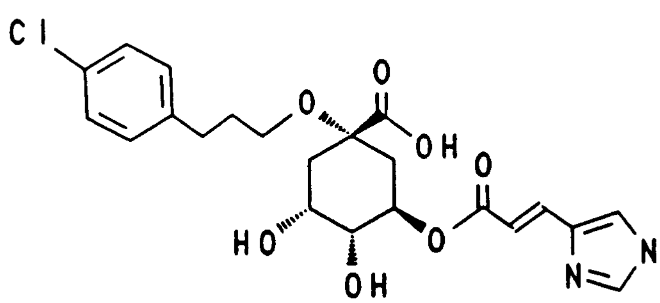

m/e = 465 (M+H+)

Beispiel 99

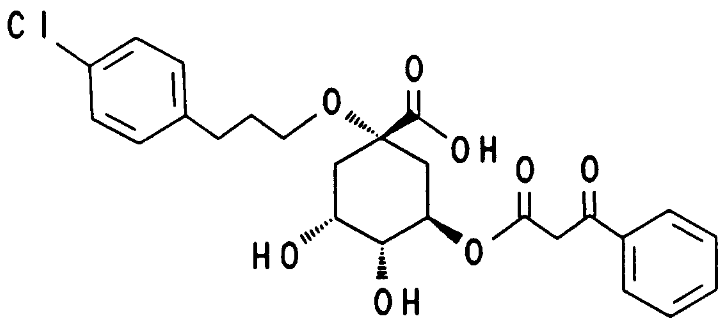

m/e = 491 (M+H⁺)

Beispiel 100

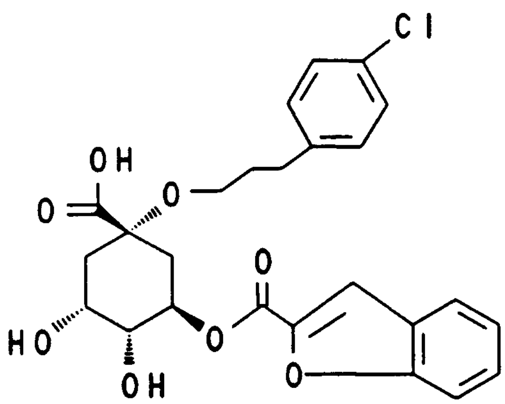

m/e = 489 (M+H⁺)

Beispiel 101

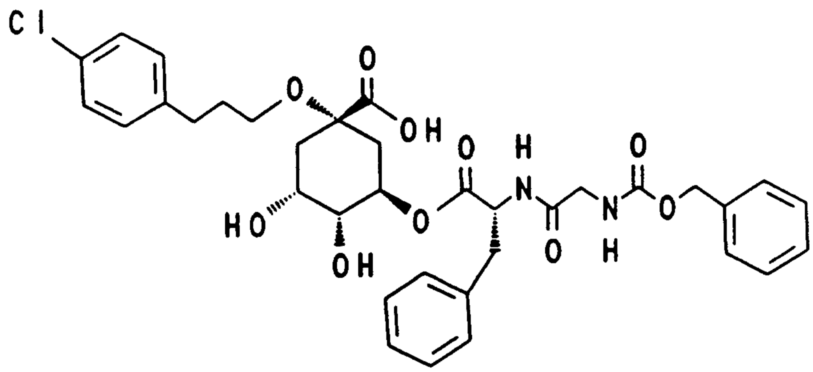

m/e = 683 (M+H⁺)

Beispiel 102

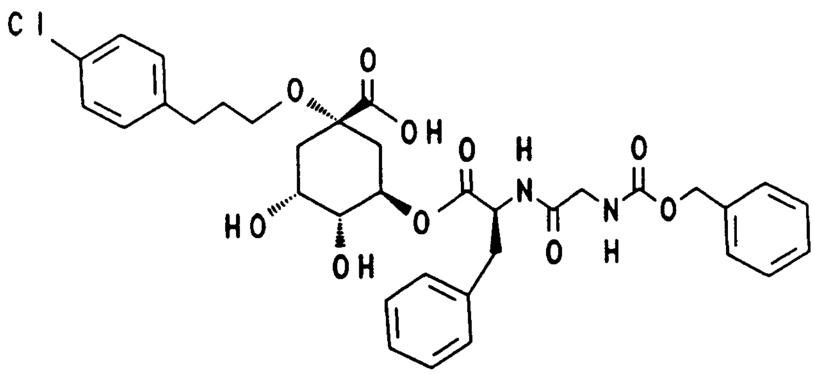

m/e = 683 (M+H⁺)

Beispiel 103

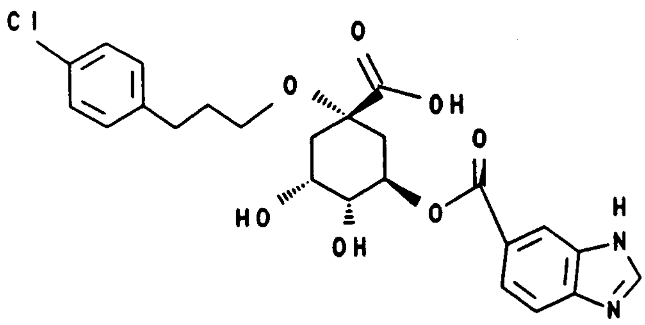

m/e = 489 (M+H⁺)

Beispiel 104

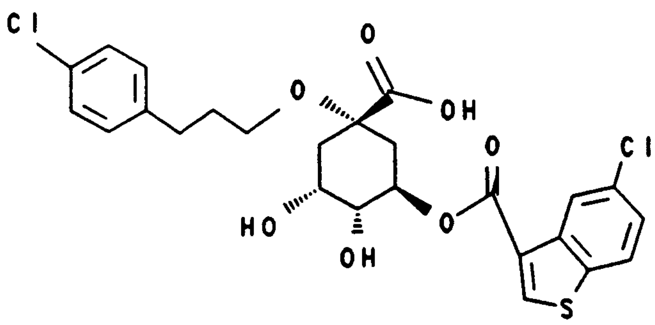

m/e = 539 (M+H⁺)

Nach Verfahren C, analog der Beschreibung vor Beispiel 45 wurden die Verbindungen der folgenden Beispiele hergestellt:

Beispiel 105

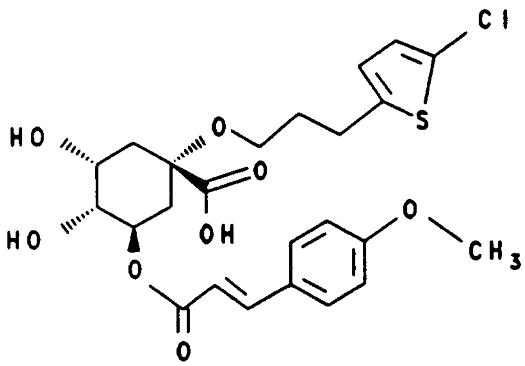

m/e = 507 (M+H+)

Beispiel 106

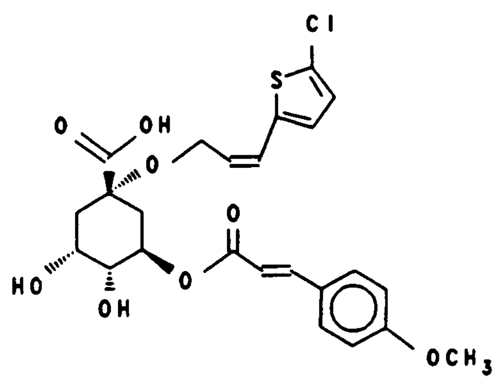

m/e = 509 (M+H+)

Beispiel 107

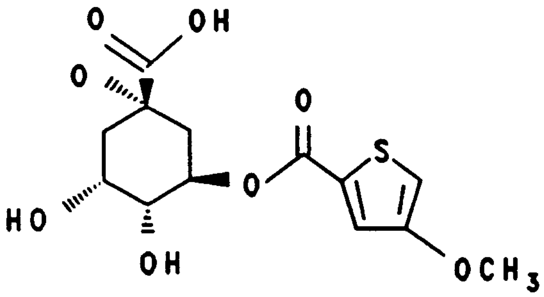

m/e = 483 (M+H⁺) I.p.: 135°C

Beispiel 108

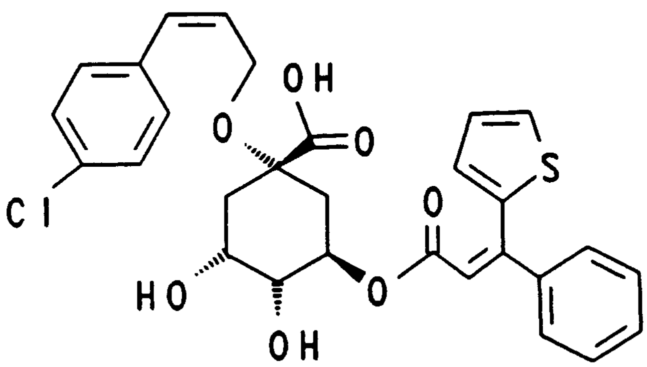

m/e = 555 (M+H⁺)

Beispiel 109

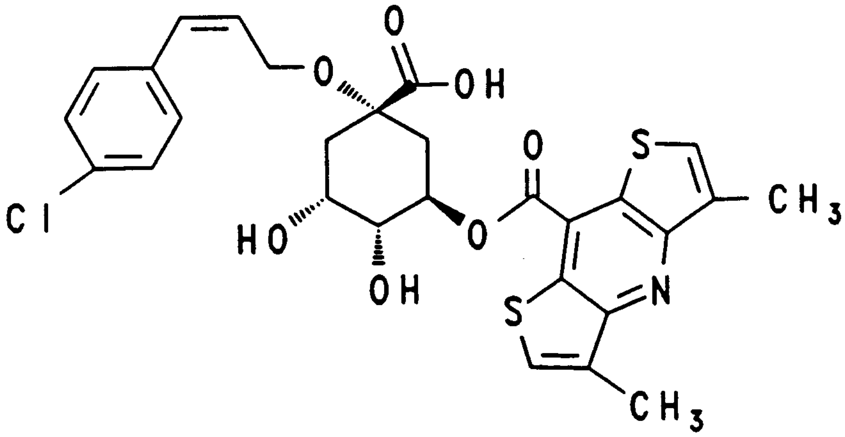

m/e = 588 (M+H⁺)

Beispiel 110

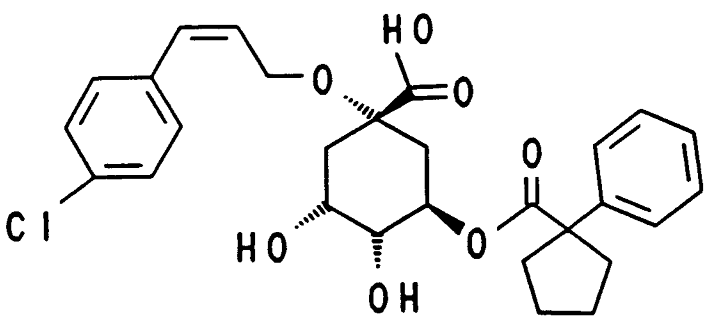

m/e = 515 (M+H⁺)

Beispiel 111

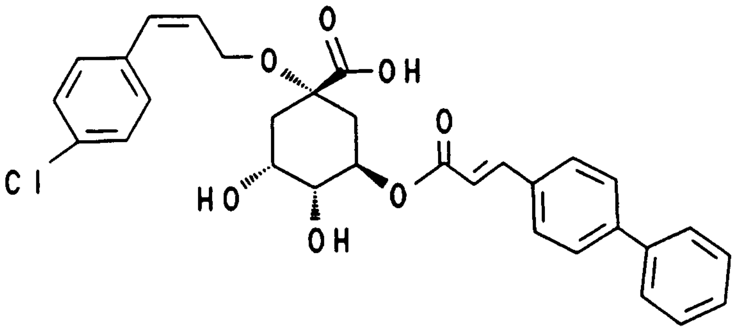

F.p.: 161°C

Beispiel 112

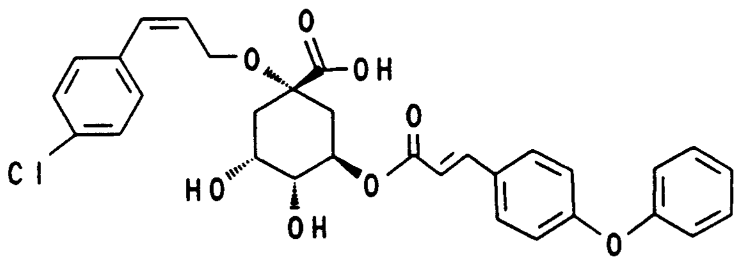

m/e = 565 (M+H⁺)

Beispiel 113

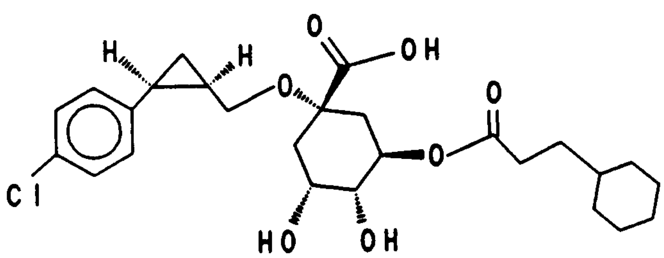

m/e = 495 (M+H⁺)

Beispiel 114

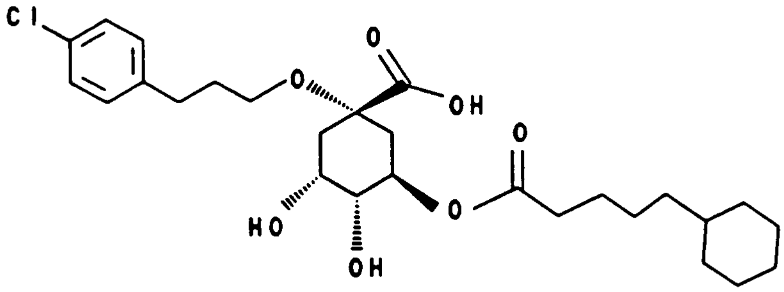

m/e = 511 (M+H+)

Beispiel 115

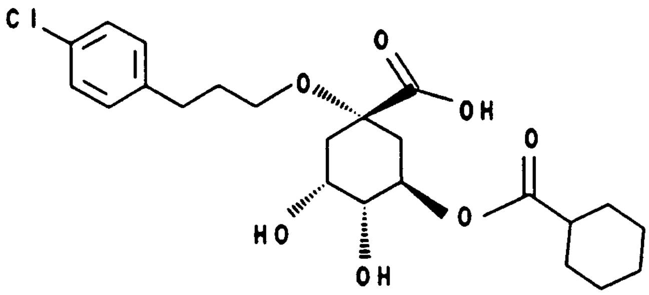

m/e = 455 (M+H+)

Beispiel 116

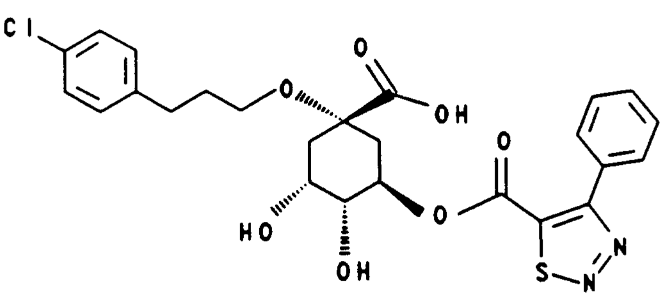

m/e = 533 (M+H+)

Beispiel 117

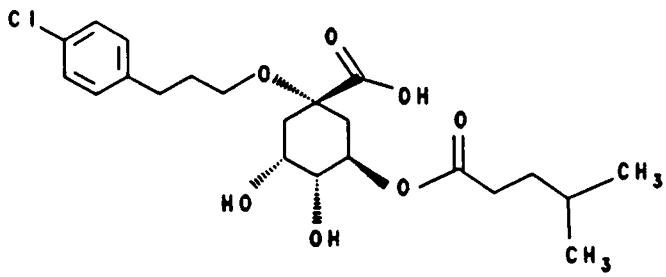

m/e = 443 (M+H⁺)

Beispiel 118

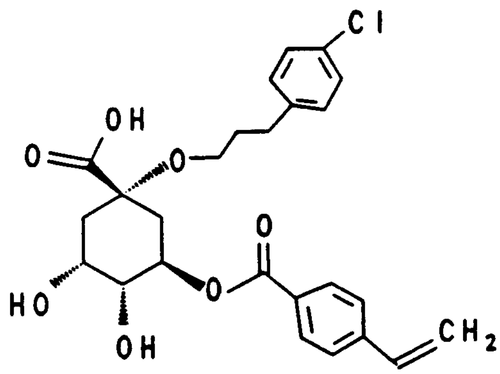

m/e = 475 (M+H⁺)

Beispiel 119

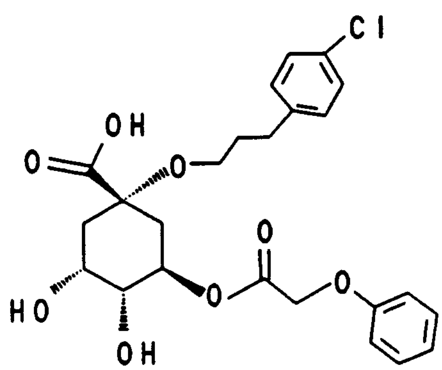

m/e = 479 (M+H⁺)

Beispiel 120

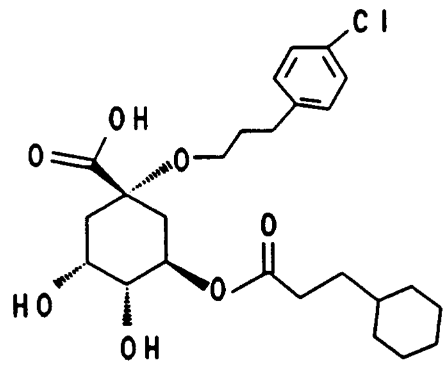

m/e = 483 (M+H⁺)

**Patentansprüche**

1. Cyclohexan-Derivate der Formel I

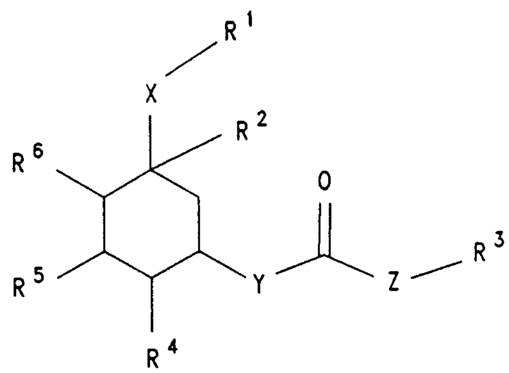

I

worin die Reste die folgenden Bedeutungen haben:

| | |
|---|---|
| $R^1$: | CN, COOH, eine mit einer Schutzgruppe geschützte COOH-Gruppe, $C_1$-$C_4$-Alkanoyl, $SO_3$-$C_1$-$C_4$-Alkyl, $SO_3H$, $SO_2NR^8R^9$, $PO(OH)_2$, $PO(OH)(O$-$C_1$-$C_4$-Alkyl), $PO(O$-$C_1$-$C_4$-Alkyl$)_2$, |
| $R^2$: | $C_1$-$C_{10}$-Alkyl $(R^{11})_n$, O-$C_1$-$C_{10}$-Alkyl $(R^{11})_n$, $C_2$-$C_{10}$-Alkenyl $(R^{11})_n$, O-$C_3$-$C_{10}$-Alkenyl $(R^{11})_n$, $C_2$-$C_{10}$-Alkinyl $(R^{11})_n$, O-$C_3$-$C_{10}$-Alkinyl $(R^{11})_n$, S-$C_1$-$C_{10}$-Alkyl $(R^{11})_n$, S-$C_3$-$C_{10}$-Alkenyl $(R^{11})_n$, S-$C_3$-$C_{10}$-Alkinyl $(R^{11})_n$, NH-$C_1$-$C_{10}$-Alkyl $(R^{11})_n$, NH-$C_3$-$C_{10}$-Alkenyl $(R^{11})_n$ oder NH-$C_3$-$C_{10}$-Alkinyl $(R^{11})_n$, wobei $R^{11}$ gegebenenfalls jeweils mit $R^{12}$ substituiert ist; |
| $R^3$, $R^{11}$ und $R^{13}$: | Alkyl mit 1 bis 10 C-Atomen, Cycloalkyl mit 3-8 Ring-C-Atomen, Phenyl, Naphtyl, Phenanthryl, Pyridyl, Thienyl, Furyl, Pyrimidyl, Indolyl, Imidazolyl, Coumarinyl, Phthaliminyl, Chinolyl, Piperazinyl, Tetrazolyl, Triazolyl, Oxazolyl oder deren thieno-, pyridino-, pyrimidino- oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O- |

Benzyl gleich oder verschieden substituiert sein kann, und $R^3$, $R^{11}$ und $R^{13}$ gleich oder verschieden sind;

$R^4$, $R^5$ und $R^6$: H, OH, eine durch übliche Alkoholschutzgruppen geschützte OH-Gruppe, F, Cl, Br oder die für $R^2$ angegebenen Bedeutungen haben, wobei $R^4$, $R^5$ und $R^6$ gleich oder verschieden sind;

$R^7$: $C_1$-$C_4$-Alkyl, Phenyl oder Benzyl;

$R^8$ und $R^9$: H, $C_1$-$C_4$-Alkyl, $C_1$-$C_4$-Alkanoyl, Phenyl, das gegebenenfalls substituiert ist mit F, Cl, Br, J, OH, O-$C_1$-$C_4$-Alkyl, $CF_3$, -$NO_2$ oder CN, wobei $R^8$ und $R^9$ gleich oder verschieden sind, oder $R^8$ und $R^9$ bilden zusammen mit dem Stickstoffatom einen 4 bis 10gliedrigen, gesättigten heterocyclischen Ring, bei dem gegebenenfalls eine $CH_2$-Gruppe durch O, S oder $NR^{10}$ ersetzt sein kann,

$R^{10}$: H, $C_1$-$C_4$-Alkyl, Phenyl oder Benzyl

$R^{12}$: Phenyl, Naphtyl, Phenanthryl, Pyridyl, Thienyl, Furyl, Pyrimidyl, Indolyl, Imidazolyl, Cumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, J, OH, $CF_3$, -$NO_2$, CN, $C_1$-$C_4$-Alkoxy, $C_1$-$C_4$-Alkyl, $C_2$-$C_4$-Alkenyl, $NR^8R^9$, Phenyl, Benzyl, Thienyl, Furyl, Imidazolyl, Pyridyl, O-Phenyl oder O-Benzyl gleich oder verschieden substituiert sein kann;

X: $(CH_2)_m$, -CH = CH-, -C ≡ C-, -$CH_2$-O-$CH_2$-, -$CH_2$-S-$CH_2$- oder

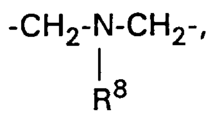

Y: $(CH_2)_m$, O, S, $NR^8$,

Z: $(CH_2)_m$, S, O, S-$C_1$-$C_{10}$-Alkyl, O-$C_1$-$C_{10}$-Alkyl, CH = CH, CH = CF, CH = CCl, CH = CBr, $CH_2$-CO, $CH_2$-CHF, $CH_2$-CHCl, $CH_2$-CHBr, $CH_2$-CHJ, $C_3$-$C_{10}$-Cycloalkylen, $C_3$-$C_{10}$-Cycloalkenylen, wobei 1 bis 3 Ring-C-Atome durch Schwefel-, Sauerstoff- oder Stickstoffatme ersetzt sein können, $COOR^7$, C ≡ CH = C($C_1$-$C_4$-Alkyl), CH =C(CN), CH = C($NR^8R^9$), CH = C($C_1$-$C_4$-Alkanoyl), CH = C($R^{13}$), $NR^8$, und wenn Y Sauerstoff ist, kann

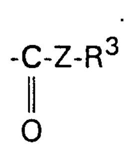

gemeinsam einen Aminosäurerest, ausgewählt aus der Gruppe bestehend aus Ala, Arg, Asn, Asp, Cys, Gln, Glu, Gly, His, Ile,Leu, Lys, Phe, Pro, Ser, Thr, Trp, Tyr und deren durch übliche Schutzgruppen geschützte Derivate, darstellen,

n: null, 1 oder 2

m: null, 1, 2, 3 oder 4,

sowie physiologisch verträgliche Salze von Verbindungen der Formel I, wobei die Verbindung der Formel Ia

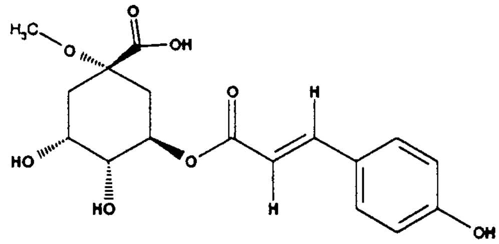

I a

ausgenommen ist.

**2.** Cyclohexan-Derivate der Formel I

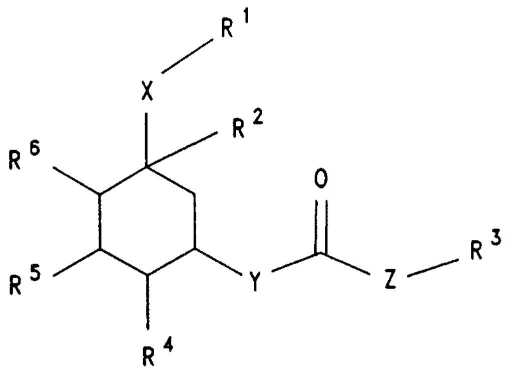

I

worin die Reste die folgenden Bedeutungen haben:

| | |
|---|---|
| $R^1$: | CN, COOH, eine mit einer Schutzgruppe geschützte COOH-Gruppe, $C_1$-$C_4$-Alkanoyl, $SO_3$-$C_1$-$C_4$-Alkyl, $SO_3H$, $SO_2NR^8R^9$, $PO(OH)_2$, $PO(OH)(O$-$C_1$-$C_4$-Alkyl), PO(O-$C_1$-$C_4$-Alkyl$)_2$, |
| $R^2$: | $C_1$-$C_{10}$-Alkyl $(R^{11})_n$, O-$C_1$-$C_{10}$-Alkyl $(R^{11})_n$, $C_2$-$C_{10}$-Alkenyl $(R^{11})_n$, O-$C_3$-$C_{10}$-Alkenyl $(R^{11})_n$, $C_2$-$C_{10}$-Alkinyl $(R^{11})_n$, O-$C_3$-$C_{10}$-Alkinyl $(R^{11})_n$, S-$C_1$-$C_{10}$-Alkyl $(R^{11})_n$, S-$C_3$-$C_{10}$-Alkenyl $(R^{11})_n$, S-$C_3$-$C_{10}$-Alkinyl $(R^{11})_n$, NH-$C_1$-$C_{10}$-Alkyl $(R^{11})_n$, NH-$C_3$-$C_{10}$-Alkenyl $(R^{11})_n$ oder NH-$C_3$-$C_{10}$-Alkinyl $(R^{11})_n$, wobei $R^{11}$ gegebenenfalls jeweils mit $R^{12}$ substituiert ist; |
| $R^3$, $R^{11}$ und $R^{13}$: | Phenyl, Naphtyl, Phenanthryl, Pyridyl, Thienyl, Furyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, J, OH, $CF_3$, - $NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann, und $R^3$, $R^{11}$ und $R^{13}$ gleich oder verschieden sind; |
| $R^4$, $R^5$ und $R^6$: | H, OH, eine durch übliche Alkoholschutzgruppen geschützte OH-Gruppe, F, Cl, Br oder die für $R^2$ angegebenen Bedeutungen haben, wobei $R^4$, $R^5$ und $R^6$ gleich oder verschieden sind; |
| $R^7$: | $C_1$-$C_4$-Alkyl, Phenyl oder Benzyl; |

$R^8$ und $R^9$: H, $C_1$-$C_4$-Alkyl, $C_1$-$C_4$-Alkanoyl, Phenyl, das gegebenenfalls substituiert ist mit F, Cl, Br, J, OH, O-$C_1$-$C_4$-Alkyl, $CF_3$, $-NO_2$ oder CN, wobei $R^8$ und $R^9$ gleich oder verschieden sind, oder $R^8$ und $R^9$ bilden zusammen mit dem Stickstoffatom einen 4 bis 10gliedrigen, gesättigten heterocyclischen Ring, bei dem gegebenenfalls eine $CH_2$-Gruppe durch O, S oder $NR^{10}$ ersetzt sein kann,

$R^{10}$: H, $C_1$-$C_4$-Alkyl, Phenyl oder Benzyl

$R^{12}$: Phenyl, Naphtyl, Phenanthryl, Pyridyl, Thienyl, Furyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann;

X: $(CH_2)_m$, $-CH=CH-$, $-C\equiv C-$, $-CH_2-O-CH_2-$, $-CH_2-S-CH_2-$

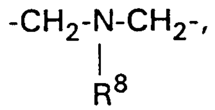

oder

Y: $(CH_2)_m$, O, S, $NR_8$,

Z: $(CH_2)_m$, S, O, S-$C_1$-$C_{10}$-Alkyl, CH = CH, CH = CF, CH = CCl, CH = CBr, $CH_2$-CO, $CH_2$-CHF, $CH_2$-CHCl, $CH_2$-CHBr, $CH_2$-CHJ, $C_3$-$C_{10}$-Cycloalkylen, $C_3$-$C_{10}$-Cycloalkenylen, $COOR^7$, $C\equiv C$, CH = C($C_1$-$C_4$-Alkyl), CH = C(CN), CH = C($NR^8R^9$), CH = C($C_1$-$C_4$-Alkanoyl), CH = C($R^{13}$), $NR^8$, und wenn Y Sauerstoff ist, kann

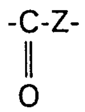

$R^3$ gemeinsam einen Aminosäurerest, ausgewählt aus der Gruppe bestehend aus Ala, Arg, Asn, Asp, Cys, Gln, Glu, Gly, His, Ile,Leu, Lys, Phe, Pro, Ser, Thr, Trp, Tyr und deren durch übliche Schutzgruppen geschützte Derivate, darstellen,

n: null, 1 oder 2

m: null, 1, 2, 3 oder 4,

sowie physiologisch verträgliche Salze von Verbindungen der Formel I, wobei die Verbindung der Formel Ia

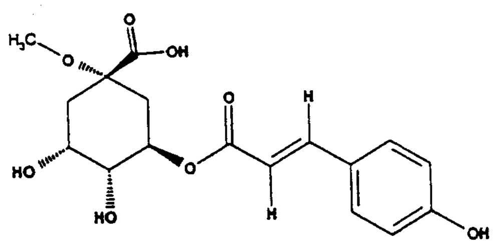

I a

ausgenommen ist.

3. Verbindungen gemäß Anspruch 2, dadurch gekennzeichnet, daß in Formel I $R^1$ CN, COOH, eine mit einer Schutzgruppe geschützte COOH-Gruppe oder $C_1$-$C_4$-Alkanoyl bedeutet und die übrigen Reste die in Anspruch 1 angegebenen Bedeutungen haben.

4. Verbindungen gemäß Anspruch 2, dadurch gekennzeichnet, daß in Formel I, die Reste folgende Bedeutungen haben:

$R^1$: CN, COOH, eine mit einer Schutzgruppe geschützte COOH-Gruppe oder $C_1$-$C_4$-Alkanoyl

$R^2$: O-$C_1$-$C_{10}$-Alkyl($R^{11}$)$_n$ (n = 0,1,2), wobei der Alkylteil unverzweigt, verzweigt oder cyclisch ist, sowie eine der $CH_2$-Gruppen durch O ersetzt sein kann und $R_{11}$ mit $R_{12}$ substituiert sein kann und bei n = 2 die beiden Reste $R^{11}$ gleich oder verschieden sind,
O-$C_3$-$C_{10}$-Alkenyl($R^{11}$)$_n$ (n = 0,1,2), wobei der Alkenylteil unverzweigt, verzweigt oder cyclisch ist, eine der $CH_2$-Gruppen durch O, S, SO, $SO_2$, oder $NR^8$ ersetzt sein kann sowie ein- oder mehrfach ungesättigt ist, und $R^{11}$ mit $R^{12}$ substituiert sein kann und bei n = 2 die beiden Reste $R^{11}$ gleich oder verschieden sind, O-$C_3$-$C_{10}$-Alkinyl($R^{11}$)$_n$ (n = 0,1,2), wobei der Alkinylteil unverzweigt, verzweigt oder cyclisch ist, ein- oder mehrfach ungesättigt ist sowie eine der $CH_2$-Gruppen durch O, S, SO, $SO_2$, oder $NR^8$ ersetzt sein kann und $R^{11}$ mit $R^{12}$ substituiert sein kann und bei n = 2 die beiden Reste $R^{11}$ gleich oder verschieden sind,
$R^3$ bis $R^{12}$ und $R^{13}$ die in Anspruch 2 angegebenen Bedeutungen haben,

X: $(CH_2)_m$, (m = 0,1,2,3,4) CH = CH, C ≡ C, $CH_2OCH_2$, $CH_2SCH_2$

Y: $(CH_2)_m$, (m = 0,1,2,3,4) O, S, $NR^8$,

Z: $(CH_2)_m$, (m = 0,1,2,3,4) S, O, S-$C_1$-$C_{10}$-Alkyl, (unverzweigt oder verzweigt), CH = CH, CH = CF, CH = CCl, CH = CBr, $CH_2$-C(O), $CH_2$-CHF, $CH_2$-CHCl, $CH_2$-CHBr, $CH_2$-CHJ, $C_3$-$C_{10}$-Cycloalkylen, $C_3$-$C_{10}$-Cycloalkenylen, $COOR^7$, C ≡ C, CH = C($C_1$-$C_4$-Alkyl) (unverzweigt oder verzweigt), CH = C(CN), CH = C($R^{13}$), $NR_8$.

5. Anwendung von Verbindungen nach Anspruch 1 zur Herstellung von Arzneimitteln zur Behandlung des Typ II Diabetes und anderer Erkrankungen, die durch einen erhöhten Glucose-Ausstoß aus der Leber oder eine erhöhte Aktivität des Glucose-6-Phosphatase-Systems gekennzeichnet sind.

6. Arzneimittel enthaltend eine Verbindung nach Anspruch 1.

7. Arzneimittel enthaltend eine Verbindung nach Anspruch 2.

## Claims

1. A cyclohexane derivative of the formula I

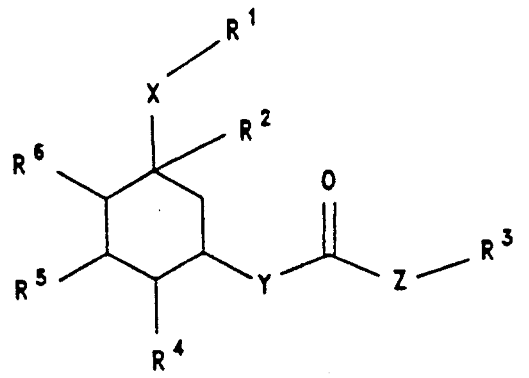

I

in which the radicals have the following meanings:

| | |
|---|---|
| $R^1$: | CN, COOH, a COOH group protected by a protective group, $C_1$-$C_4$-alkanoyl, $SO_3$-$C_1$-$C_4$-alkyl, $SO_3H$, $SO_2NR^8R^9$, $PO(OH)_2$, $PO(OH)(O$-$C_1$-$C_4$-alkyl), $PO(O$-$C_1$-$C_4$-alkyl$)_2$, |
| $R^2$: | $C_1$-$C_{10}$-alkyl $(R^{11})_n$, O-$C_1$-$C_{10}$-alkyl $(R^{11})_n$, $C_2$-$C_{10}$-alkenyl $(R^{11})_n$, O-$C_3$-$C_{10}$-alkenyl $(R^{11})_n$, $C_2$-$C_{10}$-alkynyl $(R^{11})_n$, O-$C_3$-$C_{10}$-alkynyl $(R^{11})_n$, S-$C_1$-$C_{10}$-alkyl $(R^{11})_n$, S-$C_3$-$C_{10}$-alkenyl $(R^{11})_n$, S-$C_3$-$C_{10}$-alkynyl $(R^{11})_n$, NH-$C_1$-$C_{10}$-alkyl $(R^{11})_n$, NH-$C_3$-$C_{10}$-alkenyl $(R^{11})_n$ or NH-$C_3$-$C_{10}$-alkynyl $(R^{11})_n$, where $R^{11}$ is optionally substituted in each case by $R^{12}$; |
| $R^3$, $R^{11}$ and $R^{13}$: | alkyl with 1 to 10 carbon atoms, cycloalkyl with 3-8 ring carbon atoms, phenyl, naphthyl, phenanthryl, pyridyl, thienyl, furyl, pyrimidyl, indolyl, imidazolyl, coumarinyl, phthaliminyl, quinolyl, piperazinyl, tetrazolyl, triazolyl, oxazolyl or their thieno-, pyridino-, pyrimidino-or benzo-fused derivatives, it being possible for the aromatic or heteroaromatic system to be substituted one or more times, identically or differently, by F, Cl, Br, I, OH, $CF_3$, -$NO_2$, CN, $C_1$-$C_4$-alkoxy, $C_1$-$C_4$-alkyl, $NR^8R^9$, phenyl, benzyl, thienyl, furyl, imidazolyl, pyridyl, O-phenyl or O-benzyl, and $R^3$, $R^{11}$ and $R^{13}$ are identical or different; |
| $R^4$, $R^5$ and $R^6$: | H, OH, an OH group which is protected by conventional alcohol-protective groups, F, Cl, Br or have the meanings stated for $R^2$, where $R^4$, $R^5$ and $R^6$ are identical or different; |
| $R^7$: | $C_1$-$C_4$-alkyl, phenyl or benzyl; |
| $R^8$ and $R^9$: | H, $C_1$-$C_4$-alkyl, $C_1$-$C_4$-alkanoyl, phenyl which is optionally substituted by F, Cl, Br, I, OH, O-$C_1$-$C_4$-alkyl, $CF_3$, -$NO_2$ or CN, where $R^8$ and $R^9$ are identical or different, or $R^8$ and $R^9$ form together with the nitrogen atom a 4- to 10-membered, saturated heterocyclic ring in which a $CH_2$ group can optionally be replaced by O, S or $NR^{10}$, |
| $R^{10}$: | H, $C_1$-$C_4$-alkyl, phenyl or benzyl |
| $R^{12}$: | phenyl, naphthyl, phenanthryl, pyridyl, thienyl, furyl, pyrimidyl, indolyl, imidazolyl, coumarinyl, phthaliminyl, quinolyl, piperazinyl, tetrazolyl, triazolyl, oxazolyl or their thieno- or benzo-fused derivatives, it being possible for the aromatic or heteroaromatic system to be substituted one or more times, identically or differently, by F, Cl, Br, I, OH, $CF_3$, -$NO_2$, CN, $C_1$-$C_4$-alkoxy, $C_1$-$C_4$-alkyl, $C_2$-$C_4$-alkenyl, $NR^8R^9$, phenyl, benzyl, thienyl, furyl, imidazolyl, pyridyl, O-phenyl or O-benzyl; |

X: $(CH_2)_m$, -CH = CH-, -C ≡ C-, $-CH_2-O-CH_2-$, $-CH_2-S-CH_2-$or

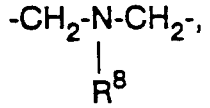

Y: $(CH_2)_m$, O, S, $NR^8$,

Z: $(CH_2)_m$, S, O, S-$C_1$-$C_{10}$-alkyl, O-$C_1$-$C_{10}$-alkyl, CH=CH, CH=CF, CH=CCl, CH=CBr, $CH_2$-CO, $CH_2$-CHF, $CH_2$-CHCl, $CH_2$-CHBr, $CH_2$-CHI, $C_3$-$C_{10}$-cycloalkylene, $C_3$-$C_{10}$-cycloalkenylene, it being possible for 1 to 3 ring carbon atoms to be replaced by sulfur, oxygen or nitrogen atoms, $COOR^7$, C≡C, CH=C($C_1$-$C_4$-alkyl), CH=C(CN), CH=C($NR^8R^9$), CH=C($C_1$-$C_4$-alkanoyl), CH=C($R^{13}$), $NR^8$ and when Y is oxygen,

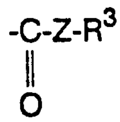

can together be an aminoacid residue selected from the group comprising Ala, Arg, Asn, Asp, Cys, Gln, Glu, Gly, His, Ile, Leu, Lys, Phe, Pro, Ser, Thr, Trp, Tyr and their derivatives protected by conventional protective groups,

n: zero, 1 or 2

m: zero, 1, 2, 3 or 4,

and physiologically tolerated salts of compounds of the formula I, the compound of the formula 1a

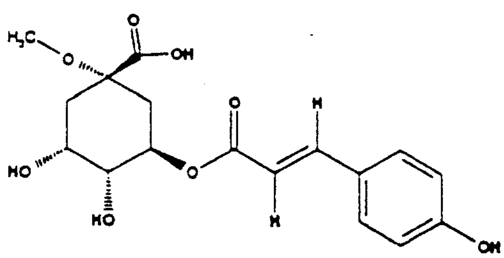

I a

being excepted.

**2.** A cyclohexane derivative of the formula I

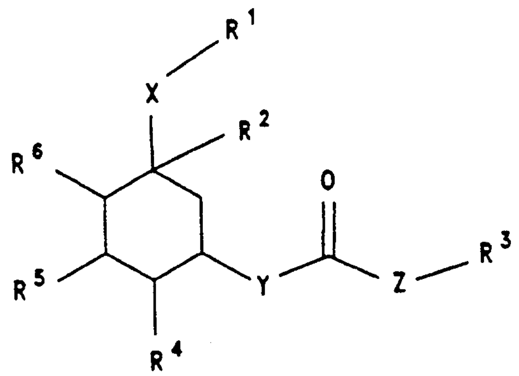

I

in which the radicals have the following meanings:

| | |
|---|---|
| $R^1$: | CN, COOH, a COOH group protected by a protective group, $C_1$-$C_4$-alkanoyl, $SO_3$-$C_1$-$C_4$-alkyl, $SO_3H$, $SO_2NR^8R^9$, $PO(OH)_2$, $PO(OH)(O\text{-}C_1\text{-}C_4\text{-alkyl})$, $PO(O\text{-}C_1\text{-}C_4\text{-alkyl})_2$, |
| $R^2$: | $C_1$-$C_{10}$-alkyl $(R^{11})_n$, O-$C_1$-$C_{10}$-alkyl $(R^{11})_n$, $C_2$-$C_{10}$-alkenyl $(R^{11})_n$, O-$C_3$-$C_{10}$-alkenyl $(R^{11})_n$, $C_2$-$C_{10}$-alkynyl $(R^{11})_n$, O-$C_3$-$C_{10}$-alkynyl $(R^{11})_n$, S-$C_1$-$C_{10}$-alkyl $(R^{11})_n$, S-$C_3$-$C_{10}$-alkenyl $(R^{11})_n$, S-$C_3$-$C_{10}$-alkynyl $(R^{11})_n$, NH-$C_1$-$C_{10}$-alkyl $(R^{11})_n$, NH-$C_3$-$C_{10}$-alkenyl $(R^{11})_n$ or NH-$C_3$-$C_{10}$-alkynyl $(R^{11})_n$, where $R^{11}$ is optionally substituted in each case by $R^{12}$; |
| $R^3$, $R^{11}$ and $R^{13}$: | phenyl, naphthyl, phenanthryl, pyridyl, thienyl, furyl, pyrimidyl, indolyl, imidazolyl, coumarinyl, phthaliminyl, quinolyl, piperazinyl, tetrazolyl, triazolyl, oxazolyl or their thieno- or benzo-fused derivatives, it being possible for the aromatic or heteroaromatic system to be substituted one or more times, identically or differently, by F, Cl, Br, I, OH, $CF_3$, $-NO_2$, CN, $C_1$-$C_4$-alkoxy, $C_1$-$C_4$-alkyl, $NR^8R^9$, phenyl, benzyl, thienyl, furyl, imidazolyl, pyridyl, O-phenyl or O-benzyl, and $R^3$, $R^{11}$ and $R^{13}$ are identical or different; |
| $R^4$, $R^5$ and $R^6$: | H, OH, an OH group which is protected by conventional alcohol-protective groups, F, Cl, Br or the meanings stated for $R^2$, where $R^4$, $R^5$ and $R^6$ are identical or different; |
| $R^7$: | $C_1$-$C_4$-alkyl, phenyl or benzyl; |
| $R^8$ and $R^9$: | H, $C_1$-$C_4$-alkyl, $C_1$-$C_4$-alkanoyl, phenyl which is optionally substituted by F, Cl, Br, I, OH, O-$C_1$-$C_4$-alkyl, $CF_3$, $-NO_2$ or CN, where $R^8$ and $R^9$ are identical or different, or $R^8$ and $R^9$ form together with the nitrogen atom a 4- to 10-membered, saturated heterocyclic ring in which a $CH_2$ group can optionally be replaced by O, S or $NR^{10}$, |
| $R^{10}$: | H, $C_1$-$C_4$-alkyl, phenyl or benzyl |
| $R^{12}$: | phenyl, naphthyl, phenanthryl, pyridyl, thienyl, furyl, pyrimidyl, indolyl, imidazolyl, coumarinyl, phthaliminyl, quinolyl, piperazinyl, tetrazolyl, triazolyl, oxazolyl or their thieno- or benzo-fused derivatives, it being possible for the aromatic or heteroaromatic system to be substituted one or more times, identically or differently, by F, Cl, Br, I, OH, $CF_3$, $-NO_2$, CN, $C_1$-$C_4$-alkoxy, $C_1$-$C_4$-alkyl, $NR^8R^9$, phenyl, benzyl, thienyl, furyl, imidazolyl, pyridyl, O-phenyl or O-benzyl; |
| X: | $(CH_2)_m$, $-CH=CH-$, $-C\equiv C-$, $-CH_2\text{-}O\text{-}CH_2-$, $-CH_2\text{-}S\text{-}CH_2-$ or |

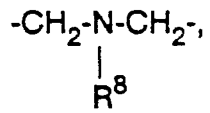

| | |
|---|---|
| Y: | $(CH_2)_m$, O, S, $NR^8$, |
| Z: | $(CH_2)_m$, S, O, S-$C_1$-$C_{10}$-alkyl, CH=CH, CH=CF, CH=CCl, CH=CBr, $CH_2$-CO, $CH_2$-CHF, $CH_2$-CHCl, $CH_2$-CHBr, $CH_2$-CHI, $C_3$-$C_{10}$-cycloalkylene, $C_3$-$C_{10}$-cycloalkenylene, $COOR^7$, C≡C, CH=C($C_1$-$C_4$-alkyl), CH=C(CN), CH=C($NR^8R^9$), CH=C($C_1$-$C_4$-alkanoyl), CH=C($R^{13}$), $NR^8$ and when Y is oxygen, |

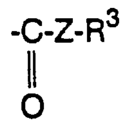

can together be an amino-acid residue selected from the group comprising Ala, Arg, Asn, Asp, Cys, Gln, Glu, Gly, His, Ile, Leu, Lys, Phe, Pro, Ser, Thr, Trp, Tyr and their derivatives protected by conventional protective groups,

| | |
|---|---|
| n: | zero, 1 or 2 |
| m: | zero, 1, 2, 3 or 4, |

and physiologically tolerated salts of compounds of the formula I, the compound of the formula 1a

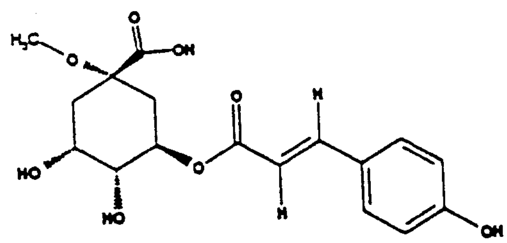

I a

being excepted.

3. A compound as claimed in claim 2, wherein $R^1$ in formula I is CN, COOH, a COOH group protected by a protective group, or $C_1$-$C_4$-alkanoyl, and the other radicals have the meanings stated in claim 1.

4. A compound as claimed in claim 2, wherein the radicals in formula I have the following meanings:

$R^1$: CN, COOH, a COOH group which is protected by a protective group, or $C_1$-$C_4$-alkanoyl

$R^2$: O-$C_1$-$C_{10}$-alkyl$(R^{11})_n$ (n = 0,1,2), where the alkyl moiety is unbranched, branched or cyclic, and one of the $CH_2$ groups can be replaced by O, and $R^{11}$ can be substituted by $R^{12}$, and when n = 2 the two $R^{11}$ radicals are identical or different,
O-$C_3$-$C_{10}$-alkenyl$(R^{11})_n$ (n = 0,1,2), where the alkenyl moiety is unbranched, branched or cyclic, one of the $CH_2$ groups can be replaced by O, S, SO, $SO_2$ or $NR^8$, and is unsaturated one or more times, and $R^{11}$ can be substituted by $R^{12}$, and when n = 2 the two $R^{11}$ radicals are identical or different, O-$C_3$-$C_{10}$-alkynyl$(R^{11})_n$

(n = 0,1,2), where the alkynyl moiety is unbranched, branched or cyclic and is unsaturated one or more times, and one of the $CH_2$ groups can be replaced by O, S, SO, $SO_2$ or $NR^8$, and $R^{11}$ can be substituted by $R^{12}$, and when n = 2 the two $R^{11}$ radicals are identical or different,
$R^3$ to $R^{12}$ and $R^{13}$ have the meanings stated in claim 2,

X: $(CH_2)_m$ (m=0,1,2,3,4), CH=CH, C≡C, $CH_2OCH_2$, $CH_2SCH_2$

Y: $(CH_2)_m$ (m=0,1,2,3,4), O, S, $NR^8$,

Z: $(CH_2)_m$ (m=0,1,2,3,4), S, O, S-$C_1$-$C_{10}$-alkyl, (unbranched or branched), CH=CH, CH=CF, CH=CCl, CH=CBr, $CH_2$-C(O), $CH_2$-CHF, $CH_2$-CHCl, $CH_2$-CHBr, $CH_2$-CHI, $C_3$-$C_{10}$-cycloalkylene, $C_3$-$C_{10}$-cycloalkenylene, $COOR^7$, C≡C, CH=C($C_1$-$C_4$-alkyl) (unbranched or branched), CH=C(CN), CH=C($R^{13}$), $NR^8$.

**5.** The use of compounds as claimed in claim 1 for the production of pharmaceuticals for the treatment of type II diabetes and other disorders characterized by an increased output of glucose from the liver or an increased activity of the glucose-6-phosphatase system.

**6.** A pharmaceutical containing a compound as claimed in claim 1.

**7.** A pharmaceutical containing a compound as claimed in claim 2.

**Revendications**

**1.** Dérivés du cyclohexane de formule I

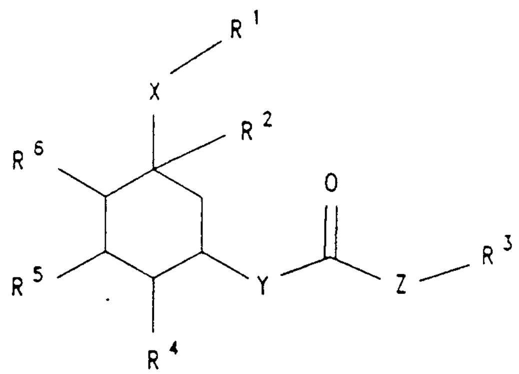

dans laquelle les groupes ont les significations suivantes :
$R^1$ : CN, COOH, un groupe COOH protégé par un groupe protecteur, alcanoyle-$C_1$-$C_4$, $SO_3$-alkyle-$C_1$-$C_4$, $SO_3H$, $SO_2NR^8R^9$, $PO(OH)_2$, PO(OH)(O-alkyle-$C_1$-$C_4$), PO(O-alkyle-$C_1$-$C_4$)$_2$,
$R^2$ : alkyl-$C_1$-$C_{10}$-$(R^{11})_n$, O-alkyl-$C_1$-$C_{10}$-$(R^{11})_n$, alcényl-$C_2$-$C_{10}$-$(R^{11})_n$, O-alcényl-$C_3$-$C_{10}$-$(R^{11})_n$, alcynyl-$C_2$-$C_{10}$-$(R^{11})_n$, O-alcynyl-$C_3$-$C_{10}$-$(R^{11})_n$, S-alkyl-$C_1$-$C_{10}$-$(R^{11})_n$, S-alcényl-$C_3$-$C_{10}$-$(R^{11})_n$, S-alcynyl-$C_3$-$C_{10}$-$(R^{11})_n$, NH-alkyl-$C_1$-$C_{10}$-$(R^{11})_n$, NH-alcényl-$C_3$-$C_{10}$-$(R^{11})_n$ ou NH-alcynyl-$C_3$-$C_{10}$-$(R^{11})_n$, où $R^{11}$ est éventuellement substitué par $R^{12}$ ;
$R^3$, $R^{11}$ et $R^{13}$ : groupe alkyle ayant 1 à 10 atomes de carbone, cycloalkyle avec un cycle de 3 à 8 atomes de carbone phényle, naphtyle, phénanthryle, pyridyle, thiényle, furyle, pyrimidyle, indolyle, imidazolyle, coumarinyle, phtaliminyle, quinoléyle, pipérazinyle, tétrazolyle, triazolyle, oxazolyle ou leurs dérivés condensés avec des noyaux thiéno, pyridino, pyrimidino ou benzo, où le cycle aromatique ou hétéroaromatique peut être substitué avec un ou plusieurs substituants, identiques ou différents, parmi F, Cl, Br I, OH, $CF_3$, -$NO_2$, CN, alkoxy-$C_1$-$C_4$, alkyle-C-$C_4$, $NR^8R^9$, phényle, benzyle, thiényle, furyle, imidazolyle, pyridyle, O-phényle ou O-benzyle, les groupes $R^3$, $R^{11}$ et $R^{13}$ étant identiques ou différents ;
$R^4$, $R^5$ et $R^6$ : H, OH, un groupe OH protégé par un groupe protecteur généralement utilisé pour la protection des alcools, F, Cl, Br, ou un des groupes proposés pour le groupe $R^2$, les groupes $R^4$, $R^5$ et $R^6$ étant identiques ou différents ;
$R^7$ : alkyle-$C_1$-$C_4$, phényle ou benzyle ;
$R^8$ et $R^9$ : H, alkyle-$C_1$-$C_4$, alcanoyle-$C_1$-$C_4$, phényle, éventuellement substitué par F, Cl, Br, I, OH, O-alkyle-$C_1$-$C_4$,

$CF_3$, $-NO_2$ ou CN, - $R^8$ et $R^9$ étant identiques ou différents, ou bien $R^8$ et $R^9$ formant ensemble avec l'atome d'azote un hétérocycle saturé de 4 à 10 atomes dans lequel un groupe $CH_2$ peut éventuellement être remplacé par O, S ou $NR^{10}$,

$R^{10}$ : H, alkyle-$C_1$-$C_4$, phényle ou benzyle

$R^{12}$ : phényle, naphtyle, phénanthryle, pyridyle, thiényle, furyle, pyrimidyle, indolyle, imidazolyle, coumarinyle, phtaliminyle, quinoléyle, pipérazinyle, tétrazolyle, triazolyle, oxazolyle ou leur dérivés condensés avec des noyaux thiéno ou benzoannelé, où le cycle aromatique ou hétéroaromatique peut être substitué avec un ou plusieurs substituants, identiques ou différents, parmi F, Cl, Br, I, OH, $CF_3$, $-NO_2$, CN, alkoxy-$C_1$-$C_4$, alkyle-$C_1$-$C_4$, alcényl-$C_2$-$C_4$, $NR^8R^9$, phényle, benzyle, thiényle, furyle, imidazolyle, pyridyle, O-phényle ou O-benzyle ;

X : $(CH_2)_m$, -CH=CH-, -C≡C-, $-CH_2-O-CH_2-$, $-CH_2-S-CH_2-$ ou $-CH_2-N(R^8)-CH_2-$,

Y : $(CH_2)_m$, O, S, $NR^8$,

Z : $(CH_2)_m$, S, O, S-alkyle-$C_1$-$C_{10}$, O-alkyle-$C_1$-$C_{10}$, CH=CH, CH=CF, CH=CCl, CH=CBr, $CH_2$-CO, $CH_2$-CHF, $CH_2$-CHCl $CH_2$-CHBr, $CH_2$-CHI, cycloalkylène-$C_3$-$C_{10}$, cycloalcénylène-$C_3$-$C_{10}$, dans lesquels 1 à 3 atomes de carbone du cycle peuvent être remplacés par un atome de soufre, d'oxygène, d'azote, $COOR^7$, C≡C, CH=C(alkyle-$C_1$-$C_4$) CH=C(CN), CH=C($NR^8R^9$), CH=C(alcanoyle-$C_1$-$C_4$), CH=C($R^{13}$), $NR^8$, et, si Y est un atome d'oxygène, le groupe -C(O)-Z-$R^3$ peut représenter un reste d'un acide aminé choisi parmi Ala, Arg, Asn, Asp, Cys, Gln, Glu, Gly, His, Ile, Leu, Lys, Phe, Pro, Ser, Thr, Trp, Tyr, et leurs dérivés protégés par les groupes protecteurs habituellement utilisés,

n : 0, 1 ou 2

m : 0, 1, 2, 3 ou 4,

ainsi que les sels physiologiquement acceptables des composés de formule I, à l'exclusion du composé de formule Ia.

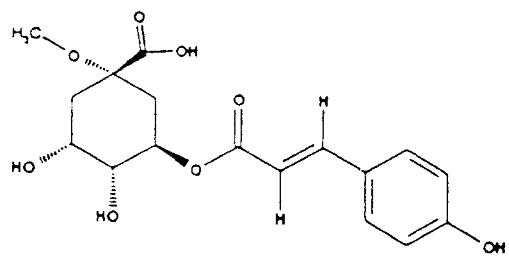

2. Dérives du cyclohexane de formule I

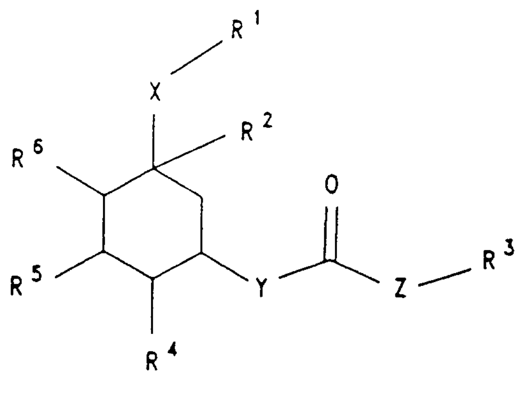

dans laquelle les groupes ont les significations suivantes :

$R^1$ : CN, COOH, un groupe COOH protégé par un groupe protecteur, alcanoyle-$C_1$-$C_4$, $SO_3$-alkyle-$C_1$-$C_4$, $SO_3H$, $SO_2NR^8R^9$, $PO(OH)_2$, PO(OH)(O-alkyle-$C_1$-$C_4$), PO(O-alkyle-$C_1$-$C_4)_2$,

$R^2$ : alkyl-$C_1$-$C_{10}$-$(R^{11})_n$, O-alkyl-$C_1$-$C_{10}$-$(R^{11})_n$, alcényl-$C_2$-$C_{10}$-$(R^{11})_n$, O-alcényl-$C_3$-$C_{10}$-$(R^{11})_n$, alcynyl-$C_2$-$C_{10}$-$(R^{11})_n$, O-alcynyl-$C_3$-$C_{10}$-$(R^{11})_n$, S-alkyl-$C_1$-$C_{10}$-$(R^{11})_n$, S-alcényl-$C_3$-$C_{10}$-$(R^{11})_n$, S-alcynyl-$C_3$-$C_{10}$-$(R^{11})_n$, NH-alkyl-$C_1$-$C_{10}$-$(R^{11})_n$, NH-alcényl-$C_3$-$C_{10}$-$(R^{11})_n$ ou NH-alcynyl-$C_3$-$C_{10}$-$(R^{11})_n$, où $R^{11}$ est éventuellement substitué par $R^{12}$

;

$R^3$, $R^{11}$ et $R^{13}$ : phényle, naphtyle, phénanthryle, pyridyle, thiényle, furyle, pyrimidyle, indolyle, imidazolyle, coumarinyle, phtaliminyle, quinoléyle, pipérazinyle, tétrazolyle, triazolyle, oxazolyle ou leurs dérivés condensés avec des noyaux thiéno ou benzo, où le cycle aromatique ou hétéroaromatique peut être substitué avec un ou plusieurs substituants, identiques ou différents, parmi F, Cl, Br, I, OH, $CF_3$, $-NO_2$, CN, alcoxy-$C_1$-$C_4$, alkyle-$C_1$-$C_4$, $NR^8R^9$, phényle, benzyle, thiényle, furyle, imidazolyle, pyridyle, O-phényle ou O-benzyle, les groupes $R^3$, $R^{11}$ et $R^{13}$ étant identiques ou différents ;

$R^4$, $R^5$ et $R^6$ : H, OH, un groupe OH protégé par un groupe protecteur généralement utilisé pour la protection des alcools, F, Cl, Br, ou un des groupes proposés pour le groupe $R^2$, les groupes $R^4$, $R^5$ et $R^6$ étant identiques ou différents ;

$R^7$ : alkyle-$C_1$-$C_4$, phényle ou benzyle ;

$R^8$ et $R^9$ : H, alkyle-$C_1$-$C_4$, alcanoyle-$C_1$-$C_4$, phényle, éventuellement substitué par F, Cl, Br, I, OH, O-alkyle-$C_1$-$C_4$, $CF_3$, $-NO_2$ ou CN, $R^8$ et $R^9$ étant identiques ou différents, ou bien $R^8$ et $R^9$ forment ensemble avec l'atome d'azote un hétérocycle saturé de 4 à 10 atomes dans lequel un groupe $CH_2$ peut éventuellement être remplacé par O, S ou $NR^{10}$,

$R^{10}$ : H, alkyle-$C_1$-$C_4$, phényle, benzyle

$R^{12}$ : phényle, naphtyle, phénanthryle, pyridyle, thiényle, furyle, pyrimidyle, indolyle, imidazolyle, coumarinyle, phtaliminyle, quinoléyle, pipérazinyle, tétrazolyle, triazolyle, oxazolyle ou leurs dérivés condensés avec des noyaux thiéno ou benzo, où le cycle aromatique ou hétéroaromatique peut être substitué avec un ou plusieurs substituants, identiques ou différents, parmi F, Cl, Br, I, OH, $CF_3$, $-NO_2$, CN, alcoxy-$C_1$-$C_4$, alkyle-$C_1$-$C_4$, $NR^8R^9$, phényle, benzyle, thiényle, furyle, imidazolyle, pyridyle, O-phényle ou O-benzyle ;

X : $(CH_2)_m$, -CH=CH-, -C≡C-, $-CH_2-O-CH_2-$, $-CH_2-S-CH_2-$ ou $-CH_2-N(R^8)-CH_2-$,

Y : $(CH_2)_m$, O, S, $NR^8$,

Z : $(CH_2)_m$, S, O, S-alkyle-$C_1$-$C_{10}$, CH=CH, CH=CF, CH=CCl, CH=CBr, $CH_2$-CO, $CH_2$-CHF, $CH_2$-CHCl, $CH_2$-CHBr, $CH_2$-CHI, cycloalkylène-$C_3$-$C_{10}$, cycloalcénylène-$C_3$-$C_{10}$, $COOR^7$, C≡C, CH=C(alkyle-$C_1$-$C_4$), CH=C(CN), $CH=C(NR^8R^9)$, CH=C(alcanoyle-$C_1$-$C_4$), $CH=C(R^{13})$, $NR^8$, et, si Y est un atome d'oxygène, le groupe -C(O)-Z-$R^3$ peut représenter un reste d'acide aminé choisi parmi Ala, Arg, Asn, Asp, Cys, Gln, Glu, Gly, His, Ile, Leu, Lys, Phe, Pro, Ser, Thr, Trp, Tyr, et leurs dérivés protégés par les groupes protecteurs habituellement utilisés,

n : 0, 1 ou 2

m : 0, 1, 2, 3 ou 4,

ainsi que les sels physiologiquement acceptables des composés de formule I, à l'exclusion du composé de formule Ia.

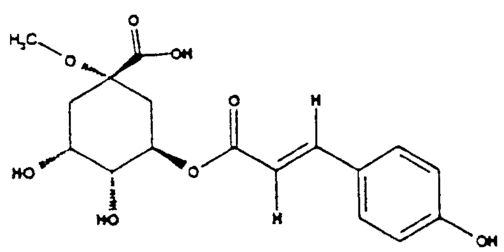

3. Composés selon la revendication 2, caractérisés en ce que, dans la formule I,

R1 représente CN, COOH, un groupe COOH protégé par un groupe protecteur, ou un groupe alcanoyle-$C_1$-$C_4$,

et les autres groupes ont les significations données dans la revendication 1.

4. Composés selon la revendication 2, caractérisés en ce que, dans la formule I, les groupes ont les significations suivantes :

$R^1$ : CN, COOH, un groupe COOH protégé par un groupe protecteur, ou un groupe alcanoyle-$C_1$-$C_4$,

$R^2$ : O-alkyl-$C_1$-$C_{10}$-$(R^{11})_n$ (n = 0,1,2), où la partie alkyle peut être non ramifiée, ramifiée ou cyclique, de même qu'un des groupes $CH_2$ peut être remplacé par un atome O et que $R^{11}$ peut être substitué par $R^{12}$, et dans le cas où n = 2 les deux groupes $R^{11}$ peuvent être identiques ou différents, O-alcényl-$C_3$-$C_{10}$-$(R^{11})_n$ (n = 0,1,2), où la partie alcényle peut être non ramifiée, ramifiée ou cyclique, un des groupes $CH_2$ pouvant être

remplacé par O, S, SO, $SO_2$ ou $NR^8$, et est une ou plusieurs fois insaturée, $R^{11}$ peut être substitué par $R^{12}$, et dans le cas où n = 2 les deux groupes $R^{11}$ peuvent être identiques ou différents, O-alcynyl-$C_3$-$C_{10}$-$(R^{11})_n$ (n = 0,1,2), où la partie alcynyle peut être non ramifiée, ramifiée ou cyclique, une ou plusieurs fois insaturée, un des groupes $CH_2$ pouvant être remplacé par O, S, SO, $SO_2$ ou $NR^8$, $R^{11}$ peut être substitué par $R^{12}$, et dans le cas où n = 2 les deux groupes $R^{11}$ peuvent être identiques ou différents,
$R^3$ à $R^{12}$ et $R^{13}$ ont la signification donnée dans la revendication 2,

X : $(CH_2)_m$ (m = 0,1,2,3,4), -CH=CH-, -C≡C-, -$CH_2$-O-$CH_2$-, -$CH_2$-S-$CH_2$-

Y : $(CH_2)_m$ (m = 0,1,2,3,4), O, S, $NR^8$,

Z : $(CH_2)_m$ (m = 0,1,2,3,4), S, O, S-alkyle-$C_1$-$C_{10}$ (non ramifié ou ramifié), CH=CH, CH=CF, CH=CCl, CH=CBr, $CH_2$-C(O), $CH_2$-CHF, $CH_2$-CHCl, $CH_2$-CHBr, $CH_2$-CHI, cycloalkylène-$C_3$-$C_{10}$, cycloalcénylène-$C_3$-$C_{10}$, $COOR^7$, C≡C, CH=C(alkyle-$C_1$-$C_4$) (non ramifié ou ramifié), CH=C(CN), CH=C($R^{13}$), $NR^8$.

**5.** Utilisation de composés selon la revendication 1 pour la fabrication de médicaments pour le traitement du diabète de type II et d'autres maladies caractérisées par l'augmentation de la production de glucose par le foie ou par l'activité accrue du système de la glucose-6-phosphatase.

**6.** Médicament contenant un composé selon la revendication 1.

**7.** Médicament contenant un composé selon la revendication 2.